# EUROPEAN PATENT APPLICATION

(11) **EP 4 763 862 A1**
(43) Date of publication of application: **24.06.2026**
(21) Application number: 24867561.3
(22) Date of filing: 20.09.2024
(51) Int. Cl.: C07K 16/28, A61K 39/395, A61P 35/00, A61P 35/02, A61K 47/68, A61K 39/00, C12N 15/63, C12N 5/10, C07K 19/00

(54) **B7-H3-BINDING ANTIBODY AND USE THEREOF**

(30) Priority: 21.09.2023 CN 202311224031
(71) Applicant: Beijing Tide Pharmaceutical Co., Ltd., Beijing 100176 (CN)
(72) Inventor: LI, Wei, Beijing 100176 (CN); ZHANG, Min, Beijing 100176 (CN); HE, Jingchang, Beijing 100176 (CN); GUO, Tingting, Beijing 100176 (CN); ZHANG, Qi, Beijing 100176 (CN); WANG, Yanming, Beijing 100176 (CN)
(74) Representative: Novitas Patent AB
(86) International application number: PCT/CN2024/120084
(87) International publication number: WO 2025/061156

(57) **Abstract**

The present application provides an anti-B7-H3 monoclonal antibody, specifically a high-affinity B7-H3 humanized monoclonal antibody having cross-activity with humans, crab-eating macaques, and mice, as well as a nucleic acid comprising the encoded antibody, a vector comprising the nucleic acid, and a host cell comprising the nucleic acid or the vector. The present application further discloses a pharmaceutical composition and a conjugate comprising the antibody, and a treatment method using the antibody.

## Description

### TECHNICAL FIELD

The present disclosure relates to the field of biomedicine, and particularly to an antibody binding to B7-H3 and use thereof in cancer treatment.

### BACKGROUND

B7-H3 (CD276) is a member of the B7 family of proteins and plays a key role in the development of cancer. As one of the immune checkpoint targets, B7-H3 is selectively expressed on tumor cells and immune cells in the tumor microenvironment, and is involved in the proliferation and metastasis of tumor cells, and is related to drug resistance. The protein expression levels of B7-H3 in normal tissues and tumor tissues exhibit significant differences. B7-H3 is highly expressed in various tumor tissues, such as prostate cancer, pancreatic cancer, hepatocellular carcinoma, etc., while rarely expressed in normal tissues (Zhou WT, Jin WL. B7-H3/CD276: An Emerging Cancer Immunotherapy. Front Immunol. 2021; 12:701006.). Utilizing this characteristic, targeting B7-H3 with drugs may specifically kill cancer tissues while minimizing damage to healthy cells. These properties make B7-H3 a promising cancer therapeutic target.

### SUMMARY OF THE INVENTION

The present disclosure provides a novel antibody or antigen-binding fragment thereof that targets B7-H3.

In a first aspect, the present disclosure provides an antibody that specifically binds to B7-H3or an antigen-binding fragment thereof, wherein the antibody comprises a heavy chain variable region (VH) and a light chain variable region (VL).

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 3, and the VL comprises LCDR1, LCDR2, and LCDR3 in a light chain variable region having the amino acid sequence as set forth in SEQ ID NO: 13.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 26, and the VL comprises LCDR1, LCDR2, and LCDR3 in a light chain variable region having the amino acid sequence as set forth in SEQ ID NO: 27.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 28, and the VL comprises LCDR1, LCDR2, and LCDR3 in a light chain variable region having the amino acid sequence as set forth in SEQ ID NO: 29.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 30, and the VL comprises LCDR1, LCDR2, and LCDR3 in a light chain variable region having the amino acid sequence as set forth in SEQ ID NO: 29.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 22, and the VL comprises LCDR1, LCDR2, and LCDR3 in a light chain variable region having the amino acid sequence as set forth in SEQ ID NO: 23.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 33, and the VL comprises LCDR1, LCDR2, and LCDR3 in a light chain variable region having the amino acid sequence as set forth in SEQ ID NO: 42.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 34, and the VL comprises LCDR1, LCDR2, and LCDR3 in a light chain variable region having the amino acid sequence as set forth in SEQ ID NO: 42.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 33, and the VL comprises LCDR1, LCDR2, and LCDR3 in a light chain variable region having the amino acid sequence as set forth in SEQ ID NO: 41.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 34, and the VL comprises LCDR1, LCDR2, and LCDR3 in a light chain variable region having the amino acid sequence as set forth in SEQ ID NO: 39.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 34, and the VL comprises LCDR1, LCDR2, and LCDR3 in a light chain variable region having the amino acid sequence as set forth in SEQ ID NO: 40.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 34, and the VL comprises LCDR1, LCDR2, and LCDR3 in a light chain variable region having the amino acid sequence as set forth in SEQ ID NO: 41.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 1, and the VL comprises LCDR1, LCDR2, and LCDR3 in a light chain variable region having the amino acid sequence as set forth in SEQ ID NO: 2.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 3, and the VL comprises LCDR1, LCDR2, and LCDR3 in a light chain variable region having the amino acid sequence as set forth in SEQ ID NO: 4.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 5, and the VL comprises LCDR1, LCDR2, and LCDR3 in a light chain variable region having the amino acid sequence as set forth in SEQ ID NO: 6.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 7, and the VL comprises LCDR1, LCDR2, and LCDR3 in a light chain variable region having the amino acid sequence as set forth in SEQ ID NO: 8.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 9, and the VL comprises LCDR1, LCDR2, and LCDR3 in a light chain variable region having the amino acid sequence as set forth in SEQ ID NO: 10.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 11, and the VL comprises LCDR1, LCDR2, and LCDR3 in a light chain variable region having the amino acid sequence as set forth in SEQ ID NO: 12.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 14, and the VL comprises LCDR1, LCDR2, and LCDR3 in a light chain variable region having the amino acid sequence as set forth in SEQ ID NO: 15.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 16, and the VL comprises LCDR1, LCDR2, and LCDR3 in a light chain variable region having the amino acid sequence as set forth in SEQ ID NO: 17.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 18, and the VL comprises LCDR1, LCDR2, and LCDR3 in a light chain variable region having the amino acid sequence as set forth in SEQ ID NO: 19.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 20, and the VL comprises LCDR1, LCDR2, and LCDR3 in a light chain variable region having the amino acid sequence as set forth in SEQ ID NO: 21.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 24, and the VL comprises LCDR1, LCDR2, and LCDR3 in a light chain variable region having the amino acid sequence as set forth in SEQ ID NO: 25.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises HCDR1 as set forth in SEQ ID NO: 84, HCDR2 as set forth in SEQ ID NO: 86, and HCDR3 as set forth in SEQ ID NO: 88, and the VL comprises LCDR1 as set forth in SEQ ID NO: 173, LCDR2 as set forth in SEQ ID NO: 175, and LCDR3 as set forth in SEQ ID NO: 177.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1 as set forth in SEQ ID NO: 84, HCDR2 as set forth in SEQ ID NO: 86, and HCDR3 as set forth in SEQ ID NO: 126, and the VL comprises LCDR1 as set forth in SEQ ID NO: 213, LCDR2 as set forth in SEQ ID NO: 175, and LCDR3 as set forth in SEQ ID NO: 177.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1 as set forth in SEQ ID NO: 111, HCDR2 as set forth in SEQ ID NO: 113, and HCDR3 as set forth in SEQ ID NO: 115, and the VL comprises LCDR1 as set forth in SEQ ID NO: 201, LCDR2 as set forth in the amino acid sequence NAK, and LCDR3 as set forth in SEQ ID NO: 205.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1 as set forth in SEQ ID NO: 111, HCDR2 as set forth in SEQ ID NO: 113, and HCDR3 as set forth in SEQ ID NO: 115, and the VL comprises LCDR1 as set forth in SEQ ID NO: 201, LCDR2 as set forth in the amino acid sequence NAK, and LCDR3 as set forth in SEQ ID NO: 203.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1 as set forth in SEQ ID NO: 44, HCDR2 as set forth in SEQ ID NO: 46, and HCDR3 as set forth in SEQ ID NO: 48, and the VL comprises LCDR1 as set forth in SEQ ID NO: 135, LCDR2 as set forth in the amino acid sequence YAS, and LCDR3 as set forth in SEQ ID NO: 139.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1 as set forth in SEQ ID NO: 51, HCDR2 as set forth in SEQ ID NO: 53, and HCDR3 as set forth in SEQ ID NO: 55, and the VL comprises LCDR1 as set forth in SEQ ID NO: 142, LCDR2 as set forth in the amino acid sequence YAS, and LCDR3 as set forth in SEQ ID NO: 145.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1 as set forth in SEQ ID NO: 58, HCDR2 as set forth in SEQ ID NO: 60, and HCDR3 as set forth in SEQ ID NO: 62, and the VL comprises LCDR1 as set forth in SEQ ID NO: 148, LCDR2 as set forth in the amino acid sequence KVS, and LCDR3 as set forth in SEQ ID NO: 152.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1 as set forth in SEQ ID NO: 65, HCDR2 as set forth in SEQ ID NO: 67, and HCDR3 as set forth in SEQ ID NO: 69, and the VL comprises LCDR1 as set forth in SEQ ID NO: 154, LCDR2 as set forth in the amino acid sequence RMS, and LCDR3 as set forth in SEQ ID NO: 158.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1 as set forth in SEQ ID NO: 72, HCDR2 as set forth in SEQ ID NO: 74, and HCDR3 as set forth in SEQ ID NO: 76, and the VL comprises LCDR1 as set forth in SEQ ID NO: 160, LCDR2 as set forth in the amino acid sequence GAS, and LCDR3 as set forth in SEQ ID NO: 164.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1 as set forth in SEQ ID NO: 79, HCDR2 as set forth in SEQ ID NO: 81, and HCDR3 as set forth in SEQ ID NO: 83, and the VL comprises LCDR1 as set forth in SEQ ID NO: 166, LCDR2 as set forth in the amino acid sequence SGS, and LCDR3 as set forth in SEQ ID NO: 170.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1 as set forth in SEQ ID NO: 90, HCDR2 as set forth in SEQ ID NO: 92, and HCDR3 as set forth in SEQ ID NO: 94, and the VL comprises LCDR1 as set forth in SEQ ID NO: 179, LCDR2 as set forth in the amino acid sequence FAS, and LCDR3 as set forth in SEQ ID NO: 183.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1 as set forth in SEQ ID NO: 96, HCDR2 as set forth in SEQ ID NO: 98, and HCDR3 as set forth in SEQ ID NO: 100, and the VL comprises LCDR1 as set forth in SEQ ID NO: 185, LCDR2 as set forth in the amino acid sequence KVS, and LCDR3 as set forth in SEQ ID NO: 186.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1 as set forth in SEQ ID NO: 101, HCDR2 as set forth in SEQ ID NO: 102, and HCDR3 as set forth in SEQ ID NO: 104, and the VL comprises LCDR1 as set forth in SEQ ID NO: 188, LCDR2 as set forth in the amino acid sequence DTS, and LCDR3 as set forth in SEQ ID NO: 192.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1 as set forth in SEQ ID NO: 79, HCDR2 as set forth in SEQ ID NO: 107, and HCDR3 as set forth in SEQ ID NO: 109, and the VL comprises LCDR1 as set forth in SEQ ID NO: 195, LCDR2 as set forth in the amino acid sequence WAS, and LCDR3 as set forth in SEQ ID NO: 199.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1 as set forth in SEQ ID NO: 117, HCDR2 as set forth in SEQ ID NO: 119, and HCDR3 as set forth in SEQ ID NO: 121, and the VL comprises LCDR1 as set forth in SEQ ID NO: 208, LCDR2 as set forth in the amino acid sequence DTS, and LCDR3 as set forth in SEQ ID NO: 211.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 3, and the VL comprises an amino acid sequence having at least 80%, at least 85%, at least 90%, at least 95%, at least 98%, at least 99% or 100% sequence identity to SEQ ID NO: 13.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 26, and the VL comprises an amino acid sequence having at least 80%, at least 85%, at least 90%, at least 95%, at least 98%, at least 99% or 100% sequence identity to SEQ ID NO: 27.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 28, and the VL comprises an amino acid sequence having at least 80%, at least 85%, at least 90%, at least 95%, at least 98%, at least 99% or 100% sequence identity to SEQ ID NO: 29.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 30, and the VL comprises an amino acid sequence having at least 80%, at least 85%, at least 90%, at least 95%, at least 98%, at least 99% or 100% sequence identity to SEQ ID NO: 29.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 22, and the VL comprises an amino acid sequence having at least 80%, at least 85%, at least 90%, at least 95%, at least 98%, at least 99% or 100% sequence identity to SEQ ID NO: 23.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 33, and the VL comprises an amino acid sequence having at least 80%, at least 85%, at least 90%, at least 95%, at least 98%, at least 99% or 100% sequence identity to SEQ ID NO: 42.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 34, and the VL comprises an amino acid sequence having at least 80%, at least 85%, at least 90%, at least 95%, at least 98%, at least 99% or 100% sequence identity to SEQ ID NO: 42.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 33, and the VL comprises an amino acid sequence having at least 80%, at least 85%, at least 90%, at least 95%, at least 98%, at least 99% or 100% sequence identity to SEQ ID NO: 41.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 34, and the VL comprises an amino acid sequence having at least 80%, at least 85%, at least 90%, at least 95%, at least 98%, at least 99% or 100% sequence identity to SEQ ID NO: 39.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 34, and the VL comprises an amino acid sequence having at least 80%, at least 85%, at least 90%, at least 95%, at least 98%, at least 99% or 100% sequence identity to SEQ ID NO: 40.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 34, and the VL comprises an amino acid sequence having at least 80%, at least 85%, at least 90%, at least 95%, at least 98%, at least 99% or 100% sequence identity to SEQ ID NO: 41.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 1, and the VL comprises an amino acid sequence having at least 80%, at least 85%, at least 90%, at least 95%, at least 98%, at least 99% or 100% sequence identity to SEQ ID NO: 2.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 3, and the VL comprises an amino acid sequence having at least 80%, at least 85%, at least 90%, at least 95%, at least 98%, at least 99% or 100% sequence identity to SEQ ID NO: 4.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 5, and the VL comprises an amino acid sequence having at least 80%, at least 85%, at least 90%, at least 95%, at least 98%, at least 99% or 100% sequence identity to SEQ ID NO: 6.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 7, and the VL comprises an amino acid sequence having at least 80%, at least 85%, at least 90%, at least 95%, at least 98%, at least 99% or 100% sequence identity to SEQ ID NO: 8.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 9, and the VL comprises an amino acid sequence having at least 80%, at least 85%, at least 90%, at least 95%, at least 98%, at least 99% or 100% sequence identity to SEQ ID NO: 10.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 11, and the VL comprises an amino acid sequence having at least 80%, at least 85%, at least 90%, at least 95%, at least 98%, at least 99% or 100% sequence identity to SEQ ID NO: 12.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 14, and the VL comprises an amino acid sequence having at least 80%, at least 85%, at least 90%, at least 95%, at least 98%, at least 99% or 100% sequence identity to SEQ ID NO: 15.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 16, and the VL comprises an amino acid sequence having at least 80%, at least 85%, at least 90%, at least 95%, at least 98%, at least 99% or 100% sequence identity to SEQ ID NO: 17.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 18, and the VL comprises an amino acid sequence having at least 80%, at least 85%, at least 90%, at least 95%, at least 98%, at least 99% or 100% sequence identity to SEQ ID NO: 19.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 20, and the VL comprises an amino acid sequence having at least 80%, at least 85%, at least 90%, at least 95%, at least 98%, at least 99% or 100% sequence identity to SEQ ID NO: 21.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 24, and the VL comprises an amino acid sequence having at least 80%, at least 85%, at least 90%, at least 95%, at least 98%, at least 99% or 100% sequence identity to SEQ ID NO: 25.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 3, and the VL comprises the amino acid sequence as set forth in SEQ ID NO: 13.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 26, and the VL comprises the amino acid sequence as set forth in SEQ ID NO: 27.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 28, and the VL comprises the amino acid sequence as set forth in SEQ ID NO: 29.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 30, and the VL comprises the amino acid sequence as set forth in SEQ ID NO: 29.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 22, and the VL comprises the amino acid sequence as set forth in SEQ ID NO: 23.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 33, and the VL comprises the amino acid sequence as set forth in SEQ ID NO: 42.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 34, and the VL comprises the amino acid sequence as set forth in SEQ ID NO: 42.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 33, and the VL comprises the amino acid sequence as set forth in SEQ ID NO: 41.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 34, and the VL comprises the amino acid sequence as set forth in SEQ ID NO: 39.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 34, and the VL comprises the amino acid sequence as set forth in SEQ ID NO: 40.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 34, and the VL comprises the amino acid sequence as set forth in SEQ ID NO: 41.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 1, and the VL comprises the amino acid sequence as set forth in SEQ ID NO: 2.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 3, and the VL comprises the amino acid sequence as set forth in SEQ ID NO: 4.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 5, and the VL comprises the amino acid sequence as set forth in SEQ ID NO: 6.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 7, and the VL comprises the amino acid sequence as set forth in SEQ ID NO: 8.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 9, and the VL comprises the amino acid sequence as set forth in SEQ ID NO: 10.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 11, and the VL comprises the amino acid sequence as set forth in SEQ ID NO: 12.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 14, and the VL comprises the amino acid sequence as set forth in SEQ ID NO: 15.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 16, and the VL comprises the amino acid sequence as set forth in SEQ ID NO: 17.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 18, and the VL comprises the amino acid sequence as set forth in SEQ ID NO: 19.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 20, and the VL comprises the amino acid sequence as set forth in SEQ ID NO: 21.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises the amino acid sequence as set forth in SEQ ID NO: 24, and the VL comprises the amino acid sequence as set forth in SEQ ID NO: 25.

In some embodiments of the antibody or antigen-binding fragment thereof disclosed herein, the antibody is a murine antibody, a chimeric antibody, a humanized antibody, or a human antibody.

In some embodiments of the antibody or antigen-binding fragment thereof disclosed herein, the antibody is of an isotype selected from the group consisting of IgG, IgA, IgM, IgE, and IgD.

In some embodiments of the antibody or antigen-binding fragment thereof disclosed herein, the antibody is of a subtype selected from the group consisting of IgG1, IgG2, IgG3, and IgG4.

In some embodiments of the antibody or antigen-binding fragment thereof disclosed herein, the antigen-binding fragment is selected from the group consisting of Fab, Fab', F(ab')2, Fv, scFv, and ds-scFv.

In some embodiments of the antibody or antigen-binding fragment thereof disclosed herein, the antibody is a monoclonal antibody.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 230, and the light chain comprises an amino acid sequence having at least 80%, at least 85%, at least 90%, at least 95%, at least 98%, at least 99% or 100% sequence identity to SEQ ID NO: 231.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 248, and the light chain comprises an amino acid sequence having at least 80%, at least 85%, at least 90%, at least 95%, at least 98%, at least 99% or 100% sequence identity to SEQ ID NO: 249.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 250, and the light chain comprises an amino acid sequence having at least 80%, at least 85%, at least 90%, at least 95%, at least 98%, at least 99% or 100% sequence identity to SEQ ID NO: 251.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 252, and the light chain comprises an amino acid sequence having at least 80%, at least 85%, at least 90%, at least 95%, at least 98%, at least 99% or 100% sequence identity to SEQ ID NO: 251.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 240, and the light chain comprises an amino acid sequence having at least 80%, at least 85%, at least 90%, at least 95%, at least 98%, at least 99% or 100% sequence identity to SEQ ID NO: 241.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 255, and the light chain comprises an amino acid sequence having at least 80%, at least 85%, at least 90%, at least 95%, at least 98%, at least 99% or 100% sequence identity to SEQ ID NO: 264.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 256, and the light chain comprises an amino acid sequence having at least 80%, at least 85%, at least 90%, at least 95%, at least 98%, at least 99% or 100% sequence identity to SEQ ID NO: 264.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 255, and the light chain comprises an amino acid sequence having at least 80%, at least 85%, at least 90%, at least 95%, at least 98%, at least 99% or 100% sequence identity to SEQ ID NO: 263.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 256, and the light chain comprises an amino acid sequence having at least 80%, at least 85%, at least 90%, at least 95%, at least 98%, at least 99% or 100% sequence identity to SEQ ID NO: 261.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 256, and the light chain comprises an amino acid sequence having at least 80%, at least 85%, at least 90%, at least 95%, at least 98%, at least 99% or 100% sequence identity to SEQ ID NO: 262.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 256, and the light chain comprises an amino acid sequence having at least 80%, at least 85%, at least 90%, at least 95%, at least 98%, at least 99% or 100% sequence identity to SEQ ID NO: 263.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 218, and the light chain comprises an amino acid sequence having at least 80%, at least 85%, at least 90%, at least 95%, at least 98%, at least 99% or 100% sequence identity to SEQ ID NO: 219.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 220, and the light chain comprises an amino acid sequence having at least 80%, at least 85%, at least 90%, at least 95%, at least 98%, at least 99% or 100% sequence identity to SEQ ID NO: 221.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 222, and the light chain comprises an amino acid sequence having at least 80%, at least 85%, at least 90%, at least 95%, at least 98%, at least 99% or 100% sequence identity to SEQ ID NO: 223.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 224, and the light chain comprises an amino acid sequence having at least 80%, at least 85%, at least 90%, at least 95%, at least 98%, at least 99% or 100% sequence identity to SEQ ID NO: 225.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 226, and the light chain comprises an amino acid sequence having at least 80%, at least 85%, at least 90%, at least 95%, at least 98%, at least 99% or 100% sequence identity to SEQ ID NO: 227.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 228, and the light chain comprises an amino acid sequence having at least 80%, at least 85%, at least 90%, at least 95%, at least 98%, at least 99% or 100% sequence identity to SEQ ID NO: 229.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 232, and the light chain comprises an amino acid sequence having at least 80%, at least 85%, at least 90%, at least 95%, at least 98%, at least 99% or 100% sequence identity to SEQ ID NO: 233.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 234, and the light chain comprises an amino acid sequence having at least 80%, at least 85%, at least 90%, at least 95%, at least 98%, at least 99% or 100% sequence identity to SEQ ID NO: 235.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 236, and the light chain comprises an amino acid sequence having at least 80%, at least 85%, at least 90%, at least 95%, at least 98%, at least 99% or 100% sequence identity to SEQ ID NO: 237.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 238, and the light chain comprises an amino acid sequence having at least 80%, at least 85%, at least 90%, at least 95%, at least 98%, at least 99% or 100% sequence identity to SEQ ID NO: 239.

In some embodiments of the antibody or antigen-binding fragment thereof disclosed herein, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 242, and the light chain comprises an amino acid sequence having at least 80%, at least 85%, at least 90%, at least 95%, at least 98%, at least 99% or 100% sequence identity to SEQ ID NO: 243.

In some embodiments of the antibody or antigen-binding fragment thereof disclosed herein, the antibody is a bispecific antibody or a multispecific antibody.

In a second aspect, the present disclosure provides a nucleic acid encoding the antibody or antigen-binding fragment thereof of the first aspect of the present disclosure.

In a third aspect, the present disclosure provides a vector comprising the nucleic acid of the second aspect of the present disclosure.

In a fourth aspect, the present disclosure provides a host cell comprising the nucleic acid of the second aspect of the present disclosure or the vector of the third aspect of the present disclosure.

In a fifth aspect, the present disclosure provides a pharmaceutical composition comprising (i) the antibody or antigen-binding fragment thereof of the first aspect of the present disclosure, the nucleic acid of the second aspect of the present disclosure, the vector of the third aspect of the present disclosure, or the host cell of the fourth aspect of the present disclosure; and (ii) a pharmaceutically acceptable carrier or excipient. In some embodiments of the pharmaceutical composition disclosed herein, the composition further comprises a second therapeutic agent. In some embodiments of the pharmaceutical composition disclosed herein, the composition further comprises a second therapeutic agent selected from the group consisting of a cytokine, an antibody, a chemotherapeutic agent, and a small molecule drug.

In a sixth aspect, the present disclosure provides a conjugate comprising the antibody or antigen-binding fragment thereof of the first aspect of the present disclosure and a chemical moiety conjugated thereto. In some embodiments of the conjugate disclosed herein, the chemical moiety conjugated to the antibody or antigen-binding fragment is selected from the group consisting of a therapeutic agent, a detectable moiety, and an immunostimulatory molecule.

In a seventh aspect, the present disclosure provides use of the antibody or antigen-binding fragment thereof of the first aspect of the present disclosure, the nucleic acid of the second aspect of the present disclosure, the vector of the third aspect of the present disclosure, or the host cell of the fourth aspect of the present disclosure, the pharmaceutical composition of the fifth aspect of the present disclosure, or the conjugate of the sixth aspect of the present disclosure in the manufacture of a medicament for treating cancer in a subject. In some embodiments, the cancer is a B7-H3 positive cancer. In some embodiments of the use disclosed herein, the cancer is selected from the group consisting of: adrenal tumor, AIDS-related cancer, alveolar soft part sarcoma, astrocytoma, adrenal cancer, bladder cancer, bone cancer, brain and spinal cord cancer, metastatic brain tumor, B-cell cancer, breast cancer, carotid body tumor, cervical cancer, chondrosarcoma, chordoma, chromophobe renal cell carcinoma, clear cell carcinoma, colon cancer, colorectal cancer, cutaneous benign fibrous histiocytoma, desmoplastic small round cell tumor, ependymoma, Ewing's tumor, extraskeletal myxoid chondrosarcoma, incomplete bone fibrogenesis, fibrous dysplasia of bone, gallbladder or bile duct cancer, gastric cancer, gestational trophoblast disease, germ cell tumor, head and neck cancer, hepatocellular carcinoma, islet cell tumor, Kaposi's sarcoma, kidney cancer, leukemia, liposarcoma/malignant lipomatous tumor, liver cancer, lymphoma, lung cancer, medulloblastoma, melanoma, meningioma, multiple endocrine neoplasia, multiple myeloma, myelodysplastic syndrome, neuroblastoma, neuroendocrine tumor, ovarian cancer, pancreatic cancer, papillary thyroid carcinoma, parathyroid tumor, pediatric cancer, peripheral nerve sheath tumor, pheochromocytoma, pituitary tumor, prostate cancer, posterious uveal melanoma, renal metastatic cancer, rhabdoid tumor, rhabdomyosarcoma, sarcoma, skin cancer, soft tissue sarcoma, squamous cell carcinoma, gastric carcinoma, synovial sarcoma, testicular carcinoma, thymic carcinoma, thymoma, metastatic thyroid cancer, and uterine cancer. In some embodiments of the use disclosed herein, the medicament is in combination with a second therapeutic agent. In some embodiments of the uses disclosed herein, the second therapeutic agent is selected from the group consisting of a cytokine, an antibody, a chemotherapeutic agent, and a small molecule drug.

In an eighth aspect, the present disclosure provides a method of treating cancer in a subject, comprising administering to the subject an effective amount of the antibody or antigen-binding fragment thereof of the first aspect of the present disclosure, the nucleic acid of the second aspect of the present disclosure, the vector of the third aspect of the present disclosure, or the host cell of the fourth aspect of the present disclosure, the pharmaceutical composition of the fifth aspect of the present disclosure, or the conjugate of the sixth aspect of the present disclosure. In some embodiments, the cancer is a B7-H3 positive cancer. In some embodiments of the use disclosed herein, the cancer is selected from the group consisting of: adrenal tumor, AIDS-related cancer, alveolar soft part sarcoma, astrocytoma, adrenal cancer, bladder cancer, bone cancer, brain and spinal cord cancer, metastatic brain tumor, B-cell cancer, breast cancer, carotid body tumor, cervical cancer, chondrosarcoma, chordoma, chromophobe renal cell carcinoma, clear cell carcinoma, colon cancer, colorectal cancer, cutaneous benign fibrous histiocytoma, desmoplastic small round cell tumor, ependymoma, Ewing's tumor, extraskeletal myxoid chondrosarcoma, incomplete bone fibrogenesis, fibrous dysplasia of bone, gallbladder or bile duct cancer, gastric cancer, gestational trophoblast disease, germ cell tumor, head and neck cancer, hepatocellular carcinoma, islet cell tumor, Kaposi's sarcoma, kidney cancer, leukemia, liposarcoma/malignant lipomatous tumor, liver cancer, lymphoma, lung cancer, medulloblastoma, melanoma, meningioma, multiple endocrine neoplasia, multiple myeloma, myelodysplastic syndrome, neuroblastoma, neuroendocrine tumor, ovarian cancer, pancreatic cancer, papillary thyroid carcinoma, parathyroid tumor, pediatric cancer, peripheral nerve sheath tumor, pheochromocytoma, pituitary tumor, prostate cancer, posterious uveal melanoma, renal metastatic cancer, rhabdoid tumor, rhabdomyosarcoma, sarcoma, skin cancer, soft tissue sarcoma, squamous cell carcinoma, gastric carcinoma, synovial sarcoma, testicular carcinoma, thymic carcinoma, thymoma, metastatic thyroid cancer, and uterine cancer. In some embodiments of the use disclosed herein, the medicament is in combination with a second therapeutic agent. In some embodiments of the uses disclosed herein, the second therapeutic agent is selected from the group consisting of a cytokine, an antibody, a chemotherapeutic agent, and a small molecule drug.

In a ninth aspect, the present disclosure provides the antibody or antigen-binding fragment thereof of the first aspect of the present disclosure, the nucleic acid of the second aspect of the present disclosure, the vector of the third aspect of the present disclosure, or the host cell of the fourth aspect of the present disclosure, the pharmaceutical composition of the fifth aspect of the present disclosure, or the conjugate of the sixth aspect of the present disclosure, for use in treating cancer in a subject. In some embodiments, the cancer is a B7-H3 positive cancer. In some embodiments of the use disclosed herein, the cancer is selected from the group consisting of: adrenal tumor, AIDS-related cancer, lveolar soft part sarcoma, astrocytoma, adrenal cancer, bladder cancer, bone cancer, brain and spinal cord cancer, metastatic brain tumor, B-cell cancer, breast cancer, carotid body tumor, cervical cancer, chondrosarcoma, chordoma, chromophobe renal cell carcinoma, clear cell carcinoma, colon cancer, colorectal cancer, cutaneous benign fibrous histiocytoma, desmoplastic small round cell tumor, ependymoma, Ewing's tumor, extraskeletal myxoid chondrosarcoma, incomplete bone fibrogenesis, fibrous dysplasia of bone, gallbladder or bile duct cancer, gastric cancer, gestational trophoblast disease, germ cell tumor, head and neck cancer, hepatocellular carcinoma, islet cell tumor, Kaposi's sarcoma, kidney cancer, leukemia, liposarcoma/malignant lipomatous tumor, liver cancer, lymphoma, lung cancer, medulloblastoma, melanoma, meningioma, multiple endocrine neoplasia, multiple myeloma, myelodysplastic syndrome, neuroblastoma, neuroendocrine tumor, ovarian cancer, pancreatic cancer, papillary thyroid carcinoma, parathyroid tumor, pediatric cancer, peripheral nerve sheath tumor, pheochromocytoma, pituitary tumor, prostate cancer, posterious uveal melanoma, renal metastatic cancer, rhabdoid tumor, rhabdomyosarcoma, sarcoma, skin cancer, soft tissue sarcoma, squamous cell carcinoma, gastric carcinoma, synovial sarcoma, testicular carcinoma, thymic carcinoma, thymoma, metastatic thyroid cancer, and uterine cancer. In some embodiments of the use disclosed herein, the medicament is in combination with a second therapeutic agent. In some embodiments of the uses disclosed herein, the second therapeutic agent is selected from the group consisting of a cytokine, an antibody, a chemotherapeutic agent, and a small molecule drug.

### BRIEF DESCRIPTION OF THE DRAWINGS

An understanding of the features and advantages of the present disclosure may be obtained by reference to the following detailed description as illustrated in exemplary embodiments utilizing the principles of the present disclosure and the accompanying drawings thereof:
FIG. 1 shows the binding ability of chimeric antibodies to CHO-S-human-B7-H3 cells (FIG. 1A), CHO-S-Cyno-B7-H3 cells (FIG. 1B), and CHO-S-mouse-B7-H3 (FIG. 1C) and CHO-S cells (FIG. 1D).
FIG. 2 shows the endocytosis efficiency of chimeric antibodies in Calu-6 cells.
FIG. 3 shows the endocytosis efficiency of chimeric antibodies in NCI-H446 cells.
FIG. 4 shows the internalization ability of humanized antibodies in Calu-6 cells (FIG.
4B) and NCI-H446 cells (FIG. 4A).
FIG. 5 shows the binding activity of humanized antibodies cAb1-z0-p7 (FIG. 5A), cAb1-z3-p7 (FIG. 5B), and cAb1-z3-p7 (FIG. 5C) to B7 family proteins PD-L1, B7-H2, B7-H4, B7-H5, B7-H6, B7-1, B7-2, PD-L2, B7-H7, and Human B7-H3-His.

### DETAILED DESCRIPTION

The foregoing features and advantages of the present disclosure, as well as additional features and advantages thereof, will be more clearly understood hereinafter in combination with the accompanying drawings and the following detailed description of embodiments.

The embodiments described herein with reference to the accompanying drawings are illustrative and exemplary, and are intended to generally understand the present disclosure. The embodiments should not be construed as limiting the scope of the present disclosure. The same or similar elements and elements having the same or similar functions are denoted by the same reference numerals throughout the specification.

Unless otherwise indicated or defined, all terms used have ordinary meanings in the art, which are clear to those skilled in the art. For example, reference is made to standard manuals such as Leuenberger, H.G.W, Nagel, B., and Klbl, H. eds., "A mµltilingual glossary of biotechnological terms: (IUPAC Recommendations)", Helvetica Chimica Acta (1995), CH-4010 Basel, Switzerland; Sambrook et al., "Molecµlar Cloning: A Laboratory Manual" (2nd Ed.), Vols. 1-3, Cold Spring Harbor Laboratory Press (1989); F. Ausubel et al., eds., "Current protocols in molecµlar biology", Green Publishing and Wiley InterScience, New York (1987); Roitt et al., "Immunology (6th Ed.), Mosby/Elsevier, Edinburgh (2001); and Janeway et al., "Immunobiology" (6th Ed.), Garland Science Publishing/Churchill Livingstone, New York (2005), as well as the general background art cited above.

### Definition

As used herein, the singular forms "a", "an", and "the" include plural objects unless clearly dictated otherwise in the context. Thus, for example, reference to "an antibody" includes a plurality of antibodies, and in some embodiments, reference to "an antibody" includes a plurality of antibodies, and the like.

Unless otherwise stated or defined, the term "comprise" and variations thereof, such as "include" and "contain," should be understood to mean the inclusion of the recited elements or steps or groups of elements or groups of steps, but not the exclusion of any other elements or steps or groups of elements or groups of steps.

As used herein, the term "antibody" refers to an immunoglobulin molecule that has the ability to specifically bind to a particular antigen. Such a molecule typically comprises two heavy (H) chains and two light (L) chains interconnected by disulfide bonds. Each heavy chain consists of a heavy chain variable region (or domain) (abbreviated herein as VH) and a heavy chain constant region. The heavy chain constant region consists of three domains, CH1, CH2, and CH3. Each light chain consists of a light chain variable region (or domain) (abbreviated herein as VL) and a light chain constant region. The light chain constant region consists of one domain, CL. The variable regions of the antibody heavy and light chains contain binding domains that interact with an antigen. The constant regions of the antibody may mediate the binding of the immunoglobulin to a host tissue or factor, including various cells of the immune system (e.g., effector cells) and components of the complement system, such as C1q (the first component in the classical pathway of complement activation).

The heavy chain of an immunoglobulin can be divided into three functional regions: an Fd region, a hinge region, and an Fc region (fragment crystallizable). The Fd region comprises VH and CH1 domains and combines with the light chain to form a Fab (antigen-binding fragment). The Fc fragment is responsible for immunoglobulin effector functions, including, for example, binding to complement and binding to homologous Fc receptors of effector cells. The hinge region found in the IgG, IgA, and IgD immunoglobulin classes acts as a flexible spacer, allowing the Fab portion to move freely in space relative to the Fc region. Hinge domains are structurally diverse, differing in both sequence and length among immunoglobulin classes and subclasses.

Based on crystallographic studies, the immunoglobulin hinge region can be further subdivided structurally and functionally into three regions: an upper hinge, a core hinge, and a lower hinge. The upper hinge includes amino acids from the carboxyl terminus of CH1 to the first residue in the hinge that restricts movement, typically the first cysteine residue that forms an interchain disulfide bond between the two heavy chains. The length of the upper hinge region correlates with the segmental flexibility of the antibody. The core hinge region contains inter-heavy chain disulfide bonds. The lower hinge region connects to the amino terminus of the CH2 domain and includes residues within the CH2 domain. The structure and flexibility of the immunoglobulin hinge region polypeptide sequence allow conformational changes, which may affect the effector function of the Fc portion of the antibody.

A "light chain variable region" (VL) or a "heavy chain variable region" (VH) consists of "framework" regions interrupted by three "complementarity determining regions" or "CDRs". The framework regions serve to align the CDRs that specifically bind to an antigenic epitope. CDRs include the amino acid residues of an antibody that are primarily responsible for antigen binding. Both the VL domain and the VH domain, from the amino terminus to the carboxy terminus, comprise the following framework regions (FRs) and CDR regions: FR1, CDR1, FR2, CDR2, FR3, CDR3, and FR4. CDR1, CDR2, and CDR3 of the VL domain are also referred to herein as LCDR1, LCDR2, and LCDR3, respectively; CDR1, CDR2, and CDR3 of the VH domain are also referred to herein as HCDR1, HCDR2, and HCDR3, respectively.

The amino acid arrangement of each VL domain and VH domain is consistent with any conventional definition of CDRs. Conventional definitions include Kabat definition (Kabat, Sequences of Proteins of Immunological Interest (National Institutes of Health, Bethesda, MD, 1987 and 1991)), Chothia definition (Chothia and Lesk, J. Mol. Biol. 196:901-917, 1987; Chothia et al., Nature 342:878-883, 1989); Composite of Chothia Kabat CDR, where CDR-H1 is a composite of Chothia CDR and Kabat CDR; AbM definition used by antibody modeling software of Oxford Molecµlar; and the CONTACT definition by Martin et al. (world wide web bioinfo.org.uk/abs). Kabat provides a widely used numbering convention (Kabat numbering system) in which corresponding residues between different heavy chains or between different light chains are assigned the same numbering. The present disclosure may use CDRs defined according to any of these numbering systems, but preferred embodiments use CDRs defined by Kabat.

Based on the amino acid sequence of the antibody heavy chain constant region, immunoglobulin molecules can be classified into five classes (isotypes): IgA, IgD, IgE, IgG, and IgM, and can be further divided into different subtypes, such as IgG1, IgG2, IgG3, IgG4, IgA1, IgA2, etc. Based on the amino acid sequence of the light chain, the light chain of an antibody can be classified into lambda (λ) chain and kappa (x) chain.

As used herein, the term "antibody" should be understood in its broadest sense and includes monoclonal antibodies (including full-length monoclonal antibodies), polyclonal antibodies, antibody fragments, and multispecific antibodies (e.g., bispecific antibodies) containing at least two antigen-binding regions. Antibodies may contain an additional modification, such as a non-naturally occurring amino acid, a mutation in the Fc region, and a mutation in a glycosylation site. Antibodies also include post-translationally modified antibodies, fusion proteins containing an antigenic determinant of an antibody, and immunoglobulin molecules containing any other modification of the antigen recognition site, as long as these antibodies exhibit the desired biological activity.

As used herein, the term "monoclonal antibody" refers to an antibody obtained from a substantially homogeneous population of antibodies. That is, the individual antibodies comprising the population are identical except for a small number of possible naturally occurring mutations. Monoclonal antibodies are highly specific and are directed against a single antigen. The term "monoclonal antibody" herein is not limited to an antibody produced by hybridoma technology, nor should it be construed as requiring an antibody produced by any particular method.

The term "bispecific antibody" is to be understood in the context of the present disclosure as an antibody having two different antigen binding regions defined by different antibody sequences. This can be understood as binding to different targets, but also includes binding to different epitopes on one target. The term "bispecific antibody" as used herein is to be understood in its broadest meaning, including full-length bispecific antibodies and antigen-binding fragments thereof. Bispecific antibodies may contain an additional modification, such as a non-naturally occurring amino acid, a mutation in the Fc region, and a mutation in a glycosylation site. Bispecific antibodies also include post-translationally modified antibodies, fusion proteins containing an antigenic determinant of an antibody, and immunoglobulin molecules containing any other modification of the antigen recognition site, as long as the antibodies exhibit the desired biological activity.

As used herein, the term "antigen-binding fragment" of an antibody refers to one or more fragments of an antibody that retain the ability to specifically bind to an antigen. It has been shown that the antigen-binding function of an antibody can be performed by fragments of a full-length antibody.

Examples of antigen-binding fragments encompassed within the term "antigen-binding portion" of an antibody encompass: (i) a Fab fragment, a monovalent fragment consisting of VL, VH, CL and CH1 domains; (ii) an F(ab')₂ fragment, a bivalent fragment comprising two Fab fragments linked by disulfide bond in the hinge region; (iii) a Fab' fragment, which is essentially a Fab with a partial hinge region; (iv) an Fd fragment, consisting of a VH and a CH1 domain; (v) an Fd 'fragment, having a VH and a CH1 domain and one or more cysteine residues at the C-terminus of the CH1 domain; (vi) an Fv fragment, consisting of a VL and a VH domain of single arm of an antibody; (vii) a dAb fragment, consisting of a VH domain; (viii) an isolated complementarity determining region (CDR); (ix) a Nanobody, a heavy chain variable region containing a single variable domain and two constant domains. Furthermore, although the two domains of the Fv fragment, VL and VH, are encoded by separate genes, they can be joined using recombinant methods by synthetic linkers, enabling them to be made as a single protein chain in which the VL and VH regions pair to form a monovalent molecule (referred to as a single-chain Fv (scFv)). Such a single-chain antibody is also intended to be encompassed within the term "antigen-binding fragment" of an antibody. In addition, the term also includes a "linear antibody" comprising a pair of tandem Fd fragments (VH-CH1-VH-CH1) that form an antigen-binding region together with a complementary light chain polypeptide, as well as modified versions of any of the foregoing fragments that retain antigen-binding activity.

These antigen-binding fragments can be obtained using conventional techniques known to those skilled in the art, and the fragments are screened for utility in the same manner as intact antibodies.

As used herein, the term "binding" or "specifically binding" refers to a non-random binding reaction between two molecules, such as between an antibody and its target antigen. The binding specificity of an antibody can be determined based on affinity and/or avidity. Affinity represents the equilibrium constant of dissociation of an antigen from an antibody (KD), which is a measure of the binding strength between an antigenic determinant and an antigen binding site on the antibody: the smaller the value of KD, the stronger the binding strength between the antigenic determinant and the antibody. Alternatively, affinity may also be expressed as an affinity constant (KA), which is 1/KD.

Avidity is a measure of the binding strength between an antibody and its associated antigen. Avidity involves both the affinity between the antigenic determinant and the antigen binding site on the antibody, as well as the number of relevant binding sites present on the antibody. Typically, the antibody will bind to the antigen with a dissociation constant (KD) of: 10⁻⁵ M to 10⁻¹² M or less, and preferably 10⁻⁷ M to 10⁻¹² M or less, and more preferably 10⁻⁸ M to 10⁻¹² M, and/or with the following binding affinity: at least 10⁷ M⁻¹, preferably at least 10⁸ M⁻¹, more preferably at least 10⁹ M⁻¹, such as at least 10¹² M⁻¹. Any K_{D} value greater than 10⁻⁴ M is generally considered to indicate non-specific binding. The specific binding of an antibody to an antigen or antigenic determinant can be determined in any known suitable manner, including, for example, Scatchard analysis and/or competitive binding assays such as radioimmunoassays (RIA), enzyme immunoassays (EIA), and sandwich competition assays, as well as various variations thereof known in the art.

The term "epitope" refers to a site on an antigen to which an antibody binds. An epitope can be formed from contiguous amino acids or from non-contiguous amino acids juxtaposed by one or more tertiary folding of a protein. Epitopes formed from contiguous amino acids (also referred to as linear epitopes) are typically retained upon exposure to denaturing solvents, while epitopes formed by tertiary folding (also referred to as conformational epitopes) are typically lost upon treatment with denaturing solvents. An Epitope typically comprises at least 3, more typically at least 5 or 8-10 amino acids in a unique spatial conformation. The epitope defines the minimum binding site for an antibody and is therefore a specific target for an antibody or antigen-binding fragment thereof.

As used herein, the term "sequence identity" refers to the extent to which two sequences (amino acids) have the same residue at the same position after alignment. For example, "an amino acid sequence is X% identical to SEQ ID NO: Y" means that the amino acid sequence is X% identical to SEQ ID NO: Y, and is stated as that X% of the residues in the amino acid sequence are identical to the residues of the sequence disclosed in SEQ ID NO: Y. Typically, such calculations are performed using computer programs. Exemplary programs for comparing and aligning sequence pairs include ALIGN (Myers and Miller, 1988), FASTA (Pearson and Lipman, 1988; Pearson, 1990), and gapped BLAST (Altschul et al., 1997), BLASTP, BLASTN, or GCG (Devereux et al., 1984).

Furthermore, when determining the degree of sequence identity between two amino acid sequences, the skilled person may take into account so-called "conservative" amino acid substitutions, which may generally be described as amino acid substitutions in which one amino acid residue is replaced with another amino acid residue having a similar chemical structure, and which have little or substantially no effect on the function, activity or other biological properties of the polypeptide. Such conservative amino acid substitutions are well known in the art.

Such conservative substitutions are preferably substitutions in which one amino acid in the following groups (a) to (e) is substituted by another amino acid residue in the same group: (a) small aliphatic, non-polar or weakly polar residues: Ala, Ser, Thr, Pro and Gly; (b) polar, negatively charged residues and their (uncharged) amides: Asp, Asn, Glu, and Gln; (c) polar, positively charged residues: His, Arg, and Lys; (d) large aliphatic, non-polar residues: Met, Leu, Ile, Val, and Cys; and (e) aromatic residues: Phe, Tyr, and Trp.

Particularly preferred conservative substitutions are as follows: Ala to Gly, or to Ser; Arg to Lys; Asn to Gln, or to His; Asp to Glu; Cys to Ser; Gln to Asn; Glu to Asp; Gly to Ala, or to Pro; His to Asn, or to Gln; Ile to Leu, or to Val; Leu to Ile, or to Val; Lys to Arg, to Gln, or to Glu; Met to Leu, to Tyr, or to Ile; Phe to Met, to Leu, or to Tyr; Ser to Thr; Thr to Ser; Trp to Tyr; Tyr to Trp; and/or Phe to Val, to Ile, or to Leu.

As used herein, the term "tumor-associated antigen" refers to an antigen that is differentially expressed in cancer cells as compared to normal cells, and thus can be used to target cancer cells.

B7-H3, also known as B7 homolog 3, PSEC0249, UNQ309/PRO352, CD276, or B7H3, is a protein with a single-spanning transmembrane structure, a member of the B7 family expressed as a costimulatory molecule on antigen presenting cells. B7-H3 is believed to act on receptors on T cells to enhance or suppress immune activity. The human B7-H3 gene is located on chromosome 15, and the mouse B7-H3 gene is located on chromosome 9.

As used herein, the term "vector" is intended to refer to a nucleic acid molecule capable of transporting another nucleic acid to which it is linked.

As used herein, the term "host cell" refers to a cell into which an expression vector has been introduced.

The term "pharmaceutically acceptable" means that the carrier or excipient is compatible with the other ingredients of the composition and is substantially non-toxic to the recipient thereof, and/or that such carrier or excipient is approved or useful for inclusion in pharmaceutical compositions for parenteral administration to humans.

As used herein, the terms "treating", "therapy", "treatment", and the like refer to the administration of an agent or the performing of a procedure for the purpose of obtaining an effect. These effects may be prophylactic in terms of complete or partial prevention of a disease or a symptom thereof, and/or may be therapeutic in terms of effecting partial or complete cure of a disease and/or a symptom thereof. As used herein, "treating" may include treating a disease or disorder (e.g., cancer) in a mammal, particularly a human, and comprises: (a) preventing the occurrence of a disease (e.g., including a disease that may be associated with or caused by a primary disease) or a symptom in a subject who is susceptible to while has not been diagnosed with the disease; (b) inhibiting a disease, i.e. preventing its development; and (c) relieving a disease, i.e. causing regression of the disease. Treatment may refer to any indicator of success in the treatment, amelioration, or prevention of a cancer, including any objective or subjective parameter, such as reducing symptoms; remission; eliminating the symptoms of the disease or making the disease condition more tolerable to the patient; slowing down the rate of deterioration or decline; or making the final point of deterioration less debilitating. Treatment or amelioration of symptoms is based on one or more objective or subjective parameters, including the results of the doctor's examination. Accordingly, the term "treatment" includes administration of the antibody or the composition or the conjugate disclosed herein to prevent or delay, alleviate, or arrest, or inhibit the development of a symptom or condition associated with a disease (e.g., cancer). The term "therapeutic effect" refers to a reduction, elimination, or prevention of a disease, a symptom of the disease, or a side effect of the disease in a subject.

As used herein, the term "effective amount" refers to an amount sufficient to achieve the treatment of a disease when administered to a subject.

As used herein, the term "subject" refers to any mammalian subject for which diagnosis, treatment, or therapy is desired. "Mammal" for therapeutic purposes refers to any animal classified as a mammal, including human, domestic animals, as well as laboratory animals, zoo animals, sports animals, or pet animals, such as dogs, horses, cats, cows, sheep, goats, pigs, mice, rats, rabbits, guinea pigs, monkeys, and the like.

### Anti-B7-H3 Antibodies

The present disclosure provides an antibody that specifically binds to B7-H3 or an antigen-binding fragment thereof, comprising a light chain variable region (VL) and a heavy chain variable region (VH).

In a first aspect, the present disclosure provides an antibody that specifically binds to B7-H3 or an antigen-binding fragment thereof, wherein the antibody comprises a heavy chain variable region (VH) and a light chain variable region (VL).

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 3, and the VL comprises LCDR1, LCDR2, and LCDR3 in a light chain variable region having the amino acid sequence as set forth in SEQ ID NO: 13.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 26, and the VL comprises LCDR1, LCDR2, and LCDR3 in a light chain variable region having the amino acid sequence as set forth in SEQ ID NO: 27.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 28, and the VL comprises LCDR1, LCDR2, and LCDR3 in a light chain variable region having the amino acid sequence as set forth in SEQ ID NO: 29.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 30, and the VL comprises LCDR1, LCDR2, and LCDR3 in a light chain variable region having the amino acid sequence as set forth in SEQ ID NO: 29.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 22, and the VL comprises LCDR1, LCDR2, and LCDR3 in a light chain variable region having the amino acid sequence as set forth in SEQ ID NO: 23.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 33, and the VL comprises LCDR1, LCDR2, and LCDR3 in a light chain variable region having the amino acid sequence as set forth in SEQ ID NO: 42.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 34, and the VL comprises LCDR1, LCDR2, and LCDR3 in a light chain variable region having the amino acid sequence as set forth in SEQ ID NO: 42.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 33, and the VL comprises LCDR1, LCDR2, and LCDR3 in a light chain variable region having the amino acid sequence as set forth in SEQ ID NO: 41.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 34, and the VL comprises LCDR1, LCDR2, and LCDR3 in a light chain variable region having the amino acid sequence as set forth in SEQ ID NO: 39.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 34, and the VL comprises LCDR1, LCDR2, and LCDR3 in a light chain variable region having the amino acid sequence as set forth in SEQ ID NO: 40.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 34, and the VL comprises LCDR1, LCDR2, and LCDR3 in a light chain variable region having the amino acid sequence as set forth in SEQ ID NO: 41.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 1, and the VL comprises LCDR1, LCDR2, and LCDR3 in a light chain variable region having the amino acid sequence as set forth in SEQ ID NO: 2.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 3, and the VL comprises LCDR1, LCDR2, and LCDR3 in a light chain variable region having the amino acid sequence as set forth in SEQ ID NO: 4.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 5, and the VL comprises LCDR1, LCDR2, and LCDR3 in a light chain variable region having the amino acid sequence as set forth in SEQ ID NO: 6.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 7, and the VL comprises LCDR1, LCDR2, and LCDR3 in a light chain variable region having the amino acid sequence as set forth in SEQ ID NO: 8.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 9, and the VL comprises LCDR1, LCDR2, and LCDR3 in a light chain variable region having the amino acid sequence as set forth in SEQ ID NO: 10.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 11, and the VL comprises LCDR1, LCDR2, and LCDR3 in a light chain variable region having the amino acid sequence as set forth in SEQ ID NO: 12.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 14, and the VL comprises LCDR1, LCDR2, and LCDR3 in a light chain variable region having the amino acid sequence as set forth in SEQ ID NO: 15.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 16, and the VL comprises LCDR1, LCDR2, and LCDR3 in a light chain variable region having the amino acid sequence as set forth in SEQ ID NO: 17.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 18, and the VL comprises LCDR1, LCDR2, and LCDR3 in a light chain variable region having the amino acid sequence as set forth in SEQ ID NO: 19.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 20, and the VL comprises LCDR1, LCDR2, and LCDR3 in a light chain variable region having the amino acid sequence as set forth in SEQ ID NO: 21.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1, HCDR2, and HCDR3 in a heavy chain variable region having the amino acid sequence as set forth in SEQ ID NO: 24, and the VL comprises LCDR1, LCDR2, and LCDR3 in a light chain variable region having the amino acid sequence as set forth in SEQ ID NO: 25.

In some embodiments, the CDR sequences are defined according to the Kabat numbering system.

In some embodiments of the antibody or antigen-binding fragment thereof disclosed herein, the B7-H3 antibody or antigen-binding fragment thereof comprises a heavy chain variable region (VH) and a light chain variable region (VL), and when the CDR sequences are defined according to the Kabat numbering system, the VH comprises HCDR1 as set forth in SEQ ID NO: 84, HCDR2 as set forth in SEQ ID NO: 86, and HCDR3 as set forth in SEQ ID NO: 88, and the VL comprises LCDR1 as set forth in SEQ ID NO: 173, LCDR2 as set forth in SEQ ID NO: 175, and LCDR3 as set forth in SEQ ID NO: 177.

In some embodiments of the antibody or antigen-binding fragment thereof disclosed herein, the B7-H3 antibody or antigen-binding fragment thereof comprises a heavy chain variable region (VH) and a light chain variable region (VL), and when the CDR sequences are defined according to the Kabat numbering system, the VH comprises HCDR1 as set forth in SEQ ID NO: 84, HCDR2 as set forth in SEQ ID NO: 86, and HCDR3 as set forth in SEQ ID NO: 126, and the VL comprises LCDR1 as set forth in SEQ ID NO: 213, LCDR2 as set forth in SEQ ID NO: 175, and LCDR3 as set forth in SEQ ID NO: 177.

In some embodiments of the antibody or antigen-binding fragment thereof disclosed herein, the B7-H3 antibody or antigen-binding fragment thereof comprises a heavy chain variable region (VH) and a light chain variable region (VL), and when the CDR sequences are defined according to the Kabat numbering system, the VH comprises HCDR1 as set forth in SEQ ID NO: 111, HCDR2 as set forth in SEQ ID NO: 113, and HCDR3 as set forth in SEQ ID NO: 115, and the VL comprises LCDR1 as set forth in SEQ ID NO: 201, LCDR2 as set forth in the amino acid sequence NAK, and LCDR3 as set forth in SEQ ID NO: 205.

In some embodiments of the antibody or antigen-binding fragment thereof disclosed herein, the B7-H3 antibody or antigen-binding fragment thereof comprises a heavy chain variable region (VH) and a light chain variable region (VL), and when the CDR sequences are defined according to the Kabat numbering system, the VH comprises HCDR1 as set forth in SEQ ID NO: 111, HCDR2 as set forth in SEQ ID NO: 113, and HCDR3 as set forth in SEQ ID NO: 115, and the VL comprises LCDR1 as set forth in SEQ ID NO: 201, LCDR2 as set forth in the amino acid sequence NAK, and LCDR3 as set forth in SEQ ID NO: 203.

In some embodiments of the antibody or antigen-binding fragment thereof disclosed herein, the B7-H3 antibody or antigen-binding fragment thereof comprises a heavy chain variable region (VH) and a light chain variable region (VL), and when the CDR sequences are defined according to the Kabat numbering system, the VH comprises HCDR1 as set forth in SEQ ID NO: 44, HCDR2 as set forth in SEQ ID NO: 46, and HCDR3 as set forth in SEQ ID NO: 48, and the VL comprises LCDR1 as set forth in SEQ ID NO: 135, LCDR2 as set forth in the amino acid sequence YAS, and LCDR3 as set forth in SEQ ID NO: 139.

In some embodiments of the antibody or antigen-binding fragment thereof disclosed herein, the B7-H3 antibody or antigen-binding fragment thereof comprises a heavy chain variable region (VH) and a light chain variable region (VL), and when the CDR sequences are defined according to the Kabat numbering system, the VH comprises HCDR1 as set forth in SEQ ID NO: 51, HCDR2 as set forth in SEQ ID NO: 53, and HCDR3 as set forth in SEQ ID NO: 55, and the VL comprises LCDR1 as set forth in SEQ ID NO: 142, LCDR2 as set forth in the amino acid sequence YAS, and LCDR3 as set forth in SEQ ID NO: 145.

In some embodiments of the antibody or antigen-binding fragment thereof disclosed herein, the B7-H3 antibody or antigen-binding fragment thereof comprises a heavy chain variable region (VH) and a light chain variable region (VL), and when the CDR sequences are defined according to the Kabat numbering system, the VH comprises HCDR1 as set forth in SEQ ID NO: 58, HCDR2 as set forth in SEQ ID NO: 60, and HCDR3 as set forth in SEQ ID NO: 62, and the VL comprises LCDR1 as set forth in SEQ ID NO: 148, LCDR2 as set forth in the amino acid sequence KVS, and LCDR3 as set forth in SEQ ID NO: 152.

In some embodiments of the antibody or antigen-binding fragment thereof disclosed herein, the B7-H3 antibody or antigen-binding fragment thereof comprises a heavy chain variable region (VH) and a light chain variable region (VL), and when the CDR sequences are defined according to the Kabat numbering system, the VH comprises HCDR1 as set forth in SEQ ID NO: 65, HCDR2 as set forth in SEQ ID NO: 67, and HCDR3 as set forth in SEQ ID NO: 69, and the VL comprises LCDR1 as set forth in SEQ ID NO: 154, LCDR2 as set forth in the amino acid sequence RMS, and LCDR3 as set forth in SEQ ID NO: 158.

In some embodiments of the antibody or antigen-binding fragment thereof disclosed herein, the B7-H3 antibody or antigen-binding fragment thereof comprises a heavy chain variable region (VH) and a light chain variable region (VL), and when the CDR sequences are defined according to the Kabat numbering system, the VH comprises HCDR1 as set forth in SEQ ID NO: 72, HCDR2 as set forth in SEQ ID NO: 74, and HCDR3 as set forth in SEQ ID NO: 76, and the VL comprises LCDR1 as set forth in SEQ ID NO: 160, LCDR2 as set forth in the amino acid sequence GAS, and LCDR3 as set forth in SEQ ID NO: 164.

In some embodiments of the antibody or antigen-binding fragment thereof disclosed herein, the B7-H3 antibody or antigen-binding fragment thereof comprises a heavy chain variable region (VH) and a light chain variable region (VL), and when the CDR sequences are defined according to the Kabat numbering system, the VH comprises HCDR1 as set forth in SEQ ID NO: 79, HCDR2 as set forth in SEQ ID NO: 81, and HCDR3 as set forth in SEQ ID NO: 83, and the VL comprises LCDR1 as set forth in SEQ ID NO: 166, LCDR2 as set forth in the amino acid sequence SGS, and LCDR3 as set forth in SEQ ID NO: 170.

In some embodiments of the antibody or antigen-binding fragment thereof disclosed herein, the B7-H3 antibody or antigen-binding fragment thereof comprises a heavy chain variable region (VH) and a light chain variable region (VL), and when the CDR sequences are defined according to the Kabat numbering system, the VH comprises HCDR1 as set forth in SEQ ID NO: 90, HCDR2 as set forth in SEQ ID NO: 92, and HCDR3 as set forth in SEQ ID NO: 94, and the VL comprises LCDR1 as set forth in SEQ ID NO: 179, LCDR2 as set forth in the amino acid sequence FAS, and LCDR3 as set forth in SEQ ID NO: 183.

In some embodiments of the antibody or antigen-binding fragment thereof disclosed herein, the B7-H3 antibody or antigen-binding fragment thereof comprises a heavy chain variable region (VH) and a light chain variable region (VL), and when the CDR sequences are defined according to the Kabat numbering system, the VH comprises HCDR1 as set forth in SEQ ID NO: 96, HCDR2 as set forth in SEQ ID NO: 98, and HCDR3 as set forth in SEQ ID NO: 100, and the VL comprises LCDR1 as set forth in SEQ ID NO: 185, LCDR2 as set forth in the amino acid sequence KVS, and LCDR3 as set forth in SEQ ID NO: 186.

In some embodiments of the antibody or antigen-binding fragment thereof disclosed herein, the B7-H3 antibody or antigen-binding fragment thereof comprises a heavy chain variable region (VH) and a light chain variable region (VL), and when the CDR sequences are defined according to the Kabat numbering system, the VH comprises HCDR1 as set forth in SEQ ID NO: 101, HCDR2 as set forth in SEQ ID NO: 102, and HCDR3 as set forth in SEQ ID NO: 104, and the VL comprises LCDR1 as set forth in SEQ ID NO: 188, LCDR2 as set forth in the amino acid sequence DTS, and LCDR3 as set forth in SEQ ID NO: 192.

In some embodiments of the antibody or antigen-binding fragment thereof disclosed herein, the B7-H3 antibody or antigen-binding fragment thereof comprises a heavy chain variable region (VH) and a light chain variable region (VL), wherein the VH comprises HCDR1 as set forth in SEQ ID NO: 79, HCDR2 as set forth in SEQ ID NO: 107, and HCDR3 as set forth in SEQ ID NO: 109, and the VL comprises LCDR1 as set forth in SEQ ID NO: 195, LCDR2 as set forth in WAS, and LCDR3 as set forth in SEQ ID NO: 199.

In some embodiments of the antibody or antigen-binding fragment thereof disclosed herein, the B7-H3 antibody or antigen-binding fragment thereof comprises a heavy chain variable region (VH) and a light chain variable region (VL), and when the CDR sequences are defined according to the Kabat numbering system, the VH comprises HCDR1 as set forth in SEQ ID NO: 117, HCDR2 as set forth in SEQ ID NO: 119, and HCDR3 as set forth in SEQ ID NO: 121, and the VL comprises LCDR1 as set forth in SEQ ID NO: 208, LCDR2 as set forth in DTS, and LCDR3 as set forth in SEQ ID NO: 211.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 3, and the VL comprises an amino acid sequence having at least 80%, at least 85%, at least 90%, at least 95%, at least 98%, at least 99% or 100% sequence identity to SEQ ID NO: 13. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 3, formed by insertion, deletion and/or substitution of one or more amino acids therein, and the VL comprises a functional variant of the amino acid sequence as set forth in SEQ ID NO: 13, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 26, and the VL comprises an amino acid sequence having at least 80%, at least 85%, at least 90%, at least 95%, at least 98%, at least 99% or 100% sequence identity to SEQ ID NO: 27. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 26, formed by insertion, deletion and/or substitution of one or more amino acids therein, and the VL comprises a functional variant of the amino acid sequence as set forth in SEQ ID NO: 27, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 28, and the VL comprises an amino acid sequence having at least 80%, at least 85%, at least 90%, at least 95%, at least 98%, at least 99% or 100% sequence identity to SEQ ID NO: 29. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 28, formed by insertion, deletion and/or substitution of one or more amino acids therein, and the VL comprises a functional variant of the amino acid sequence as set forth in SEQ ID NO: 29, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 30, and the VL comprises an amino acid sequence having at least 80%, at least 85%, at least 90%, at least 95%, at least 98%, at least 99% or 100% sequence identity to SEQ ID NO: 29. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 30, formed by insertion, deletion and/or substitution of one or more amino acids therein, and the VL comprises a functional variant of the amino acid sequence as set forth in SEQ ID NO: 29, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 22, and the VL comprises an amino acid sequence having at least 80%, at least 85%, at least 90%, at least 95%, at least 98%, at least 99% or 100% sequence identity to SEQ ID NO: 23. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 22, formed by insertion, deletion and/or substitution of one or more amino acids therein, and the VL comprises a functional variant of the amino acid sequence as set forth in SEQ ID NO: 23, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 33, and the VL comprises an amino acid sequence having at least 80%, at least 85%, at least 90%, at least 95%, at least 98%, at least 99% or 100% sequence identity to SEQ ID NO: 42. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 33, formed by insertion, deletion and/or substitution of one or more amino acids therein, and the VL comprises a functional variant of the amino acid sequence as set forth in SEQ ID NO: 42, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 34, and the VL comprises an amino acid sequence having at least 80%, at least 85%, at least 90%, at least 95%, at least 98%, at least 99% or 100% sequence identity to SEQ ID NO: 42. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 34, formed by insertion, deletion and/or substitution of one or more amino acids therein, and the VL comprises a functional variant of the amino acid sequence as set forth in SEQ ID NO: 42, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 33, and the VL comprises an amino acid sequence having at least 80%, at least 85%, at least 90%, at least 95%, at least 98%, at least 99% or 100% sequence identity to SEQ ID NO: 41. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 33, formed by insertion, deletion and/or substitution of one or more amino acids therein, and the VL comprises a functional variant of the amino acid sequence as set forth in SEQ ID NO: 41, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 34, and the VL comprises an amino acid sequence having at least 80%, at least 85%, at least 90%, at least 95%, at least 98%, at least 99% or 100% sequence identity to SEQ ID NO: 39. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 34, formed by insertion, deletion and/or substitution of one or more amino acids therein, and the VL comprises a functional variant of the amino acid sequence as set forth in SEQ ID NO: 39, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 34, and the VL comprises an amino acid sequence having at least 80%, at least 85%, at least 90%, at least 95%, at least 98%, at least 99% or 100% sequence identity to SEQ ID NO: 40. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 34, formed by insertion, deletion and/or substitution of one or more amino acids therein, and the VL comprises a functional variant of the amino acid sequence as set forth in SEQ ID NO: 40, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 34, and the VL comprises an amino acid sequence having at least 80%, at least 85%, at least 90%, at least 95%, at least 98%, at least 99% or 100% sequence identity to SEQ ID NO: 41. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 34, formed by insertion, deletion and/or substitution of one or more amino acids therein, and the VL comprises a functional variant of the amino acid sequence as set forth in SEQ ID NO: 41, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 1, and the VL comprises an amino acid sequence having at least 80%, at least 85%, at least 90%, at least 95%, at least 98%, at least 99% or 100% sequence identity to SEQ ID NO: 2. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 1, formed by insertion, deletion and/or substitution of one or more amino acids therein, and the VL comprises a functional variant of the amino acid sequence as set forth in SEQ ID NO: 2, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 3, and the VL comprises an amino acid sequence having at least 80%, at least 85%, at least 90%, at least 95%, at least 98%, at least 99% or 100% sequence identity to SEQ ID NO: 4. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 3, formed by insertion, deletion and/or substitution of one or more amino acids therein, and the VL comprises a functional variant of the amino acid sequence as set forth in SEQ ID NO: 4, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 5, and the VL comprises an amino acid sequence having at least 80%, at least 85%, at least 90%, at least 95%, at least 98%, at least 99% or 100% sequence identity to SEQ ID NO: 6. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 5, formed by insertion, deletion and/or substitution of one or more amino acids therein, and the VL comprises a functional variant of the amino acid sequence as set forth in SEQ ID NO: 6, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 7, and the VL comprises an amino acid sequence having at least 80%, at least 85%, at least 90%, at least 95%, at least 98%, at least 99% or 100% sequence identity to SEQ ID NO: 8. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 7, formed by insertion, deletion and/or substitution of one or more amino acids therein, and the VL comprises a functional variant of the amino acid sequence as set forth in SEQ ID NO: 8, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 9, and the VL comprises an amino acid sequence having at least 80%, at least 85%, at least 90%, at least 95%, at least 98%, at least 99% or 100% sequence identity to SEQ ID NO: 10. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 9, formed by insertion, deletion and/or substitution of one or more amino acids therein, and the VL comprises a functional variant of the amino acid sequence as set forth in SEQ ID NO: 10, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 11, and the VL comprises an amino acid sequence having at least 80%, at least 85%, at least 90%, at least 95%, at least 98%, at least 99% or 100% sequence identity to SEQ ID NO: 12. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 11, formed by insertion, deletion and/or substitution of one or more amino acids therein, and the VL comprises a functional variant of the amino acid sequence as set forth in SEQ ID NO: 12, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 14, and the VL comprises an amino acid sequence having at least 80%, at least 85%, at least 90%, at least 95%, at least 98%, at least 99% or 100% sequence identity to SEQ ID NO: 15. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 14, formed by insertion, deletion and/or substitution of one or more amino acids therein, and the VL comprises a functional variant of the amino acid sequence as set forth in SEQ ID NO: 15, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 16, and the VL comprises an amino acid sequence having at least 80%, at least 85%, at least 90%, at least 95%, at least 98%, at least 99% or 100% sequence identity to SEQ ID NO: 17. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 16, formed by insertion, deletion and/or substitution of one or more amino acids therein, and the VL comprises a functional variant of the amino acid sequence as set forth in SEQ ID NO: 17, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 18, and the VL comprises an amino acid sequence having at least 80%, at least 85%, at least 90%, at least 95%, at least 98%, at least 99% or 100% sequence identity to SEQ ID NO: 19. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 18, formed by insertion, deletion and/or substitution of one or more amino acids therein, and the VL comprises a functional variant of the amino acid sequence as set forth in SEQ ID NO: 19, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 20, and the VL comprises an amino acid sequence having at least 80%, at least 85%, at least 90%, at least 95%, at least 98%, at least 99% or 100% sequence identity to SEQ ID NO: 21. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 20, formed by insertion, deletion and/or substitution of one or more amino acids therein, and the VL comprises a functional variant of the amino acid sequence as set forth in SEQ ID NO: 21, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

In some embodiments of the antibody or antigen-binding fragment thereof disclosed herein, the B7-H3 antibody or antigen-binding fragment thereof comprises a heavy chain variable region (VH) and a light chain variable region (VL), and the VH comprises an amino acid sequence having at least 80%, at least 85%, at least 90%, at least 95%, at least 98%, at least 99%, or 100% sequence identity to SEQ ID NO: 24, and the VL comprises an amino acid sequence having at least 80%, at least 85%, at least 90%, at least 95%, at least 98%, at least 99% or 100% sequence identity to SEQ ID NO: 25. In some embodiments, the B7-H3 antibody or antigen-binding fragment thereof comprises a heavy chain variable region (VH) and a light chain variable region (VL), and the VH comprises a functional variant of the amino acid sequence as set forth in SEQ ID NO: 24, formed by insertion, deletion and/or substitution of one or more amino acids therein, and the VL comprises a functional variant of the amino acid sequence as set forth in SEQ ID NO: 25, formed by insertion, deletion and/or substitution of one or more amino acids therein, provided that the antibody or antigen-binding fragment thereof comprising the VH comprising the functional variant and the VL comprising the functional variant retains the ability to bind to B7-H3.

The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In the context of the functional variant, the number of amino acids inserted, deleted and/or substituted preferably is not more than 40%, more preferably not more than 35%, more preferably 1% to 33%, and more preferably 5% to 30%, more preferably 10% to 25%, more preferably 15% to 20% of the total number of amino acids in the parent amino acid sequence. For example, the number of amino acids inserted, deleted, and/or substituted may be 1 to 20, preferably 1 to 10, more preferably 1 to 7, even more preferably 1 to 5, and most preferably 1 to 2. In preferred embodiments, the number of amino acids inserted, deleted, and/or substituted is 1, 2, 3, 4, 5, 6, or 7.

In some embodiments, insertions, deletions, and/or substitutions may be made in the framework (FR) regions, such as in FR1, FR2, FR3, and/or FR4.

In some embodiments, the substitution of one or more amino acids may be a conservative substitution of one or more amino acids. Such conservative substitutions are preferably substitutions in which one amino acid in the following groups (a) to (e) is substituted by another amino acid residue in the same group: (a) small aliphatic, non-polar or weakly polar residues: Ala, Ser, Thr, Pro and Gly; (b) polar, negatively charged residues and their (uncharged) amides: Asp, Asn, Glu, and Gln; (c) polar, positively charged residues: His, Arg, and Lys; (d) large aliphatic, non-polar residues: Met, Leu, Ile, Val, and Cys; and (e) aromatic residues: Phe, Tyr, and Trp.

Particularly preferred conservative substitutions are as follows: Ala to Gly, or to Ser; Arg to Lys; Asn to Gln, or to His; Asp to Glu; Cys to Ser; Gln to Asn; Glu to Asp; Gly to Ala, or to Pro; His to Asn, or to Gln; Ile to Leu, or to Val; Leu to Ile, or to Val; Lys to Arg, to Gln, or to Glu; Met to Leu, to Tyr, or to Ile; Phe to Met, to Leu, or to Tyr; Ser to Thr; Thr to Ser; Trp to Tyr; Tyr to Trp; and/or Phe to Val, to Ile, or to Leu.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 3, and the VL comprises the amino acid sequence as set forth in SEQ ID NO: 13.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 26, and the VL comprises the amino acid sequence as set forth in SEQ ID NO: 27.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 28, and the VL comprises the amino acid sequence as set forth in SEQ ID NO: 29.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 30, and the VL comprises the amino acid sequence as set forth in SEQ ID NO: 29.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 22, and the VL comprises the amino acid sequence as set forth in SEQ ID NO: 23.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 33, and the VL comprises the amino acid sequence as set forth in SEQ ID NO: 42.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 34, and the VL comprises the amino acid sequence as set forth in SEQ ID NO: 42.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 33, and the VL comprises the amino acid sequence as set forth in SEQ ID NO: 41.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 34, and the VL comprises the amino acid sequence as set forth in SEQ ID NO: 39.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 34, and the VL comprises the amino acid sequence as set forth in SEQ ID NO: 40.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 34, and the VL comprises the amino acid sequence as set forth in SEQ ID NO: 41.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 1, and the VL comprises the amino acid sequence as set forth in SEQ ID NO: 2.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 3, and the VL comprises the amino acid sequence as set forth in SEQ ID NO: 4.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 5, and the VL comprises the amino acid sequence as set forth in SEQ ID NO: 6.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 7, and the VL comprises the amino acid sequence as set forth in SEQ ID NO: 8.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 9, and the VL comprises the amino acid sequence as set forth in SEQ ID NO: 10.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 11, and the VL comprises the amino acid sequence as set forth in SEQ ID NO: 12.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 14, and the VL comprises the amino acid sequence as set forth in SEQ ID NO: 15.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 16, and the VL comprises the amino acid sequence as set forth in SEQ ID NO: 17.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 18, and the VL comprises the amino acid sequence as set forth in SEQ ID NO: 19.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 20, and the VL comprises the amino acid sequence as set forth in SEQ ID NO: 21.

In preferred embodiments, the B7-H3 antibody or antigen-binding fragment thereof comprises a heavy chain variable region (VH) and a light chain variable region (VL), and the VH comprises the amino acid sequence as set forth in SEQ ID NO: 24, and the VL comprises the amino acid sequence as set forth in SEQ ID NO: 25.

In some embodiments of the antibody or antigen-binding fragment thereof disclosed herein, the antibody is a murine antibody, a chimeric antibody, a humanized antibody, or a human antibody.

In some embodiments, the antibody is a murine antibody, a chimeric antibody, a humanized antibody, or a human antibody.

Based on the amino acid sequence of the antibody heavy chain constant region, immunoglobulin molecules can be classified into five classes (isotypes): IgA, IgD, IgE, IgG, and IgM, and can be further divided into different subtypes, such as IgG1, IgG2, IgG3, IgG4, IgA1, IgA2, etc. Based on the amino acid sequence of the light chain, the light chain of an antibody can be classified into lambda (λ) chain and kappa (κ) chain. The antibodies disclosed herein may be of any of the classes or subtypes described above.

In some embodiments, the antibody is of an isotype selected from the group consisting of IgG, IgA, IgM, IgE, and IgD. In some embodiments, the antibody is of a subtype selected from the group consisting of IgG1, IgG2, IgG3, and IgG4. In preferred embodiments, the antibody is an IgG1 antibody.

The antibody disclosed herein may be an intact antibody or antigen-binding fragment thereof. The antigen-binding fragment may be any fragment of an antibody that retains the ability to specifically bind to B7-H3. Examples of antigen-binding fragments include, but are not limited to: Fab fragments; F(ab')2 fragments; Fab' fragments; Fd fragments; Fd' fragments; Fv fragments; scFv fragments; dAb fragments; isolated complementarity determining regions (CDRs); nanobodies; linear antibodies, consisting of a pair of tandem Fd fragments (VH-CH1-VH-CH1), and modified versions of any of the foregoing fragments that retain antigen binding activity.

In some embodiments, the antigen-binding fragment is selected from the group consisting of: Fab, Fab', F(ab')₂, Fv, scFv, and ds-scFv. In preferred embodiments, the antigen-binding fragment is a Fab. In another preferred embodiment, the antigen-binding fragment is an Fv. In another preferred embodiment, the antigen-binding fragment is an scFv.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 230, and the light chain comprises an amino acid sequence having at least 80%, at least 85%, at least 90%, at least 95%, at least 98%, at least 99% or 100% sequence identity to SEQ ID NO: 231. In some embodiments, the heavy chain comprises a functional variant of the amino acid sequence as set forth in SEQ ID NO: 230, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 231, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 248, and the light chain comprises an amino acid sequence having at least 80%, at least 85%, at least 90%, at least 95%, at least 98%, at least 99% or 100% sequence identity to SEQ ID NO: 249. In some embodiments, the heavy chain comprises a functional variant of the amino acid sequence as set forth in SEQ ID NO: 248, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 249, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 250, and the light chain comprises an amino acid sequence having at least 80%, at least 85%, at least 90%, at least 95%, at least 98%, at least 99% or 100% sequence identity to SEQ ID NO: 251. In some embodiments, the heavy chain comprises a functional variant of the amino acid sequence as set forth in SEQ ID NO: 250, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 251, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 252, and the light chain comprises an amino acid sequence having at least 80%, at least 85%, at least 90%, at least 95%, at least 98%, at least 99% or 100% sequence identity to SEQ ID NO: 251. In some embodiments, the heavy chain comprises a functional variant of the amino acid sequence as set forth in SEQ ID NO: 252, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 251, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 240, and the light chain comprises an amino acid sequence having at least 80%, at least 85%, at least 90%, at least 95%, at least 98%, at least 99% or 100% sequence identity to SEQ ID NO: 241. In some embodiments, the heavy chain comprises a functional variant of the amino acid sequence as set forth in SEQ ID NO: 240, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 241, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 255, and the light chain comprises an amino acid sequence having at least 80%, at least 85%, at least 90%, at least 95%, at least 98%, at least 99% or 100% sequence identity to SEQ ID NO: 264. In some embodiments, the heavy chain comprises a functional variant of the amino acid sequence as set forth in SEQ ID NO: 255, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 264, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 256, and the light chain comprises an amino acid sequence having at least 80%, at least 85%, at least 90%, at least 95%, at least 98%, at least 99% or 100% sequence identity to SEQ ID NO: 264. In some embodiments, the heavy chain comprises a functional variant of the amino acid sequence as set forth in SEQ ID NO: 256, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 264, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

**In** some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 255, and the light chain comprises an amino acid sequence having at least 80%, at least 85%, at least 90%, at least 95%, at least 98%, at least 99% or 100% sequence identity to SEQ ID NO: 263. In some embodiments, the heavy chain comprises a functional variant of the amino acid sequence as set forth in SEQ ID NO: 255, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 263, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

**In** some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 256, and the light chain comprises an amino acid sequence having at least 80%, at least 85%, at least 90%, at least 95%, at least 98%, at least 99% or 100% sequence identity to SEQ ID NO: 261. In some embodiments, the heavy chain comprises a functional variant of the amino acid sequence as set forth in SEQ ID NO: 256, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 261, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 256, and the light chain comprises an amino acid sequence having at least 80%, at least 85%, at least 90%, at least 95%, at least 98%, at least 99% or 100% sequence identity to SEQ ID NO: 262. In some embodiments, the heavy chain comprises a functional variant of the amino acid sequence as set forth in SEQ ID NO: 256, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 262, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 256, and the light chain comprises an amino acid sequence having at least 80%, at least 85%, at least 90%, at least 95%, at least 98%, at least 99% or 100% sequence identity to SEQ ID NO: 263. In some embodiments, the heavy chain comprises a functional variant of the amino acid sequence as set forth in SEQ ID NO: 256, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 263, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 218, and the light chain comprises an amino acid sequence having at least 80%, at least 85%, at least 90%, at least 95%, at least 98%, at least 99% or 100% sequence identity to SEQ ID NO: 219. In some embodiments, the heavy chain comprises a functional variant of the amino acid sequence as set forth in SEQ ID NO: 218, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 219, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 220, and the light chain comprises an amino acid sequence having at least 80%, at least 85%, at least 90%, at least 95%, at least 98%, at least 99% or 100% sequence identity to SEQ ID NO: 221. In some embodiments, the heavy chain comprises a functional variant of the amino acid sequence as set forth in SEQ ID NO: 220, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 221, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 222, and the light chain comprises an amino acid sequence having at least 80%, at least 85%, at least 90%, at least 95%, at least 98%, at least 99% or 100% sequence identity to SEQ ID NO: 223. In some embodiments, the heavy chain comprises a functional variant of the amino acid sequence as set forth in SEQ ID NO: 222, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 223, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 224, and the light chain comprises an amino acid sequence having at least 80%, at least 85%, at least 90%, at least 95%, at least 98%, at least 99% or 100% sequence identity to SEQ ID NO: 225. In some embodiments, the heavy chain comprises a functional variant of the amino acid sequence as set forth in SEQ ID NO: 224, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 225, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 226, and the light chain comprises an amino acid sequence having at least 80%, at least 85%, at least 90%, at least 95%, at least 98%, at least 99% or 100% sequence identity to SEQ ID NO: 227. In some embodiments, the heavy chain comprises a functional variant of the amino acid sequence as set forth in SEQ ID NO: 226, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 227, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 228, and the light chain comprises an amino acid sequence having at least 80%, at least 85%, at least 90%, at least 95%, at least 98%, at least 99% or 100% sequence identity to SEQ ID NO: 229. In some embodiments, the heavy chain comprises a functional variant of the amino acid sequence as set forth in SEQ ID NO: 228, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 229, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 232, and the light chain comprises an amino acid sequence having at least 80%, at least 85%, at least 90%, at least 95%, at least 98%, at least 99% or 100% sequence identity to SEQ ID NO: 233. In some embodiments, the heavy chain comprises a functional variant of the amino acid sequence as set forth in SEQ ID NO: 232, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 233, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 234, and the light chain comprises an amino acid sequence having at least 80%, at least 85%, at least 90%, at least 95%, at least 98%, at least 99% or 100% sequence identity to SEQ ID NO: 235. In some embodiments, the heavy chain comprises a functional variant of the amino acid sequence as set forth in SEQ ID NO: 234, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 235, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 236, and the light chain comprises an amino acid sequence having at least 80%, at least 85%, at least 90%, at least 95%, at least 98%, at least 99% or 100% sequence identity to SEQ ID NO: 237. In some embodiments, the heavy chain comprises a functional variant of the amino acid sequence as set forth in SEQ ID NO: 236, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 237, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 238, and the light chain comprises an amino acid sequence having at least 80%, at least 85%, at least 90%, at least 95%, at least 98%, at least 99% or 100% sequence identity to SEQ ID NO: 239. In some embodiments, the heavy chain comprises a functional variant of the amino acid sequence as set forth in SEQ ID NO: 238, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 239, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the antibody is a monoclonal antibody. In some embodiments, the antibody comprises a light chain and a heavy chain, wherein the heavy chain comprises an amino acid sequence having at least 80%, at least 85%, at least 90%, at least 95%, at least 98%, at least 99%, or 100% sequence identity to SEQ ID NO: 242, and the light chain comprises an amino acid sequence having at least 80%, at least 85%, at least 90%, at least 95%, at least 98%, at least 99% or 100% sequence identity to SEQ ID NO: 243. In some embodiments, the heavy chain comprises a functional variant of the amino acid sequence as set forth in SEQ ID NO: 242, formed by insertion, deletion, and/or substitution of one or more amino acids therein, provided that the functional variant retains the ability to bind to B7-H3. In some embodiments, the light chain comprises a functional variant of the amino acid sequence as set forth in SEQ ID NO: 243, formed by insertion, deletion, and/or substitution of one or more amino acids therein, provided that the functional variant retains the ability to bind to B7-H3. The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In some embodiments, the number of amino acids inserted, deleted and/or substituted preferably is not more than 40%, more preferably not more than 35%, more preferably 1% to 33%, and more preferably 5% to 30%, more preferably 10% to 25%, more preferably 15% to 20% of the total number of amino acids in the parent amino acid sequence. For example, the number of amino acids inserted, deleted, and/or substituted may be 1 to 50, preferably 1 to 20, more preferably 1 to 10, even more preferably 1 to 5. In preferred embodiments, the number of amino acids inserted, deleted, and/or substituted is 1, 2, 3, 4, 5, 6, or 7.

In some embodiments, the insertion, deletion, and/or substitution may be made in the framework (FR) region, such as FR1, FR2, FR3, and/or FR4; and/or a constant region such as CL, CH1, CH2, and/or CH3.

In some embodiments, the substitution of one or more amino acids may be a conservative substitution of one or more amino acids. Examples of conservative substitutions are as described above.

In preferred embodiments, the antibody comprises a heavy chain comprising the amino acid sequence as set forth in SEQ ID NO: 230, and a light chain comprising the amino acid sequence as set forth in SEQ ID NO: 231. In preferred embodiments, the antibody comprises a heavy chain comprising the amino acid sequence as set forth in SEQ ID NO: 248, and a light chain comprising the amino acid sequence as set forth in SEQ ID NO: 249. In preferred embodiments, the antibody comprises a heavy chain comprising the amino acid sequence as set forth in SEQ ID NO: 250, and a light chain comprising the amino acid sequence as set forth in SEQ ID NO: 251. In preferred embodiments, the antibody comprises a heavy chain comprising the amino acid sequence as set forth in SEQ ID NO: 252, and a light chain comprising the amino acid sequence as set forth in SEQ ID NO: 251. In preferred embodiments, the antibody comprises a heavy chain comprising the amino acid sequence as set forth in SEQ ID NO: 240, and a light chain comprising the amino acid sequence as set forth in SEQ ID NO: 241. In preferred embodiments, the antibody comprises a heavy chain comprising the amino acid sequence as set forth in SEQ ID NO: 255, and a light chain comprising the amino acid sequence as set forth in SEQ ID NO: 264. In preferred embodiments, the antibody comprises a heavy chain comprising the amino acid sequence as set forth in SEQ ID NO: 256, and a light chain comprising the amino acid sequence as set forth in SEQ ID NO: 264. In preferred embodiments, the antibody comprises a heavy chain comprising the amino acid sequence as set forth in SEQ ID NO: 255, and a light chain comprising the amino acid sequence as set forth in SEQ ID NO: 263. In preferred embodiments, the antibody comprises a heavy chain comprising the amino acid sequence as set forth in SEQ ID NO: 256, and a light chain comprising the amino acid sequence as set forth in SEQ ID NO: 261. In preferred embodiments, the antibody comprises a heavy chain comprising the amino acid sequence as set forth in SEQ ID NO: 256, and a light chain comprising the amino acid sequence as set forth in SEQ ID NO: 262. In preferred embodiments, the antibody comprises a heavy chain comprising the amino acid sequence as set forth in SEQ ID NO: 256, and a light chain comprising the amino acid sequence as set forth in SEQ ID NO: 263. In preferred embodiments, the antibody comprises a heavy chain comprising the amino acid sequence as set forth in SEQ ID NO: 218, and a light chain comprising the amino acid sequence as set forth in SEQ ID NO: 219. In preferred embodiments, the antibody comprises a heavy chain comprising the amino acid sequence as set forth in SEQ ID NO: 220, and a light chain comprising the amino acid sequence as set forth in SEQ ID NO: 221. In preferred embodiments, the antibody comprises a heavy chain comprising the amino acid sequence as set forth in SEQ ID NO: 222, and a light chain comprising the amino acid sequence as set forth in SEQ ID NO: 223. In preferred embodiments, the antibody comprises a heavy chain comprising the amino acid sequence as set forth in SEQ ID NO: 224, and a light chain comprising the amino acid sequence as set forth in SEQ ID NO: 225. In preferred embodiments, the antibody comprises a heavy chain comprising the amino acid sequence as set forth in SEQ ID NO: 226, and a light chain comprising the amino acid sequence as set forth in SEQ ID NO: 227. In preferred embodiments, the antibody comprises a heavy chain comprising the amino acid sequence as set forth in SEQ ID NO: 228, and a light chain comprising the amino acid sequence as set forth in SEQ ID NO: 229. In preferred embodiments, the antibody comprises a heavy chain comprising the amino acid sequence as set forth in SEQ ID NO: 232, and a light chain comprising the amino acid sequence as set forth in SEQ ID NO: 233. In preferred embodiments, the antibody comprises a heavy chain comprising the amino acid sequence as set forth in SEQ ID NO: 234, and a light chain comprising the amino acid sequence as set forth in SEQ ID NO: 235. In preferred embodiments, the antibody comprises a heavy chain comprising the amino acid sequence as set forth in SEQ ID NO: 236, and a light chain comprising the amino acid sequence as set forth in SEQ ID NO: 237. In preferred embodiments, the antibody comprises a heavy chain comprising the amino acid sequence as set forth in SEQ ID NO: 238, and a light chain comprising the amino acid sequence as set forth in SEQ ID NO: 239. In preferred embodiments, the antibody comprises a heavy chain comprising the amino acid sequence as set forth in SEQ ID NO: 242, and a light chain comprising the amino acid sequence as set forth in SEQ ID NO: 243.

In other embodiments, the antibody is a bispecific antibody or a multispecific antibody. In some embodiments, the antibody is a bispecific antibody further comprising a second antigen-binding region that binds to a second antigen. In some embodiments, the second antigen is a tumor-associated antigen or an immune cell antigen.

A number of tumor-associated antigens associated with specific cancers have been identified in the art. In some embodiments, the tumor-associated antigen is an antigen that can elicit a pronounced tumor-specific immune response. Some of these antigens are encoded by, but not necessarily expressed by, normal cells. These antigens can be characterized as antigens that are normally silent (i.e., not expressed) in normal cells, antigens that are expressed only at certain stages of differentiation, and antigens that are temporally expressed, such as embryonic and fetal antigens. Other cancer cell antigens are encoded by mutant cell genes such as oncogenes (e.g., activated ras oncogene), suppressor genes (e.g., P53 mutant), and fusion proteins resulting from internal deletions or chromosomal translocations. Other cancer antigens can be encoded by viral genes, such as those carried by RNA and DNA tumor viruses. Many other tumor-associated antigens and antibodies against them are known and/or commercially available, and can also be prepared by those skilled in the art.

The antibody specifically binding to B7-H3 of the present disclosure comprises an Fc region. The Fc region may be of any isotype, including but not limited to IgG1, IgG2, IgG3, and IgG4, and may comprise one or more mutations or modifications. In an embodiment, the Fc region is of or is derived from the IgG1 isotype, optionally with one or more mutations or modifications. In another embodiment, the Fc region is of or is derived from the IgG4 isotype, optionally with one or more mutations or modifications. In an embodiment, the Fc region is a human IgG1 Fc.

In an embodiment, the Fc region has reduced effector function, such as reduced ADCC, ADCP, CDC, and/or C1q, FcγRI, FcγRII, or FcγRIIIA binding. For example, the Fc region may be of the IgG1 isotype, or a non-IgG1 type, such as IgG2, IgG3, or IgG4, which has been mutated such that the ability to mediate effector function is reduced or even eliminated. Such mutations have been described, for example, in Dall'Acqua WF et al., J Immunol. 177(2):1129-1138 (2006), and Hezareh M, J Virol.; 75(24):12161-12168 (2001). For example, the Fc region may comprise an amino acid sequence having one or more of the following amino acid substitutions as compared to the wild-type sequence: E233P, L234A, L234F, L235A, L235E, G237A, N297A, N297D, P331S, and P329G.

In an embodiment, the Fc region comprises a mutation that removes the acceptor site for Asn-linked glycosylation or is otherwise manipulated to alter glycosylation properties. For example, in an IgG1 Fc region, an N297Q mutation may be used to remove the Asn-linked glycosylation site. Thus, in a particular embodiment, the Fc region comprises an IgG1 sequence having the N297Q mutation.

In further embodiments, the Fc region is glycoengineered to reduce fucose and thereby enhance ADCC, for example by adding compounds to the culture medium during antibody production, as described in US2009317869 or as described in van Berkel et al. (2010) Biotechnol. Bioeng. 105: 350, or by using FUT8 knockout cells, for example, as described in Yamane-Ohnuki et al. (2004) Biotechnol. Bioeng 87: 614. Alternatively, the method described by Umaña et al. (1999), Nature Biotech 17: 176, can be used to optimize ADCC. In another embodiment, the Fc region is engineered to enhance complement activation, as described, for example, in Natsume et al. (2009) Cancer Sci. 100: 2411.

In some embodiments, the Fc region comprises a modification or mutation that can inhibit Fc homodimerization. In some embodiments, the Fc region comprises a variant of the human IgG1 Fc wild-type sequence. The variant may comprise amino acid substitutions at the positions T366 and Y407 (Kabat numbering) in human IgG1. Preferably, T366 is substituted with L (leucine). Preferably, Y407 is substituted with I (isoleucine), F (phenylalanine), L (leucine), M (methionine), H (histidine), K (lysine), S (serine), Q (glutamine), T (threonine), W (tryptophan), A (alanine), G (glycine), or N (asparagine). More preferably, Y407 is substituted with H. In an embodiment, T366 is substituted with L, and Y407 is substituted with H.

In some embodiments, the Fc region may be a monomeric human IgG1 Fc (e.g., mFc7.2), as described in PCT Application No. PCT/US2018/016524, which is incorporated herein by reference in its entirety.

### Nucleic acid

The present disclosure provides a nucleic acid comprising a nucleotide sequence encoding the antibody or antigen-binding fragment thereof disclosed herein.

The term "nucleic acid" includes single-stranded and double-stranded nucleotide polymers. The nucleic acid may be a ribonucleotide or a deoxyribonucleotide or a modified form of either type of nucleotide. The modifications include base modifications such as bromouridine and inosine derivatives, ribose modifications such as 2',3'-dideoxyribose, and internucleotide linkage modifications such as phosphorothioate, phosphorodithioate, phosphoroselenoate, phosphorodiselenoate, phenyl thiophosphate, phosphoranilidate, and phosphoramidate.

For example, the present disclosure provides a nucleic acid molecule encoding any one of the heavy chain variable region sequences disclosed herein. The present disclosure also provides a nucleic acid molecule that is at least 90%, at least 95%, at least 98%, or at least 99% identical to a nucleic acid encoding any one of the heavy chain variable region sequences disclosed herein.

For example, the present disclosure provides a nucleic acid molecule encoding any one of the light chain variable region sequences disclosed herein. The present disclosure also provides a nucleic acid molecule that is at least 90%, at least 95%, at least 98%, or at least 99% identical to a nucleic acid encoding any one of the light chain variable region sequences disclosed herein.

For example, the present disclosure provides a nucleic acid molecule encoding a heavy chain variable region sequence comprising a CDR sequence of any one of the heavy chain variable region sequences disclosed herein. The present disclosure also provides a nucleic acid molecule encoding a heavy chain variable region sequence comprising a CDR sequence that is at least 90%, at least 95%, at least 98%, or at least 99% identical to a CDR sequence of any one of the heavy chain variable region sequences disclosed herein.

For example, the present disclosure provides a nucleic acid molecule encoding a light chain variable region sequence comprising a CDR sequence of any one of the light chain variable region sequences disclosed herein. The present disclosure also provides a nucleic acid molecule encoding a light chain variable region sequence comprising a CDR sequence that is at least 90%, at least 95%, at least 98%, or at least 99% identical to a CDR sequence of any one of the light chain variable region sequences disclosed herein.

In some embodiments, the nucleic acid is a ribonucleic acid (RNA) or a deoxyribonucleic acid (DNA). In some embodiments, the present disclosure provides a ribonucleic acid (RNA) comprising a nucleotide sequence encoding an antibody disclosed herein. In some embodiments, the present disclosure provides a deoxyribonucleic acid (DNA) comprising a deoxynucleotide sequence encoding an antibody disclosed herein.

In some embodiments, the deoxyribonucleic acid (DNA) can be introduced into human cells *in vivo.* In some embodiments, the deoxyribonucleic acid (DNA) of the present disclosure is comprised in a carrier or a delivery agent. In some embodiments, the deoxyribonucleic acid (DNA) of the present disclosure is integrated into the genome of a cell.

In some embodiments, the ribonucleic acid (RNA) can be introduced *in vivo* into human cells. In some embodiments, the ribonucleic acid (RNA) of the present disclosure is comprised in a carrier or a delivery agent.

### Vectors

The present disclosure provides a vector comprising the nucleic acids disclosed herein.

In some embodiments, the vector is an expression vector capable of expressing a polypeptide comprising a heavy chain variable region or a light chain variable region of an antibody. For example, the present disclosure provides an expression vector comprising any one of the nucleic acid molecules described above.

In some embodiments, the vector is an expression vector capable of expressing a polypeptide comprising a heavy chain or a light chain of an antibody. For example, the present disclosure provides an expression vector comprising any one of the nucleic acid molecules described above.

Any vector may be suitable for use in the present disclosure. In some embodiments, the vector is a viral vector. In some embodiments, the vector is a retroviral vector, a DNA vector, a murine leukemia virus vector, an SFG vector, a plasmid, an RNA vector, an adenoviral vector, a baculovirus vector, an Epstein Barr virus vector, a papovavirus vector, a vaccinia virus vector, a herpes simplex virus vector, an adenovirus-associated vector (AAV), a lentiviral vector, or any combination thereof. Suitable exemplary vectors include, for example, pGAR, pBABE-puro, pBABE-neo largeTcDNA, pBABE-hygro-hTERT, pMKO.1 GFP, MSCV-IRES-GFP, pMSCV PIG(Puro IRES GFP empty plasmid), pMSCV-loxp-dsRed-loxp-eGFP-Puro-WPRE, MSCV IRES Luciferase, pMIG, MDH1-PGK-GFP_2.0, TtRMPVIR, pMSCV-IRES-mCherry FP, pRetroX GFP T2A Cre, pRXTN, pLncEXP, and pLXIN-Luc.

The expression vector may be any suitable recombinant expression vector. Suitable vectors include those designed for propagation and amplification or for expression or both, such as plasmids and viruses. For example, the vector may be selected from pUC series (Fermentas Life Sciences, Glen Burnie, Md.), pBluescript series (Stratagene, LaJolla, Calif.), pET series (Novagen, Madison, Wis.), pGEX series (Pharmacia Biotech, Uppsala, Sweden), and pEX series (Clontech, Palo Alto, Calif.). Phage vectors such as λGT10, λGT11, λZapII (Stratagene), λEMBL4, and λNM1149 can also be used. Examples of plant expression vectors useful in the present disclosure include pBI01, pBI101.2, pBI101.3, pBI121, and pBIN19 (Clontech). Examples of animal expression vectors useful in the present disclosure include pcDNA, pEUK-Cl, pMAM, and pMAMneo (Clontech).

Recombinant expression vectors can be prepared using standard recombinant DNA techniques as described in, for example, Sambrook et al., Molecµlar Cloning: A Laboratory Manual, 3rd ed., Cold Spring Harbor Press, Cold Spring Harbor, N.Y. 2001; and Ausubel et al., Current Protocols in Molecµlar Biology, Greene Publishing Associates and John Wiley & Sons, NY, 1994. Circular or linear expression vector constructs can be prepared to contain replication system functions in prokaryotic or eukaryotic host cells. The replication system can be derived from, for example, COLEL, a 2µ plasmid, a λ, SV40, or bovine papillomavirus.

For example, the vector may be an adenoviral vector comprising a nucleotide sequence encoding an antibody disclosed herein. The vector may be administered to a subject and then introduced into a cell of the subject *in vivo,* thereby integrating the nucleotide sequence encoding the antibody disclosed herein into the genome of the cell. Subsequently, the cell expresses the antibody disclosed herein.

### Host Cells

The present disclosure provides a host cell comprising a nucleic acid disclosed herein or a vector disclosed herein.

Any cell can be used as a host cell for the nucleic acid or vector of the present disclosure. In some embodiments, the cell may be a prokaryotic cell, a fungal cell, a yeast cell, or a higher eukaryotic cell such as a mammalian cell. A suitable prokaryotic cell includes, but is not limited to, eubacteria, such as a Gram-negative or Gram-positive organism, such as *Enterobactehaceae,* such as *Escherichia,* such as *E. coli; Enterobacter; Erwinia; Klebsiella; Proteus; Salmonella,* such as *Salmonella typhimurium; Serratia,* such as *Serratia marcescans* and *Shigella; Bacilli,* such as *B. subtilis* and *B. licheniformis; Pseudomonas,* such as *P. aeruginosa;* and *Streptomyces.* In some embodiments, the cell is a human cell. In some embodiments, the cell is an immune cell. In some embodiments, the host cells include, for example, CHO cells, such as CHOS cells and CHO-K1 cells, or HEK293 cells, such as HEK293A, HEK293T, and HEK293FS.

The host cells of the present disclosure are prepared by introducing a vector disclosed herein or a nucleic acid disclosed herein in vitro or ex vivo. The host cell of the present disclosure may be administered to a subject, and the host cell expresses the antibody disclosed herein *in vivo.*

The present disclosure provides a host cell into which any of the aforementioned vectors have been introduced. The present disclosure also provides a method of preparing an antibody of the present disclosure, comprising a) culturing the host cell of the fourth aspect of the present disclosure under conditions suitable for the production of the antibody; and b) obtaining the antibody from the culture.

### Pharmaceutical Compositions

The present disclosure provides a pharmaceutical composition comprising the antibody or antigen-binding fragment thereof disclosed herein, or the bispecific antibody or antigen-binding fragment thereof disclosed herein, and a pharmaceutically acceptable carrier or excipient.

The antibody or antigen-binding fragment or agent thereof of the present disclosure (also referred to herein as "active compound") and derivatives, fragments, analogs, and homologs thereof may be incorporated into pharmaceutical compositions suitable for administration. Such compositions typically comprise an antibody or antigen-binding fragment or agent thereof and a pharmaceutically acceptable carrier. As used herein, the term "pharmaceutically acceptable carrier" is intended to include any and all of solvents, dispersion media, coatings, antibacterial agents, antifungal agents, isotonic agents, and absorption delaying agents, and the like that are compatible with the administration of the medicament. Preferred examples of such carriers or excipients include, but are not limited to, water, saline, Ringer's solution, dextrose solution, and 5% human serum albumin. Liposomes and non-aqueous vehicles such as fixed oils can also be used. The use of such media and agents for use as a pharmaceutically active substance is well known in the art. Unless any conventional medium or agent is incompatible with the active compound, uses thereof in the composition is contemplated. Supplementary active compounds may also be incorporated into the compositions.

In some embodiments, the pharmaceutical composition further comprises a second therapeutic agent. In some embodiments, the second therapeutic agent is selected from the group consisting of a cytokine, an antibody, a chemotherapeutic agent, and a small molecule drug. In some embodiments, the second therapeutic agent is selected from an interleukin (e.g., IL-2), a Bruton's tyrosine kinase (BTK) inhibitor, a PI3K inhibitor, an HDAC inhibitor, an ERK inhibitor, a MAPK inhibitor, a PD-1 inhibitor, a PD-L1 inhibitor, a CTLA-4 inhibitor, a TIGIT inhibitor, a TIM3 inhibitor, a VEGF inhibitor, a LAG3 inhibitor, and a glucocorticoid.

In some embodiments, the second therapeutic agent is a cytokine. Examples of cytokines include, but are not limited to, interleukins (e.g., IL-2, IL-7, IL-10, IL-12, IL-15) and interferons (e.g., IFNα, IFNγ). In some embodiments, the second therapeutic agent is an interleukin. In preferred embodiments, the second therapeutic agent is IL-2.

In some embodiments, the second therapeutic agent is a chemotherapeutic agent. Chemotherapeutic agents may include, for example, cytotoxic agents, antimetabolites (e.g. folate antagonists, purine analogs, pyrimidine analogs, etc.), topoisomerase inhibitors (e.g. camptothecin derivatives, anthraquinones, anthracyclines, epipodophyllotoxins, quinoline alkaloids, etc.), anti-microtubule agents (e.g. taxanes, vinca alkaloids), protein synthesis inhibitors (e.g. cephalotaxlen, camptothecin derivatives, quinoline alkaloids), alkylating agents (e.g., alkyl sulfonates, aziridines, nitrogen mustards, nitrosoureas, platinum derivatives, triazenes, etc.), alkaloids, terpenoids, and kinase inhibitors.

The pharmaceutical compositions of the present disclosure may be formulated to be compatible with their intended route of administration. Examples of routes of administration include parenteral administration, such as intravenous, intradermal, subcutaneous, oral (e.g., inhalation), transdermal (i.e., topical), transmucosal, and rectal administration. Solutions or suspensions for parenteral, intradermal or subcutaneous application may include the following components: sterile diluents such as water for injection, saline solutions, fixed oils, polyethylene glycols, glycerol, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates; and agents for tonicity adjustment, such as sodium chloride or dextrose. The pH can be adjusted with an acid or base, such as hydrochloric acid or sodium hydroxide. The parenteral formulation may be encapsulated in ampoules, disposable syringes, or multiple-dose vials made of glass or plastic.

Pharmaceutical compositions suitable for injectable use include sterile aqueous solutions (if water soluble) or dispersions and sterile powders for the extemporaneous preparation of sterile injectable solutions or dispersions. For intravenous administration, suitable carriers include physiological saline, bacteriostatic water, Cremophor ELTM (BASF, Parsippany, N.J.), or phosphate-buffered saline (PBS). In all cases, the composition must be sterile and should be a fluid present to an extent that is easily injectable. It must be stable under the conditions of preparation and storage and must be preserved against contamination by microorganisms such as bacteria and fungi. The carrier may be a solvent or dispersion medium containing, for example, water, ethanol, polyols (e.g., glycerol, propylene glycol, and liquid polyethylene glycol, and the like), and suitable mixtures thereof. The suitable fluidity may be maintained, for example, by the use of a coating such as lecithin, by the maintenance of the required particle size in the case of dispersion, and by the use of surfactants. The action of microorganisms may be prevented by various antibacterial and antifungal agents, such as parabens, chlorobutanol, phenol, ascorbic acid, and thimerosal. In many cases, it is preferred that an isotonic agent, such as a sugar, a polyol such as mannitol, sorbitol, or sodium chloride, is included in the composition. The absorption of the injectable composition may be prolonged by including in the composition an agent that delays absorption, such as aluminum monostearate and gelatin.

Sterile injectable solutions may be prepared by incorporating a desired amount of the active compound into a suitable solvent with one or a combination of the ingredients enumerated above (as required), followed by filtration and sterilization. Typically, dispersions are prepared by introducing the active compound into a sterile vehicle containing a basic dispersion medium and the required other ingredients from those enumerated above. In the case of sterile powders for the preparation of sterile injectable solutions, the method of preparation is vacuum drying and freeze-drying to yield a powder of the active ingredient plus any additional desired ingredient from the previously sterile filtered solution.

Oral compositions typically include an inert diluent or an edible carrier. They can be encapsulated in gelatin capsules or compressed into tablets. For the purpose of oral therapeutic administration, the active compound may be mixed with excipients and used in the form of tablets, lozenges, or capsules. The oral composition may also be prepared using a fluid carrier for use as a mouthwash, wherein the compound in the fluid carrier is orally administered and gargled, and spit out or swallowed. A pharmaceutically compatible binding agent and/or an adjuvant material may be included as a part of the composition. The tablets, pills, capsules, lozenges and the like may contain any of the following ingredients or compounds of similar nature: a binder such as microcrystalline cellulose, tragacanth gum or gelatin; an excipient such as starch or lactose, a disintegrant such as alginic acid, Primogel or corn starch; a lubricant such as magnesium stearate or Sterotes; a glidant such as colloidal silica; a sweetener such as sucrose or saccharin; or a flavoring agent such as peppermint oil, methyl salicylate or orange flavoring.

For administration by inhalation, the compound is delivered in the form of an aerosol spray from a pressured container, or a dispenser or a nebulizer containing a suitable propellant, for example, a gas such as carbon dioxide.

Systemic administration can also be performed by transmucosal or transdermal means. For transmucosal or transdermal administration, penetrants appropriate to the barrier to be penetrated are used in the formulation. Such penetrants are generally known in the art and include, for example, for transmucosal administration, detergents, bile salts, and fusidic acid derivatives. Transmucosal administration may be performed by use of nasal sprays or suppositories. For transdermal administration, the active compound is formulated as an ointment, a salve, a gel, or a cream, well known in the art.

The active compound may also be formulated in the form of suppositories (e.g., using conventional suppository bases such as cocoa butter and other glycerides) or retention enemas for rectal delivery.

In an embodiment, the active compound is prepared with a carrier that will protect the compound from rapid elimination in the body, such as a controlled release formulation, including implants and microencapsulated delivery systems. Biodegradable, biocompatible polymers such as ethylene vinyl acetate, polyanhydrides, polyglycolic acid, collagen, polyorthoesters, and polylactic acid may be used. Methods for preparing such formulations will be apparent to those skilled in the art.

The present disclosure provides a therapeutic composition comprising the antibody or antigen-binding fragment thereof of the present disclosure. The therapeutic composition according to the present disclosure will be administered with a suitable carrier, excipient, and other agents incorporated into the formulation to provide improved transfer, delivery, tolerance, and the like. Many suitable formulations can be found in all formulas known to medicinal chemists: Remington's Pharmaceutical Sciences, Mack Publishing Company, Easton, PA. Such formulations include, for example, powders, pastes, ointments, jellies, waxes, oils, lipids, vesicle-containing lipids (cationic or anionic) such as LIPOFECTIN^{™}), DNA conjugates, anhydrous absorption pastes, oil-in-water and water-in-oil emulsions, polyethylene glycol emulsions (polyethylene glycols of various molecular weights), semi-solid gels, and semi-solid mixtures containing polyethylene glycol. See also Powell et al., "Compendium of excipients for parenteral formulations" PDA (1998) J Pharm Sci Technol 52:238-311.

### Conjugates

The present disclosure provides a conjugate comprising the antibody or antigen-binding fragment thereof disclosed herein or the bispecific antibody or antigen-binding fragment thereof disclosed herein, and a chemical moiety conjugated thereto.

In the context of the present disclosure, a "conjugate" is an antibody or antibody fragment (such as an antigen-binding fragment) covalently linked to a chemical moiety. The chemical moiety may be, for example, a drug, a toxin, a therapeutic agent, a detectable label, a protein, a nucleic acid, a lipid, a nanoparticle, a carbohydrate, or a recombinant virus. Antibody conjugates are commonly referred to as "immunoconjugates". When the conjugate comprises an antibody linked to a drug (e.g., a cytotoxic agent), the conjugate is generally referred to as an "antibody-drug conjugate" or an "ADC".

The term "conjugated" or "linked" may refer to making two polypeptides into one contiguous polypeptide molecule. In an embodiment, the antibody is linked to a chemical moiety. In another embodiment, the antibody linked to the chemical moiety is further linked to a lipid or other molecule, to a protein or peptide, in order to increase its half-life *in vivo.* The linking may be carried out chemically or recombinantly. In an embodiment, the linkage is chemical, wherein the reaction between the antibody moiety and the chemical moiety has produced a covalent bond formed between the two molecules to form one molecule. A peptide linker (short peptide sequence) may optionally be included between the antibody and the chemical moiety.

The chemical moiety may be linked to the antibody of the present disclosure using any number of means known to those skilled in the art. Both covalent and non-covalent attachment means may be used. The procedure for attaching the chemical moiety to the antibody varies depending on the chemical structure of the chemical moiety. Polypeptides typically contain a variety of functional groups, such as a carboxylic acid (COOH), a free amine (-NH2), or a sulfhydryl (-SH) moiety, which is available for reaction with a suitable functional group on the antibody to result in binding of the chemical moiety. Alternatively, the antibody is derivatized to expose or attach an additional reactive functional group. The derivatization may involve attachment of any of a number of known linker molecules. The linker may be any molecule used to attach the antibody to the chemical moiety. The linker is capable of forming a covalent bond with both the antibody and the chemical moiety. Suitable linkers are well known to those skilled in the art and include, but are not limited to, linear or branched carbon linkers, heterocyclic carbon linkers, or peptide linkers. In case that the antibody and the chemical moiety are polypeptides, the linker may be linked to the constituent amino acids through their side groups (e.g., through a disulfide bond to a cysteine) or to the alpha carbon amino group and carboxyl group of the terminal amino acids.

In certain instances, it is desirable to release the chemical moiety from the antibody when the immunoconjugate reaches its target site. Thus, in these instances, the immunoconjugate will comprise a linkage that is cleavable in the vicinity of the target site.

The enzymatic activity or conditions to which the immunoconjugate is subjected within the target cell or in the vicinity thereof may cause cleavage of the linker to release the chemical moiety from the antibody.

In view of the numerous methods that have been reported for attaching various radiodiagnostic compounds, radiotherapeutic compounds, labels (such as enzymes or fluorescent molecules), drugs, toxins, and other agents to antibodies, those skilled in the art will be able to determine suitable methods for attaching a given agent to an antibody or other polypeptide.

The antibodies disclosed herein can be derivatized or linked to another molecule (e.g., another peptide or protein). Typically, the antibody or portion thereof is derivatized such that the binding to the target antigen is not adversely affected by derivatization or labeling. For example, an antibody can be functionally linked (by chemical coupling, genetic fusion, non-covalent association, or otherwise) to one or more other molecular entities, such as another antibody (e.g., a bispecific antibody or a bivalent antibody), a detection agent, a pharmaceutical agent, and/or a protein or peptide that can mediate association of the antibody or antibody portion with another molecule (e.g., a streptavidin core region or a polyhistidine tag).

One type of derivatized antibody is produced by cross-linking two or more antibodies (of the same type or of different types). Suitable crosslinkers include heterobifunctional or homobifunctional crosslinkers (e.g., disuccinimidyl suberate) having two distinctly reactive moieties (e.g., m-maleimidobenzoyl-N-hydroxysuccinimide ester) separated by a suitable spacer. Such linkers are commercially available.

In some embodiments of the conjugate disclosed herein, the chemical moiety is selected from the group consisting of a therapeutic agent, a detectable moiety, and an immunostimulatory molecule.

### Treatment Methods

The present disclosure provides a method of treating cancer in a subject, comprising administering to the subject an effective amount of the antibody or antigen-binding fragment thereof, the nucleic acid, the vector, the host cell, the pharmaceutical composition, or the conjugate disclosed herein.

In some embodiments of the method disclosed herein, the cancer is a B7-H3 positive cancer. In some embodiments, the cancer is selected from the group consisting of: adrenal tumor, AIDS-related cancer, alveolar soft part sarcoma, astrocytoma, adrenal cancer, bladder cancer, bone cancer, brain and spinal cord cancer, metastatic brain tumor, B-cell cancer, breast cancer, carotid body tumor, cervical cancer, chondrosarcoma, chordoma, chromophobe renal cell carcinoma, clear cell carcinoma, colon cancer, colorectal cancer, cutaneous benign fibrous histiocytoma, desmoplastic small round cell tumor, ependymoma, Ewing's tumor, extraskeletal myxoid chondrosarcoma, incomplete bone fibrogenesis, fibrous dysplasia of bone, gallbladder or bile duct cancer, gastric cancer, gestational trophoblast disease, germ cell tumor, head and neck cancer, hepatocellular carcinoma, islet cell tumor, Kaposi's sarcoma, kidney cancer, leukemia, liposarcoma/malignant lipomatous tumor, liver cancer, lymphoma, lung cancer, medulloblastoma, melanoma, meningioma, multiple endocrine neoplasia, multiple myeloma, myelodysplastic syndrome, neuroblastoma, neuroendocrine tumor, ovarian cancer, pancreatic cancer, papillary thyroid carcinoma, parathyroid tumor, pediatric cancer, peripheral nerve sheath tumor, pheochromocytoma, pituitary tumor, prostate cancer, posterious uveal melanoma, renal metastatic cancer, rhabdoid tumor, rhabdomyosarcoma, sarcoma, skin cancer, soft tissue sarcoma, squamous cell carcinoma, gastric carcinoma, synovial sarcoma, testicular carcinoma, thymic carcinoma, thymoma, metastatic thyroid cancer, and uterine cancer.

In some embodiments, the dosage administered to the subject may vary depending on the embodiment, the drug used, the method of administration, and the site and subject to be treated. However, the dosage should be sufficient to provide a therapeutic response. A clinician can determine an effective amount to be administered to a human or other subject to treat a medical disorder. The precise amount required to be therapeutically effective may depend on a number of factors, such as the activity of the antibody and the route of administration.

A dose of the antibody, composition, or conjugate described herein may be administered to a mammal in one dose, or in a series of sub-doses over a suitable period of time, for example, once daily, semi-weekly, weekly, bi-weekly, semi-monthly, bimonthly, semi-annually, or annually, as needed. A dosage unit comprising an effective amount of the antibody, composition, or conjugate may be administered in a single daily dose, or the total daily dose may be administered in two, three, four, or more divided doses administered daily as desired.

A suitable mode of administration may be selected by the physician. The route of administration may be parenteral administration, such as administration by injection, nasal administration, pulmonary administration, or transdermal administration. Systemic or local administration may be performed by intravenous injection, intramuscular injection, intraperitoneal injection, or subcutaneous injection. In some embodiments, the antibody, composition, or conjugate is selected for parenteral delivery, for inhalation, or for delivery through the digestive tract, for example, orally. The dosage and method of administration may vary depending on the weight, age, condition, etc. of the subject, and may be appropriately selected.

In some embodiments, the method further comprises administering a second therapeutic agent to the subject. In certain embodiments, the antibody, composition, or conjugate disclosed herein is administered prior to, substantially simultaneously with, or after administration of the second therapeutic agent.

### Medical Uses

The present disclosure provides the use of the antibody or antigen-binding fragment thereof disclosed herein, the bispecific antibody or antigen-binding fragment thereof disclosed herein, the nucleic acid, the host cell, the pharmaceutical composition, or the conjugate in the manufacture of a medicament for treating cancer in a subject.

The present disclosure also provides the antibody or antigen-binding fragment thereof disclosed herein, the bispecific antibody or antigen-binding fragment thereof disclosed herein, the pharmaceutical composition disclosed herein, or the conjugate disclosed herein, for use in treating cancer in a subject.

In some embodiments of the use disclosed herein, the cancer is a B7-H3 positive cancer. In some embodiments, the cancer is selected from the group consisting of: adrenal tumor, AIDS-related cancer, alveolar soft part sarcoma, astrocytoma, adrenal cancer, bladder cancer, bone cancer, brain and spinal cord cancer, metastatic brain tumor, B-cell cancer, breast cancer, carotid body tumor, cervical cancer, chondrosarcoma, chordoma, chromophobe renal cell carcinoma, clear cell carcinoma, colon cancer, colorectal cancer, cutaneous benign fibrous histiocytoma, desmoplastic small round cell tumor, ependymoma, Ewing's tumor, extraskeletal myxoid chondrosarcoma, incomplete bone fibrogenesis, fibrous dysplasia of bone, gallbladder or bile duct cancer, gastric cancer, gestational trophoblast disease, germ cell tumor, head and neck cancer, hepatocellular carcinoma, islet cell tumor, Kaposi's sarcoma, kidney cancer, leukemia, liposarcoma/malignant lipomatous tumor, liver cancer, lymphoma, lung cancer, medulloblastoma, melanoma, meningioma, multiple endocrine neoplasia, multiple myeloma, myelodysplastic syndrome, neuroblastoma, neuroendocrine tumor, ovarian cancer, pancreatic cancer, papillary thyroid carcinoma, parathyroid tumor, pediatric cancer, peripheral schwannoma, pheochromocytoma, pituitary tumor, prostate cancer, posterious uveal melanoma, renal metastatic cancer, rhabdoid tumor, rhabdomyosarcoma, sarcoma, skin cancer, soft tissue sarcoma, squamous cell carcinoma, gastric carcinoma, synovial sarcoma, testicular carcinoma, thymic carcinoma, thymoma, metastatic thyroid cancer, and uterine cancer.

### Diagnostic and Detection Methods

The present disclosure provides a method for detecting B7-H3 protein in vitro or in vivo. In some cases, B7-H3 expression is detected in a biological sample. The sample can be any sample, including, but not limited to, blood samples, tissue from biopsy, autopsy, and pathological specimens. The biological sample also includes body fluids such as blood, serum, plasma, sputum, spinal fluid, or urine. The biological sample is typically obtained from mammals, such as humans or non-human primates.

The present disclosure also provides a method of determining whether a subject has a B7-H3 positive cancer, by contacting a sample from a subject with the anti-B7-H3 antibody disclosed herein, and detecting the binding of the antibody to the sample. Increased binding of the antibody to the sample, as compared to that of the antibody to the control sample, identifies that the subject has cancer.

In another embodiment, the present disclosure provides a method of diagnosing the B7-H3 positive cancer in a subject, by contacting a sample from a subject with the anti-B7-H3 antibody disclosed herein, and detecting the binding of the antibody to the sample. Increased binding of the antibody to the sample compared to that of the antibody to the control sample confirms the diagnosis of cancer in the subject.

In some embodiments, the control sample is a sample from a subject without cancer. In a particular embodiment, the sample is a blood or tissue sample.

In some embodiments of the diagnostic and detection methods, the anti-B7-H3 antibody is directly labeled with a detectable label. In another embodiment, the antiB7-H3 antibody (primary antibody) is unlabeled while the secondary antibody or other molecule that can bind to the primary antibody is labeled. As is well known to those skilled in the art, the secondary antibody is selected that is capable of specifically binding to the primary antibody of a particular species and type. For example, if the primary antibody is human IgG, the second antibody may be anti-human IgG. Other molecules that can bind to an antibody include, but are not limited to, Protein A and Protein G, both of which are commercially available.

Suitable labels for antibodies or secondary antibodies include various enzymes, prosthetic groups, fluorescent materials, luminescent materials, magnetic agents, and radioactive materials. Non-limiting examples of suitable enzymes include horseradish peroxidase, alkaline phosphatase, beta-galactosidase, or acetylcholinesterase. Non-limiting examples of suitable prosthetic complexes include streptavidin/biotin and avidin/biotin. Non-limiting examples of suitable fluorescent materials include umbelliferone, fluorescein, fluorescein isothiocyanate, rhodamine, dichlorotriazinylamine fluorescein, dansyl chloride, or phycoerythrin. A non-limiting exemplary luminescent material is luminol; a non-limiting exemplary magnetic agent is gadolinium, and non-limiting exemplary radiolabels include 125I, 131I, 35S, or 3H.

In an alternative embodiment, B7-H3 can be determined in a biological sample by a competitive immunoassay using a B7-H3 protein standard labeled with a detectable substance and an unlabeled anti-B7-H3 antibody. In this assay, a biological sample, a labeled B7-H3 protein standard, and an anti-B7-H3 antibody are combined, and the amount of labeled B7-H3 protein standard bound to an unlabeled antibody is determined. The amount of B7-H3 in the biological sample is inversely proportional to the amount of labeled B7-H3 protein standard bound to the anti-B7-H3 antibody.

The immunoassay and method disclosed herein can be used for a variety of purposes. In an embodiment, the anti-B7-H3 antibody can be used to detect the production of B7-H3 in cells in cell culture. In another embodiment, the antibody can be used to determine the amount of B7-H3 in a biological sample, such as a tissue sample or a blood or serum sample. In some examples, the B7-H3 is a cell surface B7-H3.

### Kit

The present disclosure provides a pharmaceutical package or a kit comprising one or more containers containing one or more ingredients of the pharmaceutical composition described herein, such as the antibody or antigen-binding fragment disclosed herein.

In a particular embodiment, the kit comprises a first container containing the antibody disclosed herein. In a particular embodiment, the kit comprises a first container, which is a vial containing the antibody as a lyophilized sterile powder under vacuum, and a second container containing a pharmaceutically acceptable fluid.

In a particular embodiment, provided herein is an injection device containing an antibody. In a particular embodiment, the injection device comprises an antibody in a sterile solution. In a particular embodiment, the injection device is a syringe.

In an embodiment, the kit is provided for use in detecting B7-H3 in a biological sample, such as a blood sample or a tissue sample. For example, in order to confirm a diagnosis of cancer in a subject, a biopsy may be performed to obtain a tissue sample for histological examination. A kit for detecting a polypeptide typically comprises an anti-B7-H3 antibody, such as any of the monoclonal antibodies disclosed herein. In a further embodiment, the antibody is labeled (e.g., with a fluorescent, radioactive, or enzymatic label).

In an embodiment, the kit includes instructive material disclosing a manner in which an anti-B7-H3 antibody is used. The explanatory material may be in written, electronic form (e.g., computer floppy disks or optical disks), or visual (e.g., video files). The kit may also include additional components to facilitate the application for which the kit is designed. Thus, for example, the kit may additionally contain means for detecting the label (e.g., an enzyme substrate for the enzymatic label, a filter set for detecting the fluorescent label, a suitable secondary label such as a secondary antibody). The kit may also include buffers and other reagents conventionally used to carry out a particular method. Such kits and suitable contents are well known to those skilled in the art.

In an embodiment, the diagnostic kit comprises an immunoassay. A method of detecting B7-H3 in a biological sample typically includes the step of contacting the biological sample with an anti-B7-H3 antibody. The antibody is allowed to specifically bind under immunoreactive conditions to form an immune complex, and the presence of the immune complex (bound antibody) is detected directly or indirectly.

### EXAMPLES

The following Examples are given for the purpose of illustrating various embodiments of the present disclosure, but are not meant to limit the present disclosure in any way. The present Examples, as well as the methods described herein, are presently representative of preferred embodiments, are exemplary, and are not intended to be a limit of the scope of the present disclosure. Variations and other uses included within the spirit of the present disclosure, as defined by the scope of the claims, will occur to those skilled in the art.

### Example 1. Generation of Antibody Hybridoma

### 1.1 Mouse Immunization

All mice were raised in a barrier system, and ten 6-8 week old female Balb/c mice (SPF grade) were immunized by multiple-site subcutaneous injections with the fusion protein of human-derived B7-H3 protein (Leu 29 to Pro 245 of UniProt KB: Q5ZPR3) and Fc (Pro100 to Lys330 of UniProt KB: P01857) as the antigen. Each mouse was immunized with 100 µg of antigen primarily, followed by 50 µg of antigen per mouse. Serum titers of mice were determined after three immunizations.

### 1.2 Cell Fusion

Five mice with good immunization titer detection results were intraperitoneally injected with human B7-H3-His protein (ACRO, B73-H52E2) plus CHO-S-Mouse-B7-H3 recombinant cell line (purchased from iCarTab Biotechnology Co. Ltd., AKD052A) for boost immunization. Three days later, the mice were sacrificed by cervical dislocation. The spleen of the mouse was obtained under sterile conditions to prepare a single cell suspension of B cells, which was mixed with non-secretory SP2/0 myeloma cells at a ratio of 1:1. Cell fusion was performed using a BTX cell electrofusion instrument. After electrofusion, all cells were immediately suspended in complete medium (DMEM, 20% FBS, and HAT (a mixture of hypoxanthine, aminopterin, and thymidine) and seeded into 96-well plates, and cultured in a CO₂ incubator.

### 1.3 Hybridoma Screening

After 12 days of fusion, 100 µL of culture supernatant was taken from the 96-well plates and subjected to ELISA assays with B7-H3 antigen proteins of different species. According to the fusion titer detection results, clones with high OD values and good growth were selected. Further screening by flow cytometry was performed to select clones capable of binding to cells overexpressing human, cynomolgus monkey, or mouse B7-H3 (CHO-S-human B7-H3, CHO-S-Cyno B7-H3 or CHO-S-mouse B7-H3). Positive clones were picked based on ELISA and FACS results and subjected to two rounds of subcloning until monoclonal formation.

Specific ELISA and FACS experimental methods are as follows:
1) ELISA screening:
   96-well ELISA plates were coated with 100 µl Human B7-H3-His, Cyno B7-H3-His, or Mouse B7-H3-Fc recombinant protein (final concentration 0.5 µg/mL) at 4 °C overnight. After washing 3 times with PBST (containing 0.5% Tween), the blocking buffer (3% BSA) was added to the wells to block at 37 °C for 2 hours, and the plates were washed 3 times with PBST. 100 µl of hybridoma supernatant was added and incubated at room temperature for 1 hour. After washing with PBST for 3 times, 100 µL of 10000-fold diluted HRP Anti-Mouse IgG H&L (Biodragon, CAT # BF03001) was added to each well. The plates were incubated at room temperature for 1 hour and washed three times. 100 µL/well of TMB Substrate solution was added, and the plates were incubated in the dark at room temperature for 10 minutes. 50 µL of stop solution was added to stop the reaction, and the OD values in the wells were read with a microplate reader.
2) FACS Screening
   The CHO-S cell line, the CHO-S-human B7-H3 cell line, the CHO-S-Cyno B7-H3 cell line, and the CHO-S-mouse B7-H3 cell line were each aliquoted into several portions with 3 x 10⁵ cells per portion. 50µL of hybridoma supernatant was incubated with each of the target cells, mixed thoroughly, and then incubated at room temperature for 1 hour. The cells were washed 3 times with PBS, and PE-labeled Anti-human IgG (Invitrogen, CAT # 12-4998-82) was added, mixed thoroughly, and then incubated in the dark at room temperature for 30 minutes. The cells were washed three times with PBS and detected using flow cytometry.

### 1.4 Sequencing of Monoclonal Antibodies

According to the results of ELISA and FACS, the positive single monoclonal hybridoma cells meeting the requirements were picked for expanded culture. Cells were lysed using Trizol, and total RNA from hybridoma cells was extracted according to standard methods. After reverse transcription of the total RNA into cDNA samples using a reverse transcription kit, the cDNAs of the heavy chain variable region and the light chain variable region of the antibody were amplified by PCR using hybridoma sequencing primers. After purification and recovery, the PCR product fragments were subcloned into a T-vector, and clones were picked for sequencing.

### Example 2. Generation and Characterization of Chimeric Antibodies

### 2.1 Generation of Chimeric Antibodies

After sequencing, the antibody heavy chain variable region sequences (VH) and light chain variable region sequences (VL) were obtained, as shown in Table 1. The underlined sequences in Table 1 are CDR regions defined by the Kabat system. The heavy chain variable region gene fragments of the mouse antibody were constructed into the binding region of the multiple cloning site on the eukaryotic expression plasmid pCDNA3.4-IgG1, thereby constructing a heavy chain expression plasmid. pCDNA3.4-IgG1 was obtained by ligating the coding nucleic acid for the heavy chain constant region fragment (GenBank: ACK87036.1, Seq 2-330) into the backbone plasmid pCDNA3.4 (purchased from Invitrogen, A14697). The nucleic acid encoding the light chain variable region gene of the mouse antibody was constructed into the binding region of the multiple cloning site on the eukaryotic expression plasmid pCDNA3.4-IgKc, thereby constructing a light chain expression plasmid. pCDNA3.4-IgKc was obtained by ligating the light chain constant region fragment (GenBank: AAA59000.1, Seq 31-137) into the backbone pCDNA3.4. The constructed light chain expression plasmid and heavy chain expression plasmid were transformed into *E. coli* DH5a competent cells. Single colonies were picked, and after sequencing confirmation, plasmids were extracted on a large scale to obtain the expression plasmids for the chimeric antibody light chain and the expression plasmids for the chimeric antibody heavy chain.

The plasmids containing the heavy chain gene and the plasmids containing the light chain gene were transiently co-transfected into 293F (Gibco, R79007) cells. The transfected cells were continued to be cultured for 4-6 days, and were centrifuged at 8000 rpm for 20 minutes. The cell culture supernatant containing the target protein was collected and loaded onto a Protein A affinity chromatography column to obtain purified antibody.

The sequence of the positive control antibody M30-H1-L4 was derived from patent CN103687945A, and was also expressed and purified using the above methods.

The sequence of the positive control antibody hmAb-C was derived from CN109069633A and was also expressed and purified using the above methods.

**Table 1. Amino Acid Sequences of the Light and Heavy Chain Variable Regions of Mouse Anti-B7-H3 Antibodies**

| Antibody Clone ID | | SE Q ID N O: | Amino acid sequence |
|---|---|---|---|
| 16-B6-G3-E10 | VH | 1 | EVQLQQSGTELVRPGALVKLSCKTSGFNIKDCYMHWVKQRPEQGLEWIGWID PENGNTIYDPKFQGKATITADTSSYTAYLQLSSLTSEDTAVYYCARSRARGYAM DYWGQGTSVTVTSS |
| | VL | 2 | DIVMTQSPASLAVSLGQRATISCRASQSVSTSSYSYMHWYRQIPGQPPKLLIKYA SNLESGVPARFSGSGSGTDFTLNIHPVEEEDTATYYCQHSWEIPRTFGGGTKLEL K |
| 48-B4-D3-E3 | VH | 3 | EVQLQESGGDLVKPGGSLKLSCAASGFTFSSYGMSWVRQTPEKRLEWVASISG SGSYTYYPDSLKGRFTISRDNAKNNLYLQMSSLRSEDTALYYCARQDGYWYF |
| | | | DVWGAGTTVTVSS |
| | VL | 4 | DILMTQTPKFLLVSAGDRVTITCKASQSVSNDVAWYQQKPGQSPQLLIYYASHR YTGVPDRFTGSGYGTDFTFTISTVQAEDLAVYFCQQDNSSPLTFGAGTKLELK |
| 41-G9-G4-D3 | VH | 5 | EVQVVESGPELVKPGASVKMSCKTSAYTFSDSVLSWVKQRTGQGLEWIGEIYP RGNVTHYNGKFKGKATLTADKSSNTAFMHFSSLTSEDSAVYFCARWNNFFFDY WGQGSTLTVSS |
| | VL | 6 | DIVMTQPPLSLPVTLGDLASISCRSSQSLVHSNGITYLHWYLQKPGQSPKLLIYK VSNRFSGVPDRFSGSGSGTDFTLKISRVEAEDLGVYFCSQSTHFPWTFGGGTKL ELK |
| 19-F1-E9-B11 | VH | 7 | EVQLQQSGPSLVKPSQTLSLTCSVTGGSITSFYWNWIRKFPGNKLEYMGYISSS GSTYYNPSLKSRISITRDTSKNQSYLQLNSVTAEDTATYYCARGGVHYGFGDY WGQGTSVTVSS |
| | VL | 8 | DIVMTQSAPSVPVTPGESVSISCRSSKSLLHSNGNTYLYWFLQRPGQSPQLLIYR MSNLASGVPDRFSGSGSGTAFTLRISRVEAEDVGVYYCMQHLEYPLTFGAGTK LELK |
| 9-F1-D9-F10 | VH | 9 | EVQLVESGGGLVQPGGSLRVSCATSGFTFSDYYMSWVRQPPGKALEWLGFIRN KINGYTTEYSASVKGRFTISRDNSQSILYLQMNTLRAEDSATYYCTRDSSQRPF AYWGQGTLVTVSA |
| | VL | 10 | DIVMTQSPSSLSVSAGEKVTMTCKSSQSLLNSRSQKNFLAWYQQKPGQPPKLLI YGASTRESGVPDRFTGSGSGTDFTLTISNVQAEDLAVYYCQNDLTYPLTFGAGT KLELK |
| 16-D10-E1-E1 | VH | 11 | EVQLQQSGPELVRPGVSVKISCKGSGYTFTDYAMHWVKQSHAKSLEWIGVINT YSGNTYYSQKFKGKATMTVDKSSSTAYMELARLTSEDSAIYYCARGAGYFPY WYFDVWGAGTTVTVSS |
| | VL | 12 | DIQMIQSPSYLAASPGETITINCRASKSISKYLAWYQEKPGKTNKLLIYSGSTLQ SGIPSRFSGSGSGTDFTLTISSLEPEDFAMYYCQQHYEYPYTFGGGTKLEIK |
| 48-B4-F4-E4 | VH | 3 | EVQLQESGGDLVKPGGSLKLSCAASGFTFSSYGMSWVRQTPEKRLEWVASISG SGSYTYYPDSLKGRFTISRDNAKNNLYLQMSSLRSEDTALYYCARQDGYWYF DVWGAGTTVTVSS |
| | VL | 13 | DVLMTQTPLSLPVSLGDQVSISCRASQSLANSYGKTYLSWYLHKPGQSPQLLIY GISNRFSGVPDRFSGSGSGTDFTLKISTIKPEDLGMYYCLHGTHQPLTFGAGTKL ELK |
| 28-B5-B6-C6 | VH | 14 | EVKLQESGPGLVKPSQSLSLTCTVTGYSITSDYAWNWIRQFPGNILEWMGYISY SGSASYNPSLQSRISVTRDTSKNQFFLQLNSVTTEDTATYYCVRSLGRRWYFGF WGAGTTVTVSS |
| | VL | 15 | DIVLTQSPSSLAMTVGQKVTMSCKSSQNLLNSSNQKNYLAWYQQKPGQSPKL LVYFASTRESGVPDRFIGSGSGTDFTLTISSVQAEDLADYFCQQHYSTPLTFGGG TKLEIK |
| 10-A3-C5-C8 | VH | 16 | EVQLVESGGGLVKPGGSLKLSCAVSGFTFSSYAMSWVRQTPEKRLEWVASITD GGITFYSDSLKGRFTISRDNSGNILYLQMSSLRSEDTAMYFCARGSITTDWYFD VWGAGTTVTVSS |
| | VL | 17 | DIVMTQAPLSLPVSLGDQASISCRSSQSLVHSNGNTYLHWYLQKPGQSPKLLIY KVSNRFSGVPDRFSGSGSGTDFTLKISRVEAEDLGVYFCSQSTHVPPYTFGGGT KLEIK |
| 29-A9-G8-F6 | VH | 18 | EVQLQQSGPELVRPGVSVKISCKGSGYTFTDYSMHWVKQSHAKSLEWIGVINT SSGNTYYNLKFKGKATMTVDKSSSTAYMELARLTSEDSAIYYCSKYYGSSFHW YFDVWGAGTTVTVSS |
| | VL | 19 | DIVMTQSPVIMSASPGEKVTMTCSASSSVSYMHWYQQKSSTSPKLWIYDTSKL ASGVPGRFSGTGSGKSHSLTISSMEAEDVATYYCFQGSGYPLTFGSGTKLEIK |
| 9-A2-G3-H11 | VH | 20 | EVQLQQSGPELVRPGESVKISCKGSGYTFTDYAIHWVKQSHAKSLEWIGVISTY YDNTYYNQKFKGRATMTVDKSSSTAYMELARLTSEDSAIYYCARGYGSSFNW YFDVWGAGTTVTVSS |
| | VL | 21 | DIVMTQSPSSLAVSAGEKVTMSCKSSQSLLNSRTRKNYLAWYQQKPGQSPKLL IYWASTRESGVPDRFTGSGSGTDFTLTITSVQAEDLAVYYCKQSYNLYTFGGGT KLEIK |
| 28-B1-E8-C11 | VH | 22 | EVKLVESGAVLVKPGASVKISCKASGYTLSDFNMDWVKQSQGKSLEWVGDIN PNYDTITYNQKFRGKATLTVDRSSNTAYMELRSLTSEDSAVYYCARGELGRYFY VWGAGTTVTVSS |
| | VL | 23 | DIQLTQPPASLSASVGETVTITCRASENVYSYLAWYRQKQGRSPQLLVYNAKTL PEGVPSRFSGSGSGTQFSLKINSLQTEDLGDYYCQHHYGTPWTFGGGTKLEIK |
| 28-F8-C4-A8 | VH | 24 | EVQLQQSGAELVQPGASMKLSCKTSGYTFTTYDINWVRQRPEQGLEWIGWIFP GDGTTKYNEKFKGKATLTTDKSSSTAYMHLSRLTSEDSVVYFCARTPWFDYW GQGTTLTVSS |
| | VL | 25 | DIVLSQSPAIMSASPGEKVIMTCSASSSISYMYWYQQKPGTSPKRWIYDTSKLA SGVPARFSVSGSGTSYSLTISSMEAEDAATYYCHQRSSYPTFGGGTKLELK |
| 1-GS-E3-E2 | VH | 265 | EVQLQQSGPELVRPGVSVKISCKGSGYTFTDYAMHWVRQSHAKSLECIGVINT YSGNTNYNQKFKGKATMTVDRSSSTAYMELARLTSEDSAIYYCARVYGNYPY WYFDVWGAGTTVT |
| | VL | 266 | DILMTQSPAIMSASPGEKVTMTCRASSSVSSSYLHWYQQKSGASPKLWIYSTSN LASGVPARFSGSGSGTSYSLTISSVEAEDAATYYCQQYSGYPLTFGAGTKLE |
| 34-H11-D9-F6 | VH | 267 | EVQLQQSGAELVKPRASVRLSCRASGYTFTSYWMHWVKQRPGQGLEWIGMI HPNSGSTNYNEKFKSKATLTVDKSSSTAYMQLSSLTSEDSAVYFCARERFGLLR YFDYWGQGTTLTVSS |
| | VL | 268 | DIVMTQSPSSMYASLGERVTITCKASQDINSFLSWFQQKPGKSPKTLLYRANRL MDGVPSRFSGSGSGQDFSLTISSREYEDMGIYYCLQYDEFPFTFGSGTKLELK |

**Table 2. Amino Acid Sequences of CDRs and FRs of the Light and Heavy Chain of Mouse Anti-B7-H3 antibodies**

| Table 2-1 Amino Acid Sequences of CDRs and FRs of the Heavy Chain of Mouse Anti-B7-H3 antibodies | | | | | | | |
|---|---|---|---|---|---|---|---|
| Antib ody heavy chain | FR1 | CDR1 | FR2 | CDR2 | FR3 | CDR3 | FR4 |
| 16-B6-G3-E10 | EVQLQQSGTELVRPGA LVKLSCKTS (SEQ ID NO: 43) | GFNIKD C (SEQ ID NO: 44) | YMHWVKQRPEQ GLEWIGW (SEQ ID NO: 45) | IDPENGNT (SEQ ID NO: 46) | IYDPKFQGKATITADTSSYTAYLQLS SLTSEDTAVYYC (SEQ ID NO: 47) | ARSRARGYA MDY (SEQ ID NO: 48) | WGQGTSV TVTSS (SEQ ID NO: 49) |
| 48-B4-D3-E3 | EVQLQESGGDLVKPGG SLKLSCAAS (SEQ ID NO: 50) | GFTFSS Y (SEQ ID NO: 51) | GMSWVRQTPEKR LEWVAS (SEQ ID NO: 52) | TSGSGSYT (SEQ ID NO: 53) | YYPDSLKGRFTISRDNAKNNLYLQ MSSLRSEDTALYYC (SEQ ID NO: 54) | ARQDGYWY FDV (SEQ ID NO: 55) | WGAGTTV TVSS (SEQ ID NO: 56) |
| 41-G9-G4-D3 | EVQVVESGPELVKPGA SVKMSCKTS (SEQ ID NO: 57) | AYTFSD SV (SEQ ID NO: 58) | LSWVKQRTGQGL EWIGE (SEQ ID NO: 59) | TYPRGNVT (SEQ ID NO: 60) | HYNGKFKGKATLTADKSSNTAFMH FSSLTSEDSAVYFC (SEQ ID NO: 61) | ARWNNFFFD Y (SEQ ID NO: 62) | WGQGSTL TVSS (SEQ ID NO: 63) |
| 19-F1-E9-B11 | EVQLQQSGPSLVKPSQ TLSLTCSVT (SEQ ID NO: 64) | GGSITSF Y (SEQ ID NO: 65) | WNWIRKFPGNKL EYMGY (SEQ ID NO: 66) | TSSSGSTY (SEQ ID NO: 67) | YNPSLKSRISITRDTSKNQSYLQLNS VTAEDTATYYC (SEQ ID NO: 68) | ARGGVHYGF GDY (SEQ ID NO: 69) | WGQGTSV TVSS (SEQ ID NO: 70) |
| 9-F1-D9-F10 | EVQLVESGGGLVQPGG SLRVSCATS (SEQ ID NO: 71) | GFTFSD YY (SEQ ID NO: 72) | MSWVRQPPGKAL EWLGF (SEQ ID NO: 73) | IRNKINGYTT (SEQ ID NO: 74) | EYSASVKGRFTISRDNSQSILYLQM NTLRAEDSATYYC (SEQ ID NO: 75) | TRDSSQRPFA Y (SEQ ID NO: 76) | WGQGTLV TVSA (SEQ ID NO: 77) |
| 16-D10-E1-E1 | EVQLQQSGPELVRPGV SVKISCKGS (SEQ ID NO: 78) | GYTFTD YA (SEQ ID NO: 79) | MHWVKQSHAKS LEWIGV (SEQ ID NO: 80) | INTYSGNT (SEQ ID NO: 81) | YYSQKFKGKATMTVDKSSSTAYME LARLTSEDSAIYYC (SEQ ID NO: 82) | ARGAGYFPY WYFDV (SEQ ID NO: 83) | WGAGTTV TVSS (SEQ ID NO: 56) |
| 48-B4-F4-E4 | EVQLQESGGDLVKPGG SLKLSCAAS (SEQ ID NO: 50) | GFTFSS YGMS (SEQ ID NO: 84) | WVRQTPEKRLEW VA (SEQ ID NO: 85) | SISGSGSYTYY PDSLKG (SEQ ID NO: 86) | RFTISRDNAKNNLYLQMSSLRSEDT ALYYCAR (SEQ ID NO: 87) | QDGYWYFD V (SEQ ID NO: 88) | WGAGTTV TVSS (SEQ ID NO: 56) |
| 28-B5-B6-C6 | EVKLQESGPGLVKPSQ SLSLTCTVT (SEQ ID NO: 89) | GYSITS DYA (SEQ ID NO: 90) | WNWIRQFPGNIL EWMGYI (SEQ ID NO: 91) | SYSGSA (SEQ ID NO: 92) | SYNPSLQSRISVTRDTSKNQFFLQLN SVTTEDTATYYC (SEQ ID NO: 93) | VRSLGRRWY FGF (SEQ ID NO: 94) | WGAGTTV TVSS (SEQ ID NO: 56) |
| 10-A3-C5-C8 | EVQLVESGGGLVKPGG SLKLSCAVS (SEQ ID NO: 95) | GFTFSS YA (SEQ ID NO: 96) | MSWVRQTPEKRL EWVAS (SEQ ID NO: 97) | IT**DG**GIT (SEQ ID NO: 98) | FYSDSLKGRFTISRDNSGNILYLQMS SLRSEDTAMYFC (SEQ ID NO: 99) | ARGSITTDW YFDV (SEQ ID NO: 100) | WGAGTTV TVSS (SEQ ID NO: 56) |
| 29-A9-G8-F6 | EVQLQQSGPELVRPGV SVKISCKGS (SEQ ID NO: 78) | GYTFTD YS (SEQ ID NO: 101) | MHWVKQSHAKS LEWIGV (SEQ ID NO: 80) | INTSSGNT (SEQ ID NO: 102) | YYNLKFKGKATMTVDKSSSTAYME LARLTSEDSAIYYC (SEQ ID NO: 103) | SKYYGSSFH WYFDV (SEQ ID NO: 104) | WGAGTTV TVSS (SEQ ID NO: 56) |
| 9-A2-G3-H11 | EVQLQQSGPELVRPGE SVKISCKGS (SEQ ID NO: 105) | GYTFTD YA (SEQ ID NO: 79) | IHWVKQSHAKSL EWIGV (SEQ ID NO: 106) | TSTYYDNT (SEQ ID NO: 107) | YYNQKFKGRATMTVDKSSSTAYME LARLTSEDSAIYYC (SEQ ID NO: 108) | ARGYGSSFN WYFDV (SEQ ID NO: 109) | WGAGTTV TVSS (SEQ ID NO: 56) |
| 28-B1-E8-C11 | EVKLVESGAVLVKPGA SVKISCKAS (SEQ ID NO: 110) | GYTLSD FN (SEQ ID NO: 111) | MDWVKQSQGKS LEWVGD (SEQ ID NO: 112) | INPNYDTI (SEQ ID NO: 113) | TYNQKFRGKATLTVDRSSNTAYME LRSLTSEDSAVYYC (SEQ ID NO: 114) | ARGELGRYF YV (SEQ ID NO: 115) | WGAGTTV TVSS (SEQ ID NO: 56) |
| 28-F8-C4-A8 | EVQLQQSGAELVQPGA SMKLSCKTS (SEQ ID NO: 116) | GYTFTT YD (SEQ ID NO: 117) | INWVRQRPEQGL EWIGW (SEQ ID NO: 118) | IFPGDGTT (SEQ ID NO: 119) | KYNEKFKGKATLTTDKSSSTAYMH LSRLTSEDSVVYFC (SEQ ID NO: 120) | ARTPWFDY (SEQ ID NO: 121) | WGQGTTL TVSS (SEQ ID NO: 122) |

**Table 2-2 Amino Acid Sequences of CDRs and FRs of the Light Chain of Mouse Anti-B7-H3 Antibodies**

| Antib ody light chain | FR1 | CDR1 | FR2 | CDR2 | FR3 | CDR3 | FR4 |
|---|---|---|---|---|---|---|---|
| 16-B6-G3-E10 | DIVMTQSPASLAVSLGQR ATISCRAS (SEQ ID NO: 134) | QSVSTSSYSY (SEQ ID NO: 135) | MHWYRQIPGQPP KLLIK (SEQ ID NO: 136) | YAS | NLESGVPARFSGSGSGTDFTLNIHPV EEEDTATYYC (SEQ ID NO: 138) | QHSWEIP RT (SEQ ID NO: 139) | FGGGTKLE LK (SEQ ID NO: 140) |
| 48-B4-D3-E3 | DILMTQTPKFLLVSAGDR VTITCKAS (SEQ ID NO: 141) | QSVSND (SEQ ID NO: 142) | VAWYQQKPGQSP QLLIY (SEQ ID NO: 143) | YAS | HRYTGVPDRFTGSGYGTDFTFTISTV QAEDLAVYFC (SEQ ID NO: 144) | QQDNSSP LT (SEQ ID NO: 145) | FGAGTKLE LK (SEQ ID NO: 146) |
| 41-G9-G4-D3 | DIVMTQPPLSLPVTLGDLA SISCRSS (SEQ ID NO: 147) | QSLVHSNGITY (SEQ ID NO: 148) | LHWYLQKPGQSP KLLIY (SEQ ID NO: 149) | KVS |  (SEQ ID NO: 151) | SQSTHFP WT (SEQ ID NO: 152) | FGGGTKLE LK (SEQ ID NO: 140) |
| 19-F1-E9-B11 | DIVMTQSAPSVPVTPGESV SISCRSS (SEQ ID NO: 153) | KSLLHSNGNT Y (SEQ ID NO: 154) | LYWFLQRPGQSP QLLIY (SEQ ID NO: 155) | RMS | NLASGVPDRFSGSGSGTAFTLRISRV EAEDVGVYYC (SEQ ID NO: 157) | MQHLEY PLT (SEQ ID NO: 158) | FGAGTKLE LK (SEQ ID NO: 146) |
| 9-F1-D9-F10 | DIVMTQSPSSLSVSAGEKV TMTCKSS (SEQ ID NO: 159) | QSLLNSRSQKN F (SEQ ID NO: 160) | LAWYQQKPGQPP KLLIY (SEQ ID NO: 161) | GAS | TRESGVPDRFTGSGSGTDFTLTISNV QAEDLAVYYC (SEQ ID NO: 163) | QNDLTY PLT (SEQ ID NO: 164) | FGAGTKLE LK (SEQ ID NO: 146) |
| | | | | | | | |
| 16-D10-E1-E1 | DIQMIQSPSYLAASPGETIT INCRAS (SEQ ID NO: 165) | KSISKY (SEQ ID NO: 166) | LAWYQEKPGKTN KLLIY (SEQ ID NO: 167) | SGS | TLQSGIPSRFSGSGSGTDFTLTISSLEP EDFAMYYC (SEQ ID NO: 169) | QQHYEY PYT (SEQ ID NO: 170) | FGGGTKLEI K (SEQ ID NO: 171) |
| 48-B4-F4-E4 | DVLMTQTPLSLPVSLGDQ VSISC (SEQ ID NO: 172) | RASQSLANSY GKTYL (SEQ ID NO: 173) | SWYLHKPGQSPQ LLIY (SEQ ID NO: 174) | GISN RFS (SEQ ID NO: 175) | GVPDRFSGSGSGTDFTLKISTIKPEDL GMYYC (SEQ ID NO: 176) | LHGTHQ PLT (SEQ ID NO: 177) | FGAGTKLE LK (SEQ ID NO: 146) |
| 28-B5-B6-C6 | DIVLTQSPSSLAMTVGQK VTMSCKSS (SEQ ID NO: 178) | QNLLNSSNQK NY (SEQ ID NO: 179) | LAWYQQKPGQSP KLLVY (SEQ ID NO: 180) | FAS | TRESGVPDRFIGSGSGTDFTLTISSVQ AEDLADYFC (SEQ ID NO: 182) | QQHYST PLT (SEQ ID NO: 183) | FGGGTKLEI K (SEQ ID NO: 171) |
| 10-A3-C5-C8 | DIVMTQAPLSLPVSLGDQ ASISCRSS (SEQ ID NO: 184) | QSLVHSNGNT Y (SEQ ID NO: 185) | LHWYLQKPGQSP KLLIY (SEQ ID NO: 149) | KVS |  (SEQ ID NO: 151) | SQSTHVP PYT (SEQ ID NO: 186) | FGGGTKLEI K (SEQ ID NO: 171) |
| 29-A9-G8-F6 | DIVMTQSPVIMSASPGEKV TMTCSAS (SEQ ID NO: 187) | SSVSY (SEQ ID NO: 188) | MHWYQQKSSTSP KLWIY (SEQ ID NO: 189) | DTS | KLASGVPGRFSGTGSGKSHSLTISSM EAEDVATYYC (SEQ ID NO: 191) | FQGSGYP LT (SEQ ID | FGSGTKLEI K (SEQ ID NO: |
| | | | | | | NO: 192) | |
| 9-A2-G3-H11 | DIVMTQSPSSLAVSAGEK VTMSCKSS (SEQ ID NO: 194) | QSLLNSRTRKN Y (SEQ ID NO: 195) | LAWYQQKPGQSP KLLIY (SEQ ID NO: 196) | WAS | TRESGVPDRFTGSGSGTDFTLTITSVQ AEDLAVYYC (SEQ ID NO: 198) | KQSYNL YT (SEQ ID NO: 199) | FGGGTKLEI K (SEQ ID NO: 171) 193) |
| 28-B1-E8-C11 | DIQLTQPPASLSASVGETV TITCRAS (SEQ ID NO: 200) | ENVYSY (SEQ ID NO: 201) | LAWYRQKQGRSP QLLVY (SEQ ID NO: 202) | NAK | TLPEGVPSRFSGSGSGTQFSLKINSLQ TEDLGDYYC (SEQ ID NO: 204) | QHHYGT P (SEQ ID NO: 205) | WTFGGGTK LEIK (SEQ ID NO: 206) |
| 28-F8-C4-A8 | DIVLSQSPAIMSASPGEKVI MTCSAS (SEQ ID NO: 207) | SSISY (SEQ ID NO: 208) | MYWYQQKPGTSP KRWIY (SEQ ID NO: 209) | DTS | KLASGVPARFSVSGSGTSYSLTISSM EAEDAATYYC (SEQ ID NO: 210) | HQRSSYP T (SEQ ID NO: 211) | FGGGTKLE LK (SEQ ID NO: 140) |

**Table 3. Amino Acid Sequences of the Light and Heavy Chains of the Mouse Anti-B7-H3 Chimeric Antibodies**

| Clone ID | | Full-length sequence |
|---|---|---|
| 16-B6-G3-E10 | Heavy chain SEQ ID NO: 218 | EVQLQQSGTELVRPGALVKLSCKTSGFNIKDCYMHWVKQRPE QGLEWIGWIDPENGNTIYDPKFQGKATITADTSSYTAYLQLSSL TSEDTAVYYCARSRARGYAMDYWGQGTSVTVTSSASTKGPS VFPLAPSSKSTSGGTAALGCLVKDYFPEPVTVSWNSGALTSGV HTFPAVLQSSGLYSLSSVVTVPSSSLGTQTYICNVNHKPSNTKV DKKVEPKSCDKTHTCPPCPAPELLGGPSVFLFPPKPKDTLMISR TPEVTCVVVDVSHEDPEVKFNWYVDGVEVHNAKTKPREEQY NSTYRVVSVLTVLHQDWLNGKEYKCKVSNKALPAPIEKTISK AKGQPREPQVYTLPPSRDELTKNQVSLTCLVKGFYPSDIAVEW ESNGQPENNYKTTPPVLDSDGSFFLYSKLTVDKSRWQQGNVFS CSVMHEALHNHYTQKSLSLSPGK |
| | Light chain SEQ ID NO: 219 | DIVMTQSPASLAVSLGQRATISCRASQSVSTSSYSYMHWYRQI PGQPPKLLIKYASNLESGVPARFSGSGSGTDFTLNIHPVEEEDT ATYYCQHSWEIPRTFGGGTKLELKRTVAAPSVFIFPPSDEQLKS GTASVVCLLNNFYPREAKVQWKVDNALQSGNSQESVTEQDS KDSTYSLSSTLTLSKADYEKHKVYACEVTHQGLSSPVTKSFNR GEC |
| 48-B4-D3-E3 | Heavy chain SEQ ID NO: 220 | EVQLQESGGDLVKPGGSLKLSCAASGFTFSSYGMSWVRQTPE KRLEWVASISGSGSYTYYPDSLKGRFTISRDNAKNNLYLQMSS LRSEDTALYYCARQDGYWYFDVWGAGTTVTVSSASTKGPSV FPLAPSSKSTSGGTAALGCLVKDYFPEPVTVSWNSGALTSGVH TFPAVLQSSGLYSLSSVVTVPSSSLGTQTYICNVNHKPSNTKVD KKVEPKSCDKTHTCPPCPAPELLGGPSVFLFPPKPKDTLMISRT PEVTCVVVDVSHEDPEVKFNWYVDGVEVHNAKTKPREEQYN STYRVVSVLTVLHQDWLNGKEYKCKVSNKALPAPIEKTISKA KGQPREPQVYTLPPSRDELTKNQVSLTCLVKGFYPSDIAVEWE SNGQPENNYKTTPPVLDSDGSFFLYSKLTVDKSRWQQGNVFS CSVMHEALHNHYTQKSLSLSPGK |
| | Light chain SEQ ID NO: 221 | DILMTQTPKFLLVSAGDRVTITCKASQSVSNDVAWYQQKPGQ SPQLLIYYASHRYTGVPDRFTGSGYGTDFTFTISTVQAEDLAVY FCQQDNSSPLTFGAGTKLELKRTVAAPSVFIFPPSDEQLKSGTA SVVCLLNNFYPREAKVQWKVDNALQSGNSQESVTEQDSKDST YSLSSTLTLSKADYEKHKVYACEVTHQGLSSPVTKSFNRGEC |
| 41-G9-G4-D3 | Heavy chain SEQ ID NO: 222 | EVQVVESGPELVKPGASVKMSCKTSAYTFSDSVLSWVKQRTG QGLEWIGEIYPRGNVTHYNGKFKGKATLTADKSSNTAFMHFS SLTSEDSAVYFCARWNNFFFDYWGQGSTLTVSSASTKGPSVFP LAPSSKSTSGGTAALGCLVKDYFPEPVTVSWNSGALTSGVHTF PAVLQSSGLYSLSSVVTVPSSSLGTQTYICNVNHKPSNTKVDK KVEPKSCDKTHTCPPCPAPELLGGPSVFLFPPKPKDTLMISRTPE |
| | | VTCVVVDVSHEDPEVKFNWYVDGVEVHNAKTKPREEQYNST YRVVSVLTVLHQDWLNGKEYKCKVSNKALPAPIEKTISKAKG QPREPQVYTLPPSRDELTKNQVSLTCLVKGFYPSDIAVEWESN GQPENNYKTTPPVLDSDGSFFLYSKLTVDKSRWQQGNVFSCS VMHEALHNHYTQKSLSLSPGK |
| | Light chain SEQ ID NO: 223 | DIVMTQPPLSLPVTLGDLASISCRSSQSLVHSNGITYLHWYLQK PGQSPKLLIYKVSNRFSGVPDRFSGSGSGTDFTLKISRVEAEDL GVYFCSQSTHFPWTFGGGTKLELKRTVAAPSVFIFPPSDEQLKS GTASVVCLLNNFYPREAKVQWKVDNALQSGNSQESVTEQDS KDSTYSLSSTLTLSKADYEKHKVYACEVTHQGLSSPVTKSFNR GEC |
| 19-F1-E9-B11 | Heavy chain SEQ ID NO: 224 | EVQLQQSGPSLVKPSQTLSLTCSVTGGSITSFYWNWIRKFPGNK LEYMGYISSSGSTYYNPSLKSRISITRDTSKNQSYLQLNSVTAE DTATYYCARGGVHYGFGDYWGQGTSVTVSSASTKGPSVFPLA PSSKSTSGGTAALGCLVKDYFPEPVTVSWNSGALTSGVHTFPA VLQSSGLYSLSSVVTVPSSSLGTQTYICNVNHKPSNTKVDKKV EPKSCDKTHTCPPCPAPELLGGPSVFLFPPKPKDTLMISRTPEVT CVVVDVSHEDPEVKFNWYVDGVEVHNAKTKPREEQYNSTYR VVSVLTVLHQDWLNGKEYKCKVSNKALPAPIEKTISKAKGQP REPQVYTLPPSRDELTKNQVSLTCLVKGFYPSDIAVEWESNGQ PENNYKTTPPVLDSDGSFFLYSKLTVDKSRWQQGNVFSCSVM HEALHNHYTQKSLSLSPGK |
| | Light chain SEQ ID NO: 225 | DIVMTQSAPSVPVTPGESVSISCRSSKSLLHSNGNTYLYWFLQR PGQSPQLLIYRMSNLASGVPDRFSGSGSGTAFTLRISRVEAEDV GVYYCMQHLEYPLTFGAGTKLELKRTVAAPSVFIFPPSDEQLK SGTASVVCLLNNFYPREAKVQWKVDNALQSGNSQESVTEQDS KDSTYSLSSTLTLSKADYEKHKVYACEVTHQGLSSPVTKSFNR GEC |
| 9-F1-D9-F10 | Heavy chain SEQ ID NO: 226 | EVQLVESGGGLVQPGGSLRVSCATSGFTFSDYYMSWVRQPPG KALEWLGFIRNKINGYTTEYSASVKGRFTISRDNSQSILYLQMN TLRAEDSATYYCTRDSSQRPFAYWGQGTLVTVSAASTKGPSV FPLAPSSKSTSGGTAALGCLVKDYFPEPVTVSWNSGALTSGVH TFPAVLQSSGLYSLSSVVTVPSSSLGTQTYICNVNHKPSNTKVD KKVEPKSCDKTHTCPPCPAPELLGGPSVFLFPPKPKDTLMISRT PEVTCVVVDVSHEDPEVKFNWYVDGVEVHNAKTKPREEQYN STYRVVSVLTVLHQDWLNGKEYKCKVSNKALPAPIEKTISKA KGQPREPQVYTLPPSRDELTKNQVSLTCLVKGFYPSDIAVEWE SNGQPENNYKTTPPVLDSDGSFFLYSKLTVDKSRWQQGNVFS CSVMHEALHNHYTQKSLSLSPGK |
| | Light chain SEQ ID NO: 227 | DIVMTQSPSSLSVSAGEKVTMTCKSSQSLLNSRSQKNFLAWYQ QKPGQPPKLLIYGASTRESGVPDRFTGSGSGTDFTLTISNVQAE DLAVYYCQNDLTYPLTFGAGTKLELKRTVAAPSVFIFPPSDEQ LKSGTASVVCLLNNFYPREAKVQWKVDNALQSGNSQESVTEQ |
| | | DSKDSTYSLSSTLTLSKADYEKHKVYACEVTHQGLSSPVTKSF NRGEC |
| 16-D10-E1-E1 | Heavy chain SEQ ID NO: 228 | EVQLQQSGPELVRPGVSVKISCKGSGYTFTDYAMHWVKQSHA KSLEWIGVINTYSGNTYYSQKFKGKATMTVDKSSSTAYMELA RLTSEDSAIYYCARGAGYFPYWYFDVWGAGTTVTVSSASTKG PSVFPLAPSSKSTSGGTAALGCLVKDYFPEPVTVSWNSGALTS GVHTFPAVLQSSGLYSLSSVVTVPSSSLGTQTYICNVNHKPSNT KVDKKVEPKSCDKTHTCPPCPAPELLGGPSVFLFPPKPKDTLMI SRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSRDELTKNQVSLTCLVKGFYPSDIAV EWESNGQPENNYKTTPPVLDSDGSFFLYSKLTVDKSRWQQGN VFSCSVMHEALHNHYTQKSLSLSPGK |
| | Light chain SEQ ID NO: 229 | DIQMIQSPSYLAASPGETITINCRASKSISKYLAWYQEKPGKTN KLLIYSGSTLQSGIPSRFSGSGSGTDFTLTISSLEPEDFAMYYCQ QHYEYPYTFGGGTKLEIKRTVAAPSVFIFPPSDEQLKSGTASVV CLLNNFYPREAKVQWKVDNALQSGNSQESVTEQDSKDSTYSL SSTLTLSKADYEKHKVYACEVTHQGLSSPVTKSFNRGEC |
| 48-B4-F4-E4 | Heavy chain SEQ ID NO: 230 | EVQLQESGGDLVKPGGSLKLSCAASGFTFSSYGMSWVRQTPE KRLEWVASISGSGSYTYYPDSLKGRFTISRDNAKNNLYLQMSS LRSEDTALYYCARQDGYWYFDVWGAGTTVTVSSASTKGPSV FPLAPSSKSTSGGTAALGCLVKDYFPEPVTVSWNSGALTSGVH TFPAVLQSSGLYSLSSVVTVPSSSLGTQTYICNVNHKPSNTKVD KKVEPKSCDKTHTCPPCPAPELLGGPSVFLFPPKPKDTLMISRT PEVTCVVVDVSHEDPEVKFNWYVDGVEVHNAKTKPREEQYN STYRVVSVLTVLHQDWLNGKEYKCKVSNKALPAPIEKTISKA KGQPREPQVYTLPPSRDELTKNQVSLTCLVKGFYPSDIAVEWE SNGQPENNYKTTPPVLDSDGSFFLYSKLTVDKSRWQQGNVFS CSVMHEALHNHYTQKSLSLSPGK |
| | Light chain SEQ ID NO: 231 | DVLMTQTPLSLPVSLGDQVSISCRASQSLANSYGKTYLSWYLH KPGQSPQLLIYGISNRFSGVPDRFSGSGSGTDFTLKISTIKPEDLG MYYCLHGTHQPLTFGAGTKLELKRTVAAPSVFIFPPSDEQLKS GTASVVCLLNNFYPREAKVQWKVDNALQSGNSQESVTEQDS KDSTYSLSSTLTLSKADYEKHKVYACEVTHQGLSSPVTKSFNR GEC |
| 28-B5-B6-C6 | Heavy chain SEQ ID NO: 232 | EVKLQESGPGLVKPSQSLSLTCTVTGYSITSDYAWNWIRQFPG NILEWMGYISYSGSASYNPSLQSRISVTRDTSKNQFFLQLNSVT TEDTATYYCVRSLGRRWYFGFWGAGTTVTVSSASTKGPSVFP LAPSSKSTSGGTAALGCLVKDYFPEPVTVSWNSGALTSGVHTF PAVLQSSGLYSLSSVVTVPSSSLGTQTYICNVNHKPSNTKVDK KVEPKSCDKTHTCPPCPAPELLGGPSVFLFPPKPKDTLMISRTPE VTCVVVDVSHEDPEVKFNWYVDGVEVHNAKTKPREEQYNST YRVVSVLTVLHQDWLNGKEYKCKVSNKALPAPIEKTISKAKG QPREPQVYTLPPSRDELTKNQVSLTCLVKGFYPSDIAVEWESN GQPENNYKTTPPVLDSDGSFFLYSKLTVDKSRWQQGNVFSCS VMHEALHNHYTQKSLSLSPGK |
| | Light chain SEQ ID NO: 233 | DIVLTQSPSSLAMTVGQKVTMSCKSSQNLLNSSNQKNYLAWY QQKPGQSPKLLVYFASTRESGVPDRFIGSGSGTDFTLTISSVQA EDLADYFCQQHYSTPLTFGGGTKLEIKRTVAAPSVFIFPPSDEQ LKSGTASVVCLLNNFYPREAKVQWKVDNALQSGNSQESVTEQ DSKDSTYSLSSTLTLSKADYEKHKVYACEVTHQGLSSPVTKSF NRGEC |
| 10-A3-C5-C8 | Heavy chain SEQ ID NO: 234 | EVQLVESGGGLVKPGGSLKLSCAVSGFTFSSYAMSWVRQTPE KRLEWVASITDGGITFYSDSLKGRFTISRDNSGNILYLQMSSLR SEDTAMYFCARGSITTDWYFDVWGAGTTVTVSSASTKGPSVF PLAPSSKSTSGGTAALGCLVKDYFPEPVTVSWNSGALTSGVHT FPAVLQSSGLYSLSSVVTVPSSSLGTQTYICNVNHKPSNTKVDK KVEPKSCDKTHTCPPCPAPELLGGPSVFLFPPKPKDTLMISRTPE VTCVVVDVSHEDPEVKFNWYVDGVEVHNAKTKPREEQYNST YRVVSVLTVLHQDWLNGKEYKCKVSNKALPAPIEKTISKAKG QPREPQVYTLPPSRDELTKNQVSLTCLVKGFYPSDIAVEWESN GQPENNYKTTPPVLDSDGSFFLYSKLTVDKSRWQQGNVFSCS VMHEALHNHYTQKSLSLSPGK |
| | Light chain SEQ ID NO: 235 | DIVMTQAPLSLPVSLGDQASISCRSSQSLVHSNGNTYLHWYLQ KPGQSPKLLIYKVSNRFSGVPDRFSGSGSGTDFTLKISRVEAED LGVYFCSQSTHVPPYTFGGGTKLEIKRTVAAPSVFIFPPSDEQL KSGTASVVCLLNNFYPREAKVQWKVDNALQSGNSQESVTEQ DSKDSTYSLSSTLTLSKADYEKHKVYACEVTHQGLSSPVTKSF NRGEC |
| 29-A9-G8-F6 | Heavy chain SEQ ID NO: 236 | EVQLQQSGPELVRPGVSVKISCKGSGYTFTDYSMHWVKQSHA KSLEWIGVINTSSGNTYYNLKFKGKATMTVDKSSSTAYMELA RLTSEDSAIYYCSKYYGSSFHWYFDVWGAGTTVTVSSASTKG PSVFPLAPSSKSTSGGTAALGCLVKDYFPEPVTVSWNSGALTS GVHTFPAVLQSSGLYSLSSVVTVPSSSLGTQTYICNVNHKPSNT KVDKKVEPKSCDKTHTCPPCPAPELLGGPSVFLFPPKPKDTLMI SRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSRDELTKNQVSLTCLVKGFYPSDIAV |
| | | EWESNGQPENNYKTTPPVLDSDGSFFLYSKLTVDKSRWQQGN VFSCSVMHEALHNHYTQKSLSLSPGK |
| | Light chain SEQ ID NO: 237 | DIVMTQSPVIMSASPGEKVTMTCSASSSVSYMHWYQQKSSTSP KLWIYDTSKLASGVPGRFSGTGSGKSHSLTISSMEAEDVATYY CFQGSGYPLTFGSGTKLEIKRTVAAPSVFIFPPSDEQLKSGTASV VCLLNNFYPREAKVQWKVDNALQSGNSQESVTEQDSKDSTYS LSSTLTLSKADYEKHKVYACEVTHQGLSSPVTKSFNRGEC |
| 9-A2-G3-H11 | Heavy chain SEQ ID NO: 238 | EVQLQQSGPELVRPGESVKISCKGSGYTFTDYAIHWVKQSHAK SLEWIGVISTYYDNTYYNQKFKGRATMTVDKSSSTAYMELAR LTSEDSAIYYCARGYGSSFNWYFDVWGAGTTVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNTK VDKKVEPKSCDKTHTCPPCPAPELLGGPSVFLFPPKPKDTLMIS RTPEVTCVVVDVSHEDPEVKFNWYVDGVEVHNAKTKPREEQ YNSTYRVVSVLTVLHQDWLNGKEYKCKVSNKALPAPIEKTIS KAKGQPREPQVYTLPPSRDELTKNQVSLTCLVKGFYPSDIAVE WESNGQPENNYKTTPPVLDSDGSFFLYSKLTVDKSRWQQGNV FSCSVMHEALHNHYTQKSLSLSPGK |
| | Light chain SEQ ID NO: 239 | DIVMTQSPSSLAVSAGEKVTMSCKSSQSLLNSRTRKNYLAWY QQKPGQSPKLLIYWASTRESGVPDRFTGSGSGTDFTLTITSVQA EDLAVYYCKQSYNLYTFGGGTKLEIKRTVAAPSVFIFPPSDEQL KSGTASVVCLLNNFYPREAKVQWKVDNALQSGNSQESVTEQ DSKDSTYSLSSTLTLSKADYEKHKVYACEVTHQGLSSPVTKSF NRGEC |
| 28-B1-E8-C11 | Heavy chain SEQ ID NO: 240 | EVKLVESGAVLVKPGASVKISCKASGYTLSDFNMDWVKQSQG KSLEWVGDINPNYDTITYNQKFRGKATLTVDRSSNTAYMELR SLTSEDSAVYYCARGELGRYFYVWGAGTTVTVSSASTKGPSV FPLAPSSKSTSGGTAALGCLVKDYFPEPVTVSWNSGALTSGVH TFPAVLQSSGLYSLSSVVTVPSSSLGTQTYICNVNHKPSNTKVD KKVEPKSCDKTHTCPPCPAPELLGGPSVFLFPPKPKDTLMISRT PEVTCVVVDVSHEDPEVKFNWYVDGVEVHNAKTKPREEQYN STYRVVSVLTVLHQDWLNGKEYKCKVSNKALPAPIEKTISKA KGQPREPQVYTLPPSRDELTKNQVSLTCLVKGFYPSDIAVEWE SNGQPENNYKTTPPVLDSDGSFFLYSKLTVDKSRWQQGNVFS CSVMHEALHNHYTQKSLSLSPGK |
| | Light chain SEQ ID NO: 241 | DIQLTQPPASLSASVGETVTITCRASENVYSYLAWYRQKQGRS PQLLVYNAKTLPEGVPSRFSGSGSGTQFSLKINSLQTEDLGDYY CQHHYGTPWTFGGGTKLEIKRTVAAPSVFIFPPSDEQLKSGTAS VVCLLNNFYPREAKVQWKVDNALQSGNSQESVTEQDSKDST YSLSSTLTLSKADYEKHKVYACEVTHQGLSSPVTKSFNRGEC |
| 28-F8-C4-A8 | Heavy chain SEQ ID NO: 242 | EVQLQQSGAELVQPGASMKLSCKTSGYTFTTYDINWVRQRPE QGLEWIGWIFPGDGTTKYNEKFKGKATLTTDKSSSTAYMHLS RLTSEDSVVYFCARTPWFDYWGQGTTLTVSSASTKGPSVFPLA PSSKSTSGGTAALGCLVKDYFPEPVTVSWNSGALTSGVHTFPA VLQSSGLYSLSSVVTVPSSSLGTQTYICNVNHKPSNTKVDKKV EPKSCDKTHTCPPCPAPELLGGPSVFLFPPKPKDTLMISRTPEVT CVVVDVSHEDPEVKFNWYVDGVEVHNAKTKPREEQYNSTYR VVSVLTVLHQDWLNGKEYKCKVSNKALPAPIEKTISKAKGQP REPQVYTLPPSRDELTKNQVSLTCLVKGFYPSDIAVEWESNGQ PENNYKTTPPVLDSDGSFFLYSKLTVDKSRWQQGNVFSCSVM HEALHNHYTQKSLSLSPGK |
| | Light chain SEQ ID NO: 243 | DIVLSQSPAIMSASPGEKVIMTCSASSSISYMYWYQQKPGTSPK RWIYDTSKLASGVPARFSVSGSGTSYSLTISSMEAEDAATYYC HQRSSYPTFGGGTKLELKRTVAAPSVFIFPPSDEQLKSGTASVV CLLNNFYPREAKVQWKVDNALQSGNSQESVTEQDSKDSTYSL SSTLTLSKADYEKHKVYACEVTHQGLSSPVTKSFNRGEC |
| 1-G5-E3-E2 | Heavy chain SEQ ID NO: 244 | EVQLQQSGPELVRPGVSVKISCKGSGYTFTDYAMHWVRQSHA KSLECIGVINTYSGNTNYNQKFKGKATMTVDRSSSTAYMELA RLTSEDSAIYYCARVYGNYPYWYFDVWGAGTTVTASTKGPSV FPLAPSSKSTSGGTAALGCLVKDYFPEPVTVSWNSGALTSGVH TFPAVLQSSGLYSLSSVVTVPSSSLGTQTYICNVNHKPSNTKVD KKVEPKSCDKTHTCPPCPAPELLGGPSVFLFPPKPKDTLMISRT PEVTCVVVDVSHEDPEVKFNWYVDGVEVHNAKTKPREEQYN STYRVVSVLTVLHQDWLNGKEYKCKVSNKALPAPIEKTISKA KGQPREPQVYTLPPSRDELTKNQVSLTCLVKGFYPSDIAVEWE SNGQPENNYKTTPPVLDSDGSFFLYSKLTVDKSRWQQGNVFS CSVMHEALHNHYTQKSLSLSPGK |
| | Light chain SEQ ID NO: 245 | DILMTQSPAIMSASPGEKVTMTCRASSSVSSSYLHWYQQKSGA SPKLWIYSTSNLASGVPARFSGSGSGTSYSLTISSVEAEDAATY YCQQYSGYPLTFGAGTKLERTVAAPSVFIFPPSDEQLKSGTASV VCLLNNFYPREAKVQWKVDNALQSGNSQESVTEQDSKDSTYS LSSTLTLSKADYEKHKVYACEVTHQGLSSPVTKSFNRGEC |
| 34-H11-D9-F6 | Heavy chain SEQ ID NO: 246 | EVQLQQSGAELVKPRASVRLSCRASGYTFTSYWMHWVKQRP GQGLEWIGMIHPNSGSTNYNEKFKSKATLTVDKSSSTAYMQLS SLTSEDSAVYFCARERFGLLRYFDYWGQGTTLTVSSASTKGPS VFPLAPSSKSTSGGTAALGCLVKDYFPEPVTVSWNSGALTSGV HTFPAVLQSSGLYSLSSVVTVPSSSLGTQTYICNVNHKPSNTKV DKKVEPKSCDKTHTCPPCPAPELLGGPSVFLFPPKPKDTLMISR TPEVTCVVVDVSHEDPEVKFNWYVDGVEVHNAKTKPREEQY NSTYRVVSVLTVLHQDWLNGKEYKCKVSNKALPAPIEKTISK AKGQPREPQVYTLPPSRDELTKNQVSLTCLVKGFYPSDIAVEW ESNGQPENNYKTTPPVLDSDGSFFLYSKLTVDKSRWQQGNVFS CSVMHEALHNHYTQKSLSLSPGK |
| | Light chain SEQ ID NO: 247 | DIVMTQSPSSMYASLGERVTITCKASQDINSFLSWFQQKPGKSP KTLLYRANRLMDGVPSRFSGSGSGQDFSLTISSREYEDMGIYY CLQYDEFPFTFGSGTKLELKRTVAAPSVFIFPPSDEQLKSGTAS VVCLLNNFYPREAKVQWKVDNALQSGNSQESVTEQDSKDST YSLSSTLTLSKADYEKHKVYACEVTHQGLSSPVTKSFNRGEC |
| M30-H1-L4 | Heavy chain SEQ ID NO: 269 | QVQLVQSGAEVKKPGSSVKVSCKASGYTFTNYVMHWVRQAP GQGLEWMGYINPYNDDVKYNEKFKGRVTITADESTSTAYMEL SSLRSEDTAVYYCARWGYYGSPLYYFDYWGQGTLVTVSSAST KGPSVFPLAPSSKSTSGGTAALGCLVKDYFPEPVTVSWNSGAL TSGVHTFPAVLQSSGLYSLSSVVTVPSSSLGTQTYICNVNHKPS NTKVDKRVEPKSCDKTHTCPPCPAPELLGGPSVFLFPPKPKDTL MISRTPEVTCVVVDVSHEDPEVKFNWYVDGVEVHNAKTKPRE EQYNSTYRVVSVLTVLHQDWLNGKEYKCKVSNKALPAPIEKT ISKAKGQPREPQVYTLPPSREEMTKNQVSLTCLVKGFYPSDIAV EWESNGQPENNYKTTPPVLDSDGSFFLYSKLTVDKSRWQQGN VFSCSVMHEALHNHYTQKSLSLSP |
| | Light chain SEQ ID NO: 270 | EIVLTQSPATLSLSPGERATLSCRASSRLIYMHWYQQKPGQAPR PLIYATSNLASGIPARFSGSGSGTDFTLTISSLEPEDFAVYYCQQ WNSNPPTFGQGTKVEIKRTVAAPSVFIFPPSDEQLKSGTASVVC LLNNFYPREAKVQWKVDNALQSGNSQESVTEQDSKDSTYSLS STLTLSKADYEKHKVYACEVTHQGLSSPVTKSFNRGEC |
| hmAb-C | Heavy chain SEQ ID NO: 271 | EVQLVESGGGLVKPGGSLRLSCAASGFTFSSYGMSWVRQAPG KGLEWVATINSGGSNTYYPDSLKGRFTISRDNAKNSLYLQMNS LRAEDTAVYYCARHDGGAMDYWGQGTTVTVSSASTKGPSVF PLAPSSKSTSGGTAALGCLVKDYFPEPVTVSWNSGALTSGVHT FPAVLQSSGLYSLSSVVTVPSSSLGTQTYICNVNHKPSNTKVDK RVEPKSCDKTHTCPPCPAPELLGGPSVFLFPPKPKDTLMISRTPE VTCVVVDVSHEDPEVKFNWYVDGVEVHNAKTKPREEQYNST YRVVSVLTVLHQDWLNGKEYKCKVSNKALPAPIEKTISKAKG QPREPQVYTLPPSREEMTKNQVSLTCLVKGFYPSDIAVEWESN |
| | | GQPENNYKTTPPVLDSDGSFFLYSKLTVDKSRWQQGNVFSCS VMHEALHNHYTQKSLSLSPGK |
| | Light chain SEQ ID NO: 272 | DIQMTQSPSSLSASVGDRVTITCRASESIYSYLAWYQQKPGKAP KLLVYNTKTLPEGVPSRFSGSGSGTDFTLTISSLQPEDFATYYC QHHYGTPPWTFGQGTRLEIKRTVAAPSVFIFPPSDEQLKSGTAS VVCLLNNFYPREAKVQWKVDNALQSGNSQESVTEQDSKDST YSLSSTLTLSKADYEKHKVYACEVTHQGLSSPVTKSFNRGEC |

### 2.2 Characterization of Chimeric Antibodies

### 2.2.1 Affinity Validation of Chimeric Antibodies:

Detection was performed using Fortebio Octet R2 as follows. A murine monoclonal antibody was immobilized using an AMQ sensor with PBST (PBS+0.02% tween20) as the buffer. Human B7-H3-His protein samples were diluted to 12.5 nM, 6.25 nM, 3.13 nM, 1.56 nM, and 0 nM. The protein concentrations of antibodies were: 100 nM, 50 nM, 25 nM, 12.5 nM, 6.25 nM, and 0 nM. The settings were: equilibration for 150 seconds, association for 180 seconds, dissociation for 300 seconds, and detection temperature at 25°C. The kinetic parameters (ka: association rate; kd: dissociation rate; kD: association-dissociation equilibrium constant) for the binding of each sample to human B7-H3-His were obtained. The results are shown in Table 4.

**Table 4. Affinity Results of Chimeric Antibodies to Human B7-H3His Protein**

| Antibody | Kon(M⁻¹S⁻¹) | Koff(S⁻¹) | KD(M) |
|---|---|---|---|
| 19-F1-E9-B11 | 1.760x10⁵ | 4.077x10⁻⁴ | 2.316x10⁻⁹ |
| 28-F8-C4-A8 | 1.744x10⁶ | 7.562x10⁻⁵ | 4.336x10⁻¹¹ |
| 29-A9-G8-F6 | 1.489x10⁶ | 1.542x10⁻⁵ | 1.035x10⁻¹¹ |
| 28-B1-E8-C11 | 1.189x10⁶ | 8.491x10⁻⁵ | 7.139x10⁻¹¹ |
| 16-B6-G3-E10 | 1.305x10⁶ | < 1.0x10⁻⁷ | < 1.0x10⁻¹² |
| 34-H11-D9-F6 | 1.442x10⁶ | 7.524x10⁻⁵ | 5.217x10⁻¹¹ |
| 16-D10-E1-E1 | 1.616x10⁶ | 1.417x10⁻⁴ | 8.769x10⁻¹¹ |
| 48-B4-F4-E4 | 1.284x10⁶ | 2.692x10⁻⁴ | 2.096x10⁻¹⁰ |

### 2.2.2 FACS Binding and Species-Specificity Verification of Chimeric Antibodies

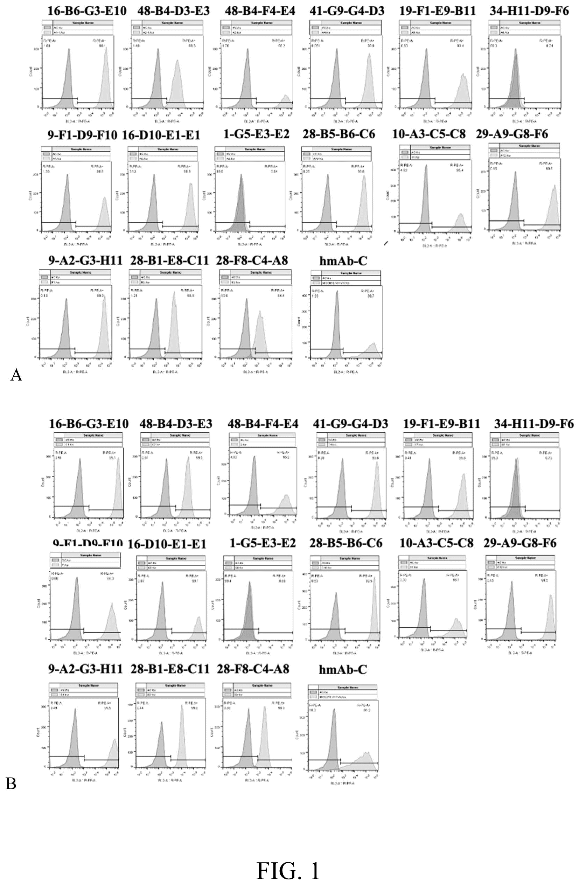

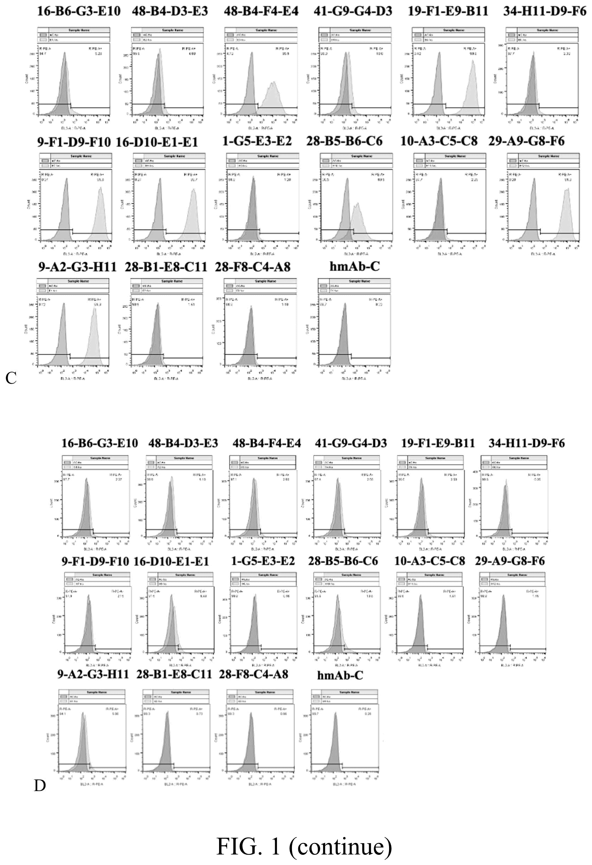

The CHO-S cell line, the CHO-S-human B7-H3 cell line recombinantly expressing human B7-H3, the CHO-Cyno B7-H3 cell line recombinantly expressing cynomolgus monkey B7-H3, and the CHO-mouse B7-H3 cell line recombinantly expressing mouse B7-H3 were each aliquoted into several portions with 3*10^5 cells per portion. 100 µL of chimeric antibody expression supernatant was incubated with each of the target cells. The B7-H3 antibody concentration for detection using human B7-H3 overexpressing cells, CHO-S-human B7-H3, and monkey B7-H3 overexpressing cells, CHO-Cyno B7-H3, was 1 µg/ml, and the B7-H3 antibody concentration for detection using mouse B7-H3 overexpressing cells, CHO-mouse B7-H3, and blank CHO cells, CHO-S, was 10 µg/ml. After thorough mixing, cells were incubated at room temperature for 1 hour. The cells were washed 3 times with PBS, and then added with PE-labeled Anti-human IgG (Invitrogen, CAT# 12-4998-82). After thorough mixing, the cells were incubated in the dark at room temperature for 30 minutes. The cells were washed 3 times with PBS and determined by flow cytometer. The results are shown in FlG. 1.

The results in FIGs. 1A to 1D show that the sequences 16-B6-G3-E10, 48-B4-D3-E3, 48-B4-F4-E4, 41-G9-G4-D3, 19-F1-E9-B11, 9-F1-D9-F10, 16-D10-E1-E1, 28-B5-B6-C6, 10-A3-C5-C8, 29-A9-G8-F6, 9-A2-G3-H11, 28-B1-E8-C11, 28-F8-C4-A8 are all capable of binding to CHO-S-human B7-H3 cells. Among them, 48-B4-F4-E4, 19-F1-E9-B11, 9-F1-D9-F10, 16-D10-E1-E1, 28-B5-B6-C6, 29-A9-G8-F6, and 9-A2-G3-H11 are capable of cross-binding human, murine, and monkey B7-H3.

### 2.2.3 Verification of Endocytosis Efficiency of Chimeric Antibodies

The internalization efficiency of chimeric antibodies 16-B6-G3-E10 (CE10), 19-F1-E9-B11 (CB11), 48-B4-F4-E4 (CE4), 28-B1-E8-C11 (CC11), and positive control antibody M30-H1-L4 into Calu-6 (a lung cancer cell) and NCI-H446 (a small cell lung cancer cell) was verified by killing assay of toxin DT3C-conjugated chimeric antibodies. Calu-6 cells or NCI-H446 cells in logarithmic growth phase were resuspended in culture medium to an appropriate concentration, and seeded at 500 cells/40 µl/well. After overnight incubation, the chimeric antibodies were incubated with DT3C at a ratio of 1: 2 for 30 min at room temperature to form antibody-DT3C conjugates. These were then added to a 384-well white plate cell culture plate and incubated at 37°C for 3 days. Subsequently, 20 µl/well of CellTiter-Glo Reagen reagent from CellTiter Glo assay kit (Promega, G7573) was added for detection. The percentage of inhibition was calculated using the following formula:
%Inhibition=100-(Signalcmpd-SignalAve_PC)/(SignalAve_VC-SignalAve_PC)x100 wherein, % Inhibition represents the inhibition rate, Signalcmpd represents the average luminescence value of the test sample, SignalAve_PC represents the average luminescence value of the blank positive control, and SignalAve_VC represents the average luminescence value of the negative control (0.1% DMSO).

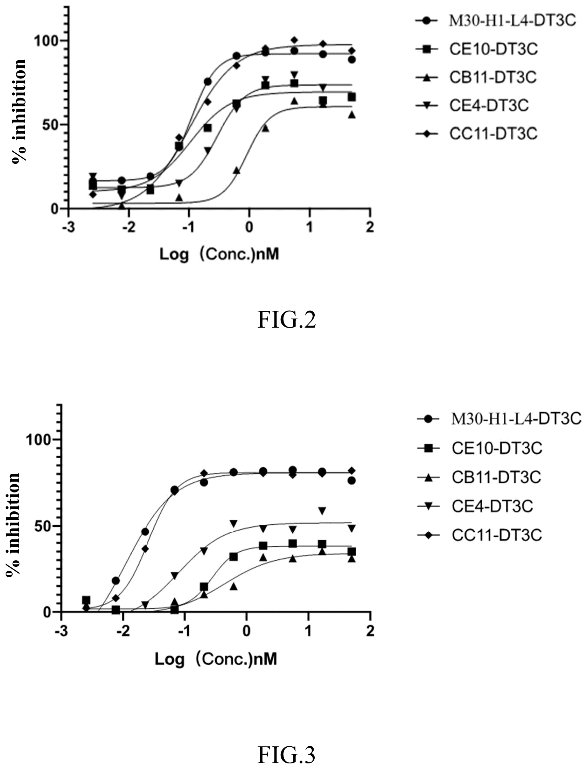

The results are shown in FIG. 2 and FIG. 3. The results show that 16-B6-G3-E10 (CE10), 19-F1-E9-B11 (CB11), and 48-B4-F4-E4 (CE4) exhibit better internalization efficiencies in Calu-6 and NCI-H446 as compared with the positive control M30-H1-L4, and 28-B1-E8-C11 (CC11) has a comparable internalization rate to the positive control M30-H1-L4 in Calu-6 and NCI-H446.

### Example 3. Humanized Antibodies

### 3.1 Designs of Humanization, Removal of Post-Translational Modification Sites, and Construction of Plasmids

According to the performance of the chimeric antibodies in various aspects, 48-B4-F4-E4 and 28-B1-E8-C11 were selected for humanized antibody engineering. According to the amino acid sequence information of the heavy and light chains of the selected murine antibodies, a homology model of the antibody was obtained by modeling, and the framework amino acids within the CDR range, which typically affect the conformation or antigen-binding activity of the CDR, were analyzed. Human germline was obtained by IMGT analysis. After splicing the selected human germline framework with the CDRs of an antibody, the framework sequences of the designed humanized antibody and the original antibody were aligned. Based on the analysis of the homology modeling results of the parent antibody, amino acids similar to the surface residues of the human antibody were selected for substitution, while maintaining the activity of the antibody and minimizing heterogeneity. Hotspots of post-translational modifications (PTMs) in the variable regions were altered to reduce PTM risk. After verifying the template sequences and codon optimization, the heavy chain variable region sequences were synthesized and constructed into a pCDNA3.4-uIgG1 plasmid containing human heavy chain CH1, hinge, CH2, and CH3 fragments. The pCDNA3.4-uIgG1 plasmid was obtained by ligating the coding nucleic acid for the heavy chain constant region fragment (GenBank: QBK47475.1 Seq 145-474) into the backbone plasmid pCDNA3.4. The sequence of the light chain variable region was synthesized and constructed into the plasmid pCDNA3.4-IgKc containing the human light chain constant region fragment. The constructed plasmids had the same constant regions, but contained different site mutations in the heavy chain variable region and the light chain variable region.

**Table 5. Amino Acid Sequences of the Heavy and Light Chain Variable Regions of Humanized Antibodies**

| ID of humanized VH or VL | | SE Q ID NO | Amino acid sequence |
|---|---|---|---|
| cAb1-z0-p2-uIgG1 | VH | 26 | EVQLVESGGGLVKPGGSLRLSCAASGFTFSSYGMSWVRQAPGKGL EWVSSISGSGSYTYYPDSLKGRFTISRDNAKNSLYLQMNSLRAEDT AVYYCARQ GYWYFDVWGQGTTVTVSS |
| cAb1-z0-p5-uK | VL | 27 | DIVMTQTPLSLPVTPGEPASISCRASQSLASSYGKTYLSWYLQKPGQ SPQLLIYGISNRFSGVPDRFSGSGSGTDFTLKISRVEAEDVGVYYCLH GTHQPLTFGQGTKLEIK |
| cAb1-z1-p2-uIgG1 | VH | 28 | EVQLVESGGGLVKPGGSLRLSCAASGFTFSSYGMSWVRQAPGK LE WVSSISGSGSYTYYPDSLKGRFTISRDNAKNSLYLQMNSLRAEDTA VYYCARQ GYWYFDVWGQGTTVTVSS |
| cAb1-z1-p5-uK | VL | 29 | D VMTQTPLSLPVTPGEPASISCRASQSLA SYGKTYLSWYLQKPGQ SPQLLIYGISNRFSGVPDRFSGSGSGTDFTLKISRVEAEDVGVYYCLH GTHQPLTFGQGTKLEIK |
| cAb1-z3-p2-uIgG1 | VH | 30 | EVQLVESGGGLVKPGGSLRLSCAASGFTFSSYGMSWVRQAPGK LE WV SISGSGSYTYYPDSLKGRFTISRDNAKNSLYLQMNSLRAEDTA VYYCARQ GYWYFDVWGQGTTVTVSS |
| cAb1-uIgG1 | VH | 31 | EVQLQESGGDLVKPGGSLKLSCAASGFTFSSYGMSWVRQTPEKRLE WVASISGSGSYTYYPDSLKGRFTISRDNAKNNLYLQMSSLRSEDTA LYYCARQDGYWYFDVWGAGTTVTVSS |
| cAb1-uK | VL | 32 | DVLMTQTPLSLPVSLGDQVSISCRASQSLANSYGKTYLSWYLHKPG QSPQLLIYGISNRFSGVPDRFSGSGSGTDFTLKISTIKPEDLGMYYCL HGTHQPLTFGAGTKLELK |
| cAb2-z1-p2-uIgG1 | VH | 33 | EVKLVESGAVLVKPGASVKISCKAS MDWVKQSQGKSLE WVGD TYNQKFRGKATLTVDRSSNTAYMELRSLTSEDSAV YYC WGAGTTVTVSS |
| cAb2-z2-p2-uIgG1 | VH | 34 | QVQLVESGAvVKKPGASVKVSCKAS MDWVRQAPGKSLE WMGD TYNQKFRGRVTLTVDRSINTAYMELSRLRSDDTAV YYC WGAGTMVTVSS |
| cAb2-z3-p2-uIgG1 | VH | 35 | EVQLVESGAvVKKPGASVKVSCKAS MDWVRQAPGKSLE WMGD TYNQKFRGRVTLTVDRSANTAYMELSSLRSEDTA VYYC WGAGTMVTVSS |
| cAb2-z4-p2-uIgG1 | VH | 36 | EVKLVESGAEVKKPGASVKVSCKAS MDWVRQAPGKSLE WMGD TYNQKFRGRVTLTVDRSTNTAYMELSSLRSEDTAV YYC WGAGTMVTVSS |
| cAb2-z5-p2-uIgG1 | VH | 37 | QVQLVESGAEVKKPGASVKVSCKAS MDWVRQAQGKSLE WMGD TYNQKFRGRVTLTVDRSTNTAYMELSSLRSEDTAV YYC WGAGTMVTVSS |
| cAb2-z1-p5-uK | VL | 38 | DIQLTQPPSSLSASVGDRVTITCRAS LAWYQQKPGRSPQLL VY TLQEGVPSRFSGSGSGTQFTLTISSLQPEDFADYYC W TFGGGTKVEIK |
| cAb2-z2-p5-uK | VL | 39 | DIQLTQPPSSLSASVGDRVTITCRAS LAWYQQKPGRSPQLLV Y TLQEGVPSRFSGSGSGTQFTLTISSLQPEDFADYYC WT FGGGTKVEIK |
| cAb2-z3-p5-uK | VL | 40 | DIQLTQPPSFLSASVGDRVTITCRAS LAWYQQKPGRSPQLL VY TLQEGVPSRFSGSGSGTEFTLTISSLQPEDFADYYC W TFGGGTKVEIK |
| cAb2-z4-p5-uK | VL | 41 | DIQLTQPPSSLSASVGDRVTITCRAS LAWYQQKPGRSPQLL VY TLQEGVPSRFSGSGSGTQFTLTISSLQPEDVADYYC W TFGGGTKVEIK |
| cAb2-z5-p5-uK | VL | 42 | DIQLTQPPSSLSASVGDRVTITCRAS LAWYQQKPGRSPQLLV Y TLPEGVPSRFSGSGSGTQFTLTISSLQPEDFADYYC WTF GGGTKVEIK |

### 3.2 Production of Humanized Antibodies

The pCDNA3.4 plasmid containing the antibody heavy chain variable region (VH) and light chain variable region (VL) genes was co-transfected into Expi293F cells. After the cells were cultured for 6 days, the supernatant was collected. The supernatant was automatically loaded onto a protein A (MabSelect SuRe, GE-17543804) affinity chromatography column using an AKTA sample injection system. After the sample loading, the column was washed with 0.1 M Tris-HCl, pH 7.0 equilibration buffer, followed by elution and collection of target proteins with 0.1 M glycine, pH 3.5 elution buffer. The sequences of the obtained humanized antibodies are shown in Table 6, Table 7, and Table 8. cAb1-z0-p7, cAbl-z3-p7, and cAbl-z5-p7 are the humanized sequences of 48-B4-F4-E4 (also known as cAb1-ulgG1K). cAb2-z0-p9, cAb2-zl-p9, cAb2-z2-p6, cAb2-z3-p6, cAb2-z4-p6, and cAb2-z5-p6 are the humanized sequences of 28-B1-E8-C11.

**Table 6. Amino Acid Sequences of the Heavy and Light Chain Variable Regions of Humanized Antibodies**

| ID of Humanized Antibody | | SE Q ID NO: | Amino acid sequence |
|---|---|---|---|
| cAb1-z0-p7 | VH | 26 | EVQLVESGGGLVKPGGSLRLSCAASGFTFSSYGMSWVRQAPGK LE WV SISGSGSYTYYPDSLKGRFTISRDNAKNSLYLQMNSLRAEDTAV YYCARQQGYWYFDVWGQGTTVTVSS |
| | VL | 27 | D VMTQTPLSLPVTPGEPASISCRASQSLASSYGKTYLSWYLQKPGQS PQLLIYGISNRFSGVPDRFSGSGSGTDFTLKISRVEAEDVGVYYCLHG THQPLTFGQGTKLEIK |
| cAb1-z3-p7 | VH | 28 | EVQLVESGGGLVKPGGSLRLSCAASGFTFSSYGMSWVRQAPGK LE WV SISGSGSYTYYPDSLKGRFTISRDNAKNSLYLQMNSLRAEDTAV YYCARQQGYWYFDVWGQGTTVTVSS |
| | VL | 29 | D VMTQTPLSLPVTPGEPASISCRASQSLASSYGKTYLSWYLQKPGQS PQLLIYGISNRFSGVPDRFSGSGSGTDFTLKISRVEAEDVGVYYCLHG THQPLTFGQGTKLEIK |
| cAb1-z5-p7 | VH | 30 | EVQLVESGGGLVKPGGSLRLSCAASGFTFSSYGMSWVRQAPGK LE WV SISGSGSYTYYPDSLKGRFTISRDNAKNSLYLQMNSLRAEDTAV YYCARQQGYWYFDVWGQGTTVTVSS |
| | VL | 29 | D VMTQTPLSLPVTPGEPASISCRASQSLASSYGKTYLSWYLQKPGQS PQLLIYGISNRFSGVPDRFSGSGSGTDFTLKISRVEAEDVGVYYCLHG THQPLTFGQGTKLEIK |
| cAb1-uIgG1 K | VH | 31 | EVQLQESGGDLVKPGGSLKLSCAASGFTFSSYGMSWVRQTPEKRLE WVASISGSGSYTYYPDSLKGRFTISRDNAKNNLYLQMSSLRSEDTAL YYCARQDGYWYFDVWGAGTTVTVSS |
| | VL | 32 | DVLMTQTPLSLPVSLGDQVSISCRASQSLANSYGKTYLSWYLHKPG QSPQLLIYGISNRFSGVPDRFSGSGSGTDFTLKISTIKPEDLGMYYCLH GTHQPLTFGAGTKLELK |
| cAb2-z0-p9 | VH | 33 | EVKLVESGAVLVKPGASVKISCKAS MDWVKQSQGKSLEW VGD TYNQKFRGKATLTVDRSSNTAYMELRSLTSEDSAVYY C WGAGTTVTVSS |
| | VL | 41 | DIQLTQPPSSLSASVGDRVTITCRAS LAWYQQKPGRSPQLLVY TLQEGVPSRFSGSGSGTQFTLTISSLQPEDVADYYC WTFGG GTKVEIK |
| cAb2-z1-p9 | VH | 33 | EVKLVESGAVLVKPGASVKISCKAS MDWVKQSQGKSLEW VGD TYNQKFRGKATLTVDRSSNTAYMELRSLTSEDSAVYY C WGAGTTVTVSS |
| | VL | 42 | DIQLTQPPSSLSASVGDRVTITCRAS LAWYQQKPGRSPQLLVY TLPEGVPSRFSGSGSGTQFTLTISSLQPEDFADYYC WTFGG GTKVEIK |
| cAb2-z2-p6 | VH | 34 | QVQLVESGAvVKKPGASVKVSCKAS MDWVRQAPGKSLEW MGD TYNQKFRGRVTLTVDRSINTAYMELSRLRSDDTAVYY C WGAGTMVTVSS |
| | VL | 39 | DIQLTQPPSSLSASVGDRVTITCRAS LAWYQQKPGRSPQLLVY TLQEGVPSRFSGSGSGTQFTLTISSLQPEDFADYYC WTFGG GTKVEIK |
| cAb2-z3-p6 | VH | 34 | QVQLVESGAvVKKPGASVKVSCKAS MDWVRQAPGKSLEW MGD TYNQKFRGRVTLTVDRSINTAYMELSRLRSDDTAVYY C WGAGTMVTVSS |
| | VL | 40 | DIQLTQPPSFLSASVGDRVTITCRAS LAWYQQKPGRSPQLLVY TLQEGVPSRFSGSGSGTEFTLTISSLQPEDFADYYC WTFGG GTKVEIK |
| cAb2-z4-p6 | VH | 34 | QVQLVESGAvVKKPGASVKVSCKAS MDWVRQAPGKSLEW MGD TYNQKFRGRVTLTVDRSINTAYMELSRLRSDDTAVYY C WGAGTMVTVSS |
| | VL | 41 | DIQLTQPPSSLSASVGDRVTITCRAS LAWYQQKPGRSPQLLVY TLQEGVPSRFSGSGSGTQFTLTISSLQPEDVADYYC WTFG GGTKVEIK |
| cAb2-z5-p6 | VH | 34 | QVQLVESGAvVKKPGASVKVSCKAS MDWVRQAPGKSLEW MGD TYNQKFRGRVTLTVDRSINTAYMELSRLRSDDTAVYY C WGAGTMVTVSS |
| | VL | 42 | DIQLTQPPSSLSASVGDRVTITCRAS LAWYQQKPGRSPQLLVY TLPEGVPSRFSGSGSGTQFTLTISSLQPEDFADYYC WTFGG GTKVEIK |

**Table 7. Amino Acid Sequences of CDRs and FRs of Humanized Antibodies**

| Table 7-1 Amino Acid Sequences of CDRs and FRs of the Heavy Chain of Humanized Antibodies | | | | | | | |
|---|---|---|---|---|---|---|---|
| Antibody ID | FR1 | CDR1 | FR2 | CDR2 | FR3 | CDR3 | FR4 |
| cAb1-z0-p7 | EVQLVESGGGLV KPGGSLRLSCAAS (SEQ ID NO: 123) | GFTFSS YGMS (SEQ ID NO: 84) | WVRQAPGKL EWV (SEQ ID NO: 124) | SISGSGSYTYY PDSLKG (SEQ ID NO: 8 6) | RFTISRDNA KNSLYLQM NSLRAEDTA VYYCAR (SEQ ID N O: 125) | QQGYWYF DV (SEQ ID N O: 126) | WGQGTTVTVSS (SEQ ID NO: 127) |
| cAb1-z3-p7 | EVQLVESGGGLV KPGGSLRLSCAAS (SEQ ID NO: 123) | GFTFSS YGMS (SEQ ID NO: 84) | WVRQAPGKL EWV (SEQ ID NO: 128) | SISGSGSYTYY PDSLKG (SEQ ID NO: 8 6) | RFTISRDNA KNSLYLQM NSLRAEDTA VYYCAR (SEQ ID N O: 125) | QQGYWYF DV (SEQ ID N O: 126) | WGQGTTVTVSS (SEQ ID NO: 127) |
| cAb1-z5-p7 | EVQLVESGGGLV KPGGSLRLSCAAS (SEQ ID NO: 123) | GFTFSS YGMS (SEQ ID NO: 84) | WVRQAPGKL EWV (SEQ ID NO: 129) | SISGSGSYTYY PDSLKG (SEQ ID NO: 8 6) | RFTISRDNA KNSLYLQM NSLRAEDTA VYYCAR (SEQ ID N O: 125) | QQGYWYF DV (SEQ ID N O: 126) | WGQGTTVTVSS (SEQ ID NO: 127) |
| cAb1-uIgG1K | EVQLQESGGDLV KPGGSLKLSCAAS (SEQ ID NO: 50) | GFTFSS YGMS (SEQ ID | WVRQTPEKR LEWVA (SEQ ID NO: | SISGSGSYTYY PDSLKG (SEQ ID NO: 8 | RFTISRDNA KNNLYLQM SSLRSEDTA | QDGYWYF DV (SEQ ID N | WGAGTTVTVSS (SEQ ID NO: 56) |
| | | NO: 84) | 85) | 6) | LYYCAR (SEQ ID N O: 87) | O: 88) | |
| cAb2-z0-p9 | EVKLVESGAVLV KPGASVKISCKAS (SEQ ID NO: 110) | (SEQ ID NO: 111) | MDWVKQSQ GKSLEWVGD (SEQ ID NO: 112) | (SEQ ID NO: 1 13) | TYNQKFRG KATLTVDRS SNTAYMEL RSLTSEDSA VYYC (SEQ ID N O: 114) | (SEQ ID NO: 115) | WGAGTTVTVSS (SEQ ID NO: 56) |
| cAb2-z1-p9 | EVKLVESGAVLV KPGASVKISCKAS (SEQ ID NO: 110) | (SEQ ID NO: 111) | MDWVKQSQ GKSLEWVGD (SEQ ID NO: 112) | (SEQ ID NO: 113) | TYNQKFRG KATLTVDRS SNTAYMEL RSLTSEDSA VYYC (SEQ ID N O: 114) | (SEQ ID NO: 115) | WGAGTTVTVSS (SEQ ID NO: 56) |
| cAb2-z2-p6 | QVQLVESGAvVK KPGASVKVSCKA S (SEQ ID NO: 130) | (SEQ ID NO: 111) | MDWVRQAP GKSLEWMGD (SEQ ID NO: 131) | (SEQ ID NO: 113) | TYNQKFRG RVTLTVDRS INTAYMELS RLRSDDTA VYYC (SEQ ID N O: 132) | (SEQ ID NO: 115) | WGAGTMVTVSS (SEQ ID NO: 133) |
| cAb2-z3-p6 | QVQLVESGAvVK | | MDWVRQAP | | TYNQKFRG | | WGAGTMVTVSS |
| | KPGASVKVSCKA S (SEQ ID NO: 130) | (SEQ ID NO: 111) | GKSLEWMGD (SEQ ID NO: 131) | (SEQ ID NO: 113) | RVTLTVDRS INTAYMELS RLRSDDTA VYYC (SEQ ID N O: 132) | (SEQ ID NO: 115) | (SEQ ID NO: 133) |
| cAb2-z4-p6 | QVQLVESGAvVK KPGASVKVSCKA S (SEQ ID NO: 130) | (SEQ ID NO: 111) | MDWVRQAP GKSLEWMGD (SEQ ID NO: 131) | (SEQ ID NO: 113) | TYNQKFRG RVTLTVDRS INTAYMELS RLRSDDTA VYYC (SEQ ID N O: 132) | (SEQ ID NO: 115) | WGAGTMVTVSS (SEQ ID NO: 133) |
| cAb2-z5-p6 | QVQLVESGAvVK KPGASVKVSCKA S (SEQ ID NO: 130) | (SEQ ID NO: 111) | MDWVRQAP GKSLEWMGD (SEQ ID NO: 131) | (SEQ ID NO: 113) | TYNQKFRG RVTLTVDRS INTAYMELS RLRSDDTA VYYC (SEQ ID N O: 132) | (SEQ ID NO: 115) | WGAGTMVTVSS (SEQ ID NO: 133) |

**Table 7-2 Amino Acid Sequences of CDRs and FRs of the Light Chain of Humanized Antibodies**

| Antibody light chain | FR1 | CDR1 | FR2 | CDR2 | FR3 | CDR3 | FR4 |
|---|---|---|---|---|---|---|---|
| cAb1-z0-p7 | D VMTQTPL | RASQSLASSY | SWYLQKP | GISNRFS | GVPDRFSGSGSG | LHGTHQPL | FGQGTKLEIK |
| | SLPVTPGEP ASISC (SEQ ID NO: 212) | GKTYL (SEQ ID NO: 213) | GQSPQLLI Y (SEQ ID N O: 214) | (SEQ ID N O: 175) | TDFTLKISRVEAE DVGVYYC (SEQ ID NO: 215) | T (SEQ ID N O: 177) | (SEQ ID NO: 216) |
| cAb1-z3-p7 | D VMTQTPL SLPVTPGEP ASISC (SEQ ID NO: 217) | RASQSLASSY GKTYL (SEQ ID NO: 213) | SWYLQKP GQSPQLLI Y (SEQ ID N O: 214) | GISNRFS (SEQ ID N O: 175) | GVPDRFSGSGSG TDFTLKISRVEAE DVGVYYC (SEQ ID NO: 215) | LHGTHQPL T (SEQ ID N O: 177) | FGQGTKLEIK (SEQ ID NO: 216) |
| cAb1-z5-p7 | D VMTQTPL SLPVTPGEP ASISC (SEQ ID NO: 217) | RASQSLASSY GKTYL (SEQ ID NO: 213) | SWYLQKP GQSPQLLI Y (SEQ ID N O: 214) | GISNRFS (SEQ ID N O: 175) | GVPDRFSGSGSG TDFTLKISRVEAE DVGVYYC (SEQ ID NO: 215) | LHGTHQPL T (SEQ ID N O: 177) | FGQGTKLEIK (SEQ ID NO: 216) |
| cAb1-uIgG1K | DVLMTQTPL SLPVSLGDQ VSISC (SEQ ID NO: 172) | RASQSLANS YGKTYL (SEQ ID NO: 173) | SWYLHKP GQSPQLLI Y (SEQ ID N O: 174) | GISNRFS (SEQ ID N O: 175) | GVPDRFSGSGSG TDFTLKISTIKPED LGMYYC (SEQ ID NO: 176) | LHGTHQPL T (SEQ ID N O: 177) | FGAGTKLELK (SEQ ID NO: 146) |
| cAb2-z0-p9 | DIQLTQPPSS LSASVGDRV | (SEQ ID NO: | LAWYQQK PGRSPQLL | | TLQEGVPSRFSGS GSGTQFTLTISSL | (SEQ ID N | WTFGGGTKVEIK (SEQ ID NO: 190) |
| | TITCRAS (SEQ ID NO: 162) | 201) | VY (SEQ ID N O: 168) | | QPEDVADYYC (SEQ ID NO: 181) | O: 205) | |
| cAb2-z1-p9 | DIQLTQPPSS LSASVGDRV TITCRAS (SEQ ID NO: 162) | (SEQ ID NO: 201) | LAWYQQK PGRSPQLL VY (SEQ ID N O: 168) | | TLPEGVPSRFSGS GSGTQFTLTISSL QPEDFADYYC (SEQ ID NO: 197) | (SEQ ID N O: 203) | WTFGGGTKVEIK (SEQ ID NO: 190) |
| cAb2-z2-p6 | DIQLTQPPSS LSASVGDRV TITCRAS (SEQ ID NO: 162) | (SEQ ID NO: 201) | LAWYQQK PGRSPQLL VY (SEQ ID N O: 168) | | TLQEGVPSRFSGS GSGTQFTLTISSL QPEDFADYYC (SEQ ID NO: 137) | (SEQ ID N O: 205) | WTFGGGTKVEIK (SEQ ID NO: 190) |
| cAb2-z3-p6 | DIQLTQPPSF LSASVGDRV TITCRAS (SEQ ID NO: 150) | (SEQ ID NO: 201) | LAWYQQK PGRSPQLL VY (SEQ ID N O: 168) | | TLQEGVPSRFSGS GSGTEFTLTISSL QPEDFADYYC (SEQ ID NO: 156) | (SEQ ID N O: 205) | WTFGGGTKVEIK (SEQ ID NO: 190) |
| cAb2-z4-p6 | DIQLTQPPSS LSASVGDRV TITCRAS (SEQ ID NO: | (SEQ ID NO: 201) | LAWYQQK PGRSPQLL VY (SEQ ID N | | TLQEGVPSRFSGS GSGTQFTLTISSL QPEDVADYYC (SEQ ID NO: 181) | (SEQ ID N O: 205) | WTFGGGTKVEIK (SEQ ID NO: 190) |
| | 162) | | O: 168) | | | | |
| cAb2-z5-p6 | DIQLTQPPSS LSASVGDRV TITCRAS (SEQ ID NO: 162) | (SEQ ID NO: 201) | LAWYQQK PGRSPQLL VY (SEQ ID N O: 168) | | TLPEGVPSRFSGS GSGTQFTLTISSL QPEDFADYYC (SEQ ID NO: 197) | (SEQ ID N O: 203) | WTFGGGTKVEIK (SEQ ID NO: 190) |

**Table 8. Amino Acid Sequences of the Heavy and Light Chains of Humanized Antibodies**

| ID of V H or V L | SEQ ID NO: | | Full-length sequence |
|---|---|---|---|
| cA b1 - z0 - p2 uI g G 1 | H ea vy ch ai n | 2 4 8 | EVQLVESGGGLVKPGGSLRLSCAASGFTFSSYGMSWVRQAPGKGLEWVSSISGSGSYTYYPDSLKGRFTISRDNAKNSL YLQMNSLRAEDTAVYYCARQQGYWYFDVWGQGTTVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSNTKVDKRVEPKSCDKTHTCPPCPAPEL LGGPSVFLFPPKPKDTLMISRTPEVTCVVVDVSHEDPEVKFNWYVDGVEVHNAKTKPREEQYNSTYRVVSVLTVLHQ DWLNGKEYKCKVSNKALPAPIEKTISKAKGQPREPQVYTLPPSREEMTKNQVSLTCLVKGFYPSDIAVEWESNGQPEN NYKTTPPVLDSDGSFFLYSKLTVDKSRWQQGNVFSCSVMHEALHNHYTQKSLSLSPGK |
| cA b1 - z0 - p5 u K | Li gh t ch ai n | 2 4 9 | DIVMTQTPLSLPVTPGEPASISCRASQSLASSYGKTYLSWYLQKPGQSPQLLIYGISNRFSGVPDRFSGSGSGTDFTLKISR VEAEDVGVYYCLHGTHQPLTFGQGTKLEIKRTVAAPSVFIFPPSDEQLKSGTASVVCLLNNFYPREAKVQWKVDNALQ SGNSQESVTEQDSKDSTYSLSSTLTLSKADYEKHKVYACEVTHQGLSSPVTKSFNRGEC |
| cA b1 - z1 - p2 uI g G 1 | H ea vy ch ai n | 2 5 0 | EVQLVESGGGLVKPGGSLRLSCAASGFTFSSYGMSWVRQAPGKRLEWVSSISGSGSYTYYPDSLKGRFTISRDNAKNSL YLQMNSLRAEDTAVYYCARQQGYWYFDVWGQGTTVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSNTKVDKRVEPKSCDKTHTCPPCPAPEL LGGPSVFLFPPKPKDTLMISRTPEVTCVVVDVSHEDPEVKFNWYVDGVEVHNAKTKPREEQYNSTYRVVSVLTVLHQ DWLNGKEYKCKVSNKALPAPIEKTISKAKGQPREPQVYTLPPSREEMTKNQVSLTCLVKGFYPSDIAVEWESNGQPEN NYKTTPPVLDSDGSFFLYSKLTVDKSRWQQGNVFSCSVMHEALHNHYTQKSLSLSPGK |
| cA b1 - z1 - p5 u K | Li gh t ch ai n | 2 5 1 | DVVMTQTPLSLPVTPGEPASISCRASQSLASSYGKTYLSWYLQKPGQSPQLLIYGISNRFSGVPDRFSGSGSGTDFTLKIS RVEAEDVGVYYCLHGTHQPLTFGQGTKLEIKRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSPVTKSFNRGEC |
| cA b1 - z3 - p2 uI g G 1 | H ea vy ch ai n | 2 5 2 | EVQLVESGGGLVKPGGSLRLSCAASGFTFSSYGMSWVRQAPGKRLEWVASISGSGSYTYYPDSLKGRFTISRDNAKNS LYLQMNSLRAEDTAVYYCARQQGYWYFDVWGQGTTVTVSSASTKGPSVFPLAPSSKSTSGGTAALGCLVKDYFPEPV TVSWNSGALTSGVHTFPAVLQSSGLYSLSSVVTVPSSSLGTQTYICNVNHKPSNTKVDKRVEPKSCDKTHTCPPCPAPE LLGGPSVFLFPPKPKDTLMISRTPEVTCVVVDVSHEDPEVKFNWYVDGVEVHNAKTKPREEQYNSTYRVVSVLTVLHQ DWLNGKEYKCKVSNKALPAPIEKTISKAKGQPREPQVYTLPPSREEMTKNQVSLTCLVKGFYPSDIAVEWESNGQPEN NYKTTPPVLDSDGSFFLYSKLTVDKSRWQQGNVFSCSVMHEALHNHYTQKSLSLSPGK |
| cA b1 - uI g G 1 | H ea vy ch ai n | 2 5 3 | EVQLQESGGDLVKPGGSLKLSCAASGFTFSSYGMSWVRQTPEKRLEWVASISGSGSYTYYPDSLKGRFTISRDNAKNN LYLQMSSLRSEDTALYYCARQDGYWYFDVWGAGTTVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSNTKVDKRVEPKSCDKTHTCPPCPAPEL LGGPSVFLFPPKPKDTLMISRTPEVTCVVVDVSHEDPEVKFNWYVDGVEVHNAKTKPREEQYNSTYRVVSVLTVLHQ DWLNGKEYKCKVSNKALPAPIEKTISKAKGQPREPQVYTLPPSREEMTKNQVSLTCLVKGFYPSDIAVEWESNGQPEN |
| | | | NYKTTPPVLDSDGSFFLYSKLTVDKSRWQQGNVFSCSVMHEALHNHYTQKSLSLSPGK |
| cA b1 - u K | Li gh t ch ai n | 2 5 4 | DVLMTQTPLSLPVSLGDQVSISCRASQSLANSYGKTYLSWYLHKPGQSPQLLIYGISNRFSGVPDRFSGSGSGTDFTLKIS TIKPEDLGMYYCLHGTHQPLTFGAGTKLELKRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSPVTKSFNRGEC |
| cA b2 - z1 - p2 uI g G 1 | H ea vy ch ai n | 2 55 | EVKLVESGAVLVKPGASVKISCKASGYTLSDFNMDWVKQSQGKSLEWVGDINPNYDTITYNQKFRGKATLTVDRSSN TAYMELRSLTSEDSAVYYCARGELGRYFYVWGAGTTVTVSSASTKGPSVFPLAPSSKSTSGGTAALGCLVKDYFPEPV TVSWNSGALTSGVHTFPAVLQSSGLYSLSSVVTVPSSSLGTQTYICNVNHKPSNTKVDKRVEPKSCDKTHTCPPCPAPE LLGGPSVFLFPPKPKDTLMISRTPEVTCVVVDVSHEDPEVKFNWYVDGVEVHNAKTKPREEQYNSTYRVVSVLTVLHQ DWLNGKEYKCKVSNKALPAPIEKTISKAKGQPREPQVYTLPPSREEMTKNQVSLTCLVKGFYPSDIAVEWESNGQPEN NYKTTPPVLDSDGSFFLYSKLTVDKSRWQQGNVFSCSVMHEALHNHYTQKSLSLSPGK |
| cA b2 - z2 - p2 uI g G 1 | H ea vy ch ai n | 2 5 6 | QVQLVESGAvVKKPGASVKVSCKASGYTLSDFNMDWVRQAPGKSLEWMGDINPNYDTITYNQKFRGRVTLTVDRSIN TAYMELSRLRSDDTAVYYCARGELGRYFYVWGAGTMVTVSSASTKGPSVFPLAPSSKSTSGGTAALGCLVKDYFPEP VTVSWNSGALTSGVHTFPAVLQSSGLYSLSSVVTVPSSSLGTQTYICNVNHKPSNTKVDKRVEPKSCDKTHTCPPCPAP ELLGGPSVFLFPPKPKDTLMISRTPEVTCVVVDVSHEDPEVKFNWYVDGVEVHNAKTKPREEQYNSTYRVVSVLTVLH |
| | | | QDWLNGKEYKCKVSNKALPAPIEKTISKAKGQPREPQVYTLPPSREEMTKNQVSLTCLVKGFYPSDIAVEWESNGQPE NNYKTTPPVLDSDGSFFLYSKLTVDKSRWQQGNVFSCSVMHEALHNHYTQKSLSLSPGK |
| cA b2 - z3 - p2 uI g G 1 | H ea vy ch ai n | 2 5 7 | EVQLVESGAvVKKPGASVKVSCKASGYTLSDFNMDWVRQAPGKSLEWMGDINPNYDTITYNQKFRGRVTLTVDRSA NTAYMELSSLRSEDTAVYYCARGELGRYFYVWGAGTMVTVSSASTKGPSVFPLAPSSKSTSGGTAALGCLVKDYFPEP VTVSWNSGALTSGVHTFPAVLQSSGLYSLSSVVTVPSSSLGTQTYICNVNHKPSNTKVDKRVEPKSCDKTHTCPPCPAP ELLGGPSVFLFPPKPKDTLMISRTPEVTCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQVSLTCLVKGFYPSDIAVEWESNGQPE NNYKTTPPVLDSDGSFFLYSKLTVDKSRWQQGNVFSCSVMHEALHNHYTQKSLSLSPGK |
| cA b2 - z4 - p2 uI g G 1 | H ea vy ch ai n | 2 5 8 | EVKLVESGAEVKKPGASVKVSCKASGYTLSDFNMDWVRQAPGKSLEWMGDINPNYDTITYNQKFRGRVTLTVDRST NTAYMELSSLRSEDTAVYYCARGELGRYFYVWGAGTMVTVSSASTKGPSVFPLAPSSKSTSGGTAALGCLVKDYFPEP VTVSWNSGALTSGVHTFPAVLQSSGLYSLSSVVTVPSSSLGTQTYICNVNHKPSNTKVDKRVEPKSCDKTHTCPPCPAP ELLGGPSVFLFPPKPKDTLMISRTPEVTCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQVSLTCLVKGFYPSDIAVEWESNGQPE NNYKTTPPVLDSDGSFFLYSKLTVDKSRWQQGNVFSCSVMHEALHNHYTQKSLSLSPGK |
| cA b2 - z5 - p2 uI g G 1 | H ea vy ch ai n | 2 5 9 | QVQLVESGAEVKKPGASVKVSCKASGYTLSDFNMDWVRQAQGKSLEWMGDINPNYDTITYNQKFRGRVTLTVDRST NTAYMELSSLRSEDTAVYYCARGELGRYFYVWGAGTMVTVSSASTKGPSVFPLAPSSKSTSGGTAALGCLVKDYFPEP VTVSWNSGALTSGVHTFPAVLQSSGLYSLSSVVTVPSSSLGTQTYICNVNHKPSNTKVDKRVEPKSCDKTHTCPPCPAP ELLGGPSVFLFPPKPKDTLMISRTPEVTCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQVSLTCLVKGFYPSDIAVEWESNGQPE NNYKTTPPVLDSDGSFFLYSKLTVDKSRWQQGNVFSCSVMHEALHNHYTQKSLSLSPGK |
| cA b2 - z1 - p5 u K | Li gh t ch ai n | 2 6 0 | DIQLTQPPSSLSASVGDRVTITCRASENVYSYLAWYQQKPGRSPQLLVYNAKTLQEGVPSRFSGSGSGTQFTLTISSLQP EDFADYYCQHHYGTPWTFGGGTKVEIKRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNRGEC |
| cA b2 - z2 - p5 u K | Li gh t ch ai n | 2 6 1 | DIQLTQPPSSLSASVGDRVTITCRASENVYSYLAWYQQKPGRSPQLLVYNAKTLQEGVPSRFSGSGSGTQFTLTISSLQP EDFADYYCQHHYGTPWTFGGGTKVEIKRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNRGEC |
| cA b2 - z3 - p5 u K | Li gh t ch ai n | 2 6 2 | DIQLTQPPSFLSASVGDRVTITCRASENVYSYLAWYQQKPGRSPQLLVYNAKTLQEGVPSRFSGSGSGTEFTLTISSLQPE DFADYYCQHHYGTPWTFGGGTKVEIKRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNRGEC |
| cA b2 - z4 - p5 u K | Li gh t ch ai n | 2 6 3 | DIQLTQPPSSLSASVGDRVTITCRASENVYSYLAWYQQKPGRSPQLLVYNAKTLQEGVPSRFSGSGSGTQFTLTISSLQP EDVADYYCQHHYGTPWTFGGGTKVEIKRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNRGEC |
| cA b2 - z5 - p5 u K | Li gh t ch ai n | 2 6 4 | DIQLTQPPSSLSASVGDRVTITCRASENVYSYLAWYQQKPGRSPQLLVYNAKTLPEGVPSRFSGSGSGTQFTLTISSLQPE DFADYYCSHHYGTPWTFGGGTKVEIKRTVAAPSVFIFPPSDEQLKSGTASVVCLLNNFYPREAKVQWKVDNALQSGNS QESVTEQDSKDSTYSLSSTLTLSKADYEKHKVYACEVTHQGLSSPVTKSFNRGEC |

**Table 9. Amino Acid Sequences of the Heavy and Light Chains of Humanized Antibodies**

| ID of Humanize d Antibody | | Amino acid sequence |
|---|---|---|
| cAb1 -z0-p7 | H ea vy ch ai n | EVQLVESGGGLVKPGGSLRLSCAASGFTFSSYGMSWVRQAPGKGLEWVSSISGSGSYTYYPDSLKGRFTISRDNAKNSL YLQMNSLRAEDTAVYYCARQQGYWYFDVWGQGTTVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTKVDKRVEPKSCDKTHTCPPCPAPELLGG PSVFLFPPKPKDTLMISRTPEVTCVVVDVSHEDPEVKFNWYVDGVEVHNAKTKPREEQYNSTYRVVSVLTVLHQDWLN GKEYKCKVSNKALPAPIEKTISKAKGQPREPQVYTLPPSREEMTKNQVSLTCLVKGFYPSDIAVEWESNGQPENNYKTTP PVLDSDGSFFLYSKLTVDKSRWQQGNVFSCSVMHEALHNHYTQKSLSLSPGK (SEQ ID NO: 248) |
| | Li gh t ch ai n | DIVMTQTPLSLPVTPGEPASISCRASQSLASSYGKTYLSWYLQKPGQSPQLLIYGISNRFSGVPDRFSGSGSGTDFTLKISR VEAEDVGVYYCLHGTHQPLTFGQGTKLEIKRTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPVTKSFNRGEC (SEQ ID NO: 249) |
| cAb1 -z3-p7 | H ea vy ch ai n | EVQLVESGGGLVKPGGSLRLSCAASGFTFSSYGMSWVRQAPGKRLEWVSSISGSGSYTYYPDSLKGRFTISRDNAKNSLY LQMNSLRAEDTAVYYCARQQGYWYFDVWGQGTTVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKRVEPKSCDKTHTCPPCPAPELLGGP SVFLFPPKPKDTLMISRTPEVTCVVVDVSHEDPEVKFNWYVDGVEVHNAKTKPREEQYNSTYRVVSVLTVLHQDWLNG KEYKCKVSNKALPAPIEKTISKAKGQPREPQVYTLPPSREEMTKNQVSLTCLVKGFYPSDIAVEWESNGQPENNYKTTPP VLDSDGSFFLYSKLTVDKSRWQQGNVFSCSVMHEALHNHYTQKSLSLSPGK (SEQ ID NO: 250) |
| | Li gh t ch ai n | DVVMTQTPLSLPVTPGEPASISCRASQSLASSYGKTYLSWYLQKPGQSPQLLIYGISNRFSGVPDRFSGSGSGTDFTLKISR VEAEDVGVYYCLHGTHQPLTFGQGTKLEIKRTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPVTKSFNRGEC (SEQ ID NO: 251) |
| cAb1 -z5-p7 | H ea vy ch ai n | EVQLVESGGGLVKPGGSLRLSCAASGFTFSSYGMSWVRQAPGKRLEWVASISGSGSYTYYPDSLKGRFTISRDNAKNSL YLQMNSLRAEDTAVYYCARQQGYWYFDVWGQGTTVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTKVDKRVEPKSCDKTHTCPPCPAPELLGG PSVFLFPPKPKDTLMISRTPEVTCVVVDVSHEDPEVKFNWYVDGVEVHNAKTKPREEQYNSTYRVVSVLTVLHQDWLN GKEYKCKVSNKALPAPIEKTISKAKGQPREPQVYTLPPSREEMTKNQVSLTCLVKGFYPSDIAVEWESNGQPENNYKTTP PVLDSDGSFFLYSKLTVDKSRWQQGNVFSCSVMHEALHNHYTQKSLSLSPGK (SEQ ID NO: 252) |
| | Li gh t ch ai n | DVVMTQTPLSLPVTPGEPASISCRASQSLASSYGKTYLSWYLQKPGQSPQLLIYGISNRFSGVPDRFSGSGSGTDFTLKISR VEAEDVGVYYCLHGTHQPLTFGQGTKLEIKRTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPVTKSFNRGEC (SEQ ID NO: 251) |
| cAb1 - uIgG 1K | H ea vy ch ai n | EVQLQESGGDLVKPGGSLKLSCAASGFTFSSYGMSWVRQTPEKRLEWVASISGSGSYTYYPDSLKGRFTISRDNAKNNL YLQMSSLRSEDTALYYCARQDGYWYFDVWGAGTTVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKRVEPKSCDKTHTCPPCPAPELLGGP SVFLFPPKPKDTLMISRTPEVTCVVVDVSHEDPEVKFNWYVDGVEVHNAKTKPREEQYNSTYRVVSVLTVLHQDWLNG KEYKCKVSNKALPAPIEKTISKAKGQPREPQVYTLPPSREEMTKNQVSLTCLVKGFYPSDIAVEWESNGQPENNYKTTPP VLDSDGSFFLYSKLTVDKSRWQQGNVFSCSVMHEALHNHYTQKSLSLSPGK |
| | | (SEQ ID NO: 253) |
| | Li gh t ch ai n | DVLMTQTPLSLPVSLGDQVSISCRASQSLANSYGKTYLSWYLHKPGQSPQLLIYGISNRFSGVPDRFSGSGSGTDFTLKIS TIKPEDLGMYYCLHGTHQPLTFGAGTKLELKRTVAAPSVFIFPPSDEQLKSGTASVVCLLNNFYPREAKVQWKVDNALQ SGNSQESVTEQDSKDSTYSLSSTLTLSKADYEKHKVYACEVTHQGLSSPVTKSFNRGEC (SEQ ID NO: 254) |
| cAb2 -z0-p9 | H ea vy ch ai n | EVKLVESGAVLVKPGASVKISCKASGYTLSDFNMDWVKQSQGKSLEWVGDINPNYDTITYNQKFRGKATLTVDRSSNTA YMELRSLTSEDSAVYYCARGELGRYFYVWGAGTTVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKRVEPKSCDKTHTCPPCPAPELLGGP SVFLFPPKPKDTLMISRTPEVTCVVVDVSHEDPEVKFNWYVDGVEVHNAKTKPREEQYNSTYRVVSVLTVLHQDWLNG KEYKCKVSNKALPAPIEKTISKAKGQPREPQVYTLPPSREEMTKNQVSLTCLVKGFYPSDIAVEWESNGQPENNYKTTPP VLDSDGSFFLYSKLTVDKSRWQQGNVFSCSVMHEALHNHYTQKSLSLSPGK (SEQ ID NO: 255) |
| | Li gh t ch ai n | DIQLTQPPSSLSASVGDRVTITCRASENVYSYLAWYQQKPGRSPQLLVYNAKTLQEGVPSRFSGSGSGTQFTLTISSLQPED VADYYCQHHYGTPWTFGGGTKVEIKRTVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRGEC (SEQ ID NO: 263) |
| cAb2 -z1-p9 | H ea vy ch ai n | EVKLVESGAVLVKPGASVKISCKASGYTLSDFNMDWVKQSQGKSLEWVGDINPNYDTITYNQKFRGKATLTVDRSSNTA YMELRSLTSEDSAVYYCARGELGRYFYVWGAGTTVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKRVEPKSCDKTHTCPPCPAPELLGGP SVFLFPPKPKDTLMISRTPEVTCVVVDVSHEDPEVKFNWYVDGVEVHNAKTKPREEQYNSTYRVVSVLTVLHQDWLNG KEYKCKVSNKALPAPIEKTISKAKGQPREPQVYTLPPSREEMTKNQVSLTCLVKGFYPSDIAVEWESNGQPENNYKTTPP VLDSDGSFFLYSKLTVDKSRWQQGNVFSCSVMHEALHNHYTQKSLSLSPGK (SEQ ID NO: 255) |
| | Li gh t ch ai n | DIQLTQPPSSLSASVGDRVTITCRASENVYSYLAWYQQKPGRSPQLLVYNAKTLPEGVPSRFSGSGSGTQFTLTISSLQPED FADYYCSHHYGTPWTFGGGTKVEIKRTVAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGEC (SEQ ID NO: 264) |
| cAb2 -z2-p6 | H ea vy ch ai n | QVQLVESGAvVKKPGASVKVSCKASGYTLSDFNMDWVRQAPGKSLEWMGDINPNYDTITYNQKFRGRVTLTVDRSINT AYMELSRLRSDDTAVYYCARGELGRYFYVWGAGTMVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTKVDKRVEPKSCDKTHTCPPCPAPELLGG PSVFLFPPKPKDTLMISRTPEVTCVVVDVSHEDPEVKFNWYVDGVEVHNAKTKPREEQYNSTYRVVSVLTVLHQDWLN GKEYKCKVSNKALPAPIEKTISKAKGQPREPQVYTLPPSREEMTKNQVSLTCLVKGFYPSDIAVEWESNGQPENNYKTTP PVLDSDGSFFLYSKLTVDKSRWQQGNVFSCSVMHEALHNHYTQKSLSLSPGK (SEQ ID NO: 256) |
| | Li gh t ch ai n | DIQLTQPPSSLSASVGDRVTITCRASENVYSYLAWYQQKPGRSPQLLVYNAKTLQEGVPSRFSGSGSGTQFTLTISSLQPED FADYYCQHHYGTPWTFGGGTKVEIKRTVAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGEC (SEQ ID NO: 261) |
| cAb2 -z3-p6 | H ea vy ch ai n | QVQLVESGAvVKKPGASVKVSCKASGYTLSDFNMDWVRQAPGKSLEWMGDINPNYDTITYNQKFRGRVTLTVDRSINT AYMELSRLRSDDTAVYYCARGELGRYFYVWGAGTMVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTKVDKRVEPKSCDKTHTCPPCPAPELLGG PSVFLFPPKPKDTLMISRTPEVTCVVVDVSHEDPEVKFNWYVDGVEVHNAKTKPREEQYNSTYRVVSVLTVLHQDWLN GKEYKCKVSNKALPAPIEKTISKAKGQPREPQVYTLPPSREEMTKNQVSLTCLVKGFYPSDIAVEWESNGQPENNYKTTP PVLDSDGSFFLYSKLTVDKSRWQQGNVFSCSVMHEALHNHYTQKSLSLSPGK (SEQ ID NO: 256) |
| | Li gh t ch ai n | DIQLTQPPSFLSASVGDRVTITCRASENVYSYLAWYQQKPGRSPQLLVYNAKTLQEGVPSRFSGSGSGTEFTLTISSLQPED FADYYCQHHYGTPWTFGGGTKVEIKRTVAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGEC (SEQ ID NO: 262) |
| cAb2 -z4-p6 | H ea vy ch ai n | QVQLVESGAvVKKPGASVKVSCKASGYTLSDFNMDWVRQAPGKSLEWMGDINPNYDTITYNQKFRGRVTLTVDRSINT AYMELSRLRSDDTAVYYCARGELGRYFYVWGAGTMVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTKVDKRVEPKSCDKTHTCPPCPAPELLGG PSVFLFPPKPKDTLMISRTPEVTCVVVDVSHEDPEVKFNWYVDGVEVHNAKTKPREEQYNSTYRVVSVLTVLHQDWLN GKEYKCKVSNKALPAPIEKTISKAKGQPREPQVYTLPPSREEMTKNQVSLTCLVKGFYPSDIAVEWESNGQPENNYKTTP PVLDSDGSFFLYSKLTVDKSRWQQGNVFSCSVMHEALHNHYTQKSLSLSPGK (SEQ ID NO: 256) |
| | Li gh t ch ai n | DIQLTQPPSSLSASVGDRVTITCRASENVYSYLAWYQQKPGRSPQLLVYNAKTLQEGVPSRFSGSGSGTQFTLTISSLQPED VADYYCQHHYGTPWTFGGGTKVEIKRTVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRGEC (SEQ ID NO: 263) |
| cAb2 -z5-p6 | H ea vy ch ai n | QVQLVESGAvVKKPGASVKVSCKASGYTLSDFNMDWVRQAPGKSLEWMGDINPNYDTITYNQKFRGRVTLTVDRSINT AYMELSRLRSDDTAVYYCARGELGRYFYVWGAGTMVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTKVDKRVEPKSCDKTHTCPPCPAPELLGG PSVFLFPPKPKDTLMISRTPEVTCVVVDVSHEDPEVKFNWYVDGVEVHNAKTKPREEQYNSTYRVVSVLTVLHQDWLN GKEYKCKVSNKALPAPIEKTISKAKGQPREPQVYTLPPSREEMTKNQVSLTCLVKGFYPSDIAVEWESNGQPENNYKTTP PVLDSDGSFFLYSKLTVDKSRWQQGNVFSCSVMHEALHNHYTQKSLSLSPGK (SEQ ID NO: 256) |
| | Li gh t ch ai n | DIQLTQPPSSLSASVGDRVTITCRASENVYSYLAWYQQKPGRSPQLLVYNAKTLPEGVPSRFSGSGSGTQFTLTISSLQPED FADYYCSHHYGTPWTFGGGTKVEIKRTVAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGEC (SEQ ID NO: 264) |

### Example 4. Characterization of Humanized Antibodies

### 4.1 Affinity Validation of Humanized Antibodies - FACS Binding

NCI-H446 cells were transferred to 96-well U-bottom plates at a density of 1 x 10⁵ cells/well, and centrifuged to remove supernatant. The prepared test supernatant was diluted with 1X PBS + 1% BSA to 146 µg/mL, followed by a 5-fold serial gradient dilution. The dilutions were added to the centrifuged 96-well plate, and incubated at 4°C for 1 hour. Then, the cells were washed twice with 1 x PBS + 1% BSA, and resuspended with 100 µL of the prepared secondary antibody (Goat anti-human Fc, 1:100 dilution) and incubated in dark at 4°C for 30 minutes. After washing the cells twice with 1X PBS + 1% BSA, the cells were filtered through a 200-mesh strainer and resuspended with 100 µL of 1X PBS + 1% BSA. Fluorescence intensity was measured by flow cytometer (BD Canto II) and analyzed by FlowJo. EC50 was calculated by Graphpad Prism 5.

**Table 10. EC50 for Binding of Humanized Antibodies and Their Parental Chimeric Antibodies to NCI-H446 Cells**

| **No.** | **Antibody** | **EC50 (nM)** |
|---|---|---|
| 1 | cAb1-uIgG1K | 3.45 |
| 2 | cAb1-z0-p7 | 9.70 |
| 3 | cAb1-z3-p7 | 4.66 |
| 4 | cAb1-z5-p7 | 3.69 |
| 5 | 28-B1-E8-C11 | 4.451 |
| 6 | cAb2-z0-p9 | 1.151 |
| 7 | cAb2-z1-p9 | 2.217 |
| 8 | cAb2-z2-p6 | 1.359 |
| 9 | cAb2-z3-p6 | 1.063 |
| 10 | cAb2-z4-p6 | 0.5519 |
| 11 | cAb2-z5-p6 | 3.918 |

### 4.2 Affinity Validation of Humanized Antibodies - SPR

The affinity of humanized antibodies for binding to the B7-H3 Human-His antigen was detected using Biacore 8K with the SPR technique. Humanized antibodies were captured by an anti-human IgG Fc antibody immobilized on a CM5 chip. The analyte B7-H3 Human-His at 7 serial gradient dilutions (18.75, 37.5, 75, 150, 300, 600, and 1200 nM) and buffer were flowed through the chip channel at an injection rate of 30 µL/min, with an association time of 180 s, and a dissociation time of 3600 s. After each association and dissociation, the chip surface was regenerated by treatment with 10 mM Glycine-HCl, pH 1.5 buffer. The final association-dissociation curve was the result of subtracting the reference channel (Fc1) and the buffer channel. The experimental data were fitted with a 1: 1 binding model to obtain kinetic data. The results are shown in Table 11.

**Table 11. Affinity for Binding of Humanized Antibodies and Their Parental Chimeric Antibodies to B7-H3 Human-His Antigen**

| Antigen | **Antibody** | ***kₐ* (1/Ms)** | ***k_{off}* (1/s)** | **K_{D} (M)** |
|---|---|---|---|---|
| B7-H3 Human-His | cAb1-uIgG1K | 2.04x10⁴ | 6.10x10⁻⁵ | 2.99 x10⁻⁹ |
| | cAb1-z0-p7 | 1.26x10⁴ | 8.20x10⁻⁵ | 6.53 x10⁻⁹ |
| | cAb1-z3-p7 | 1.71x10⁴ | 6.87x10⁻⁵ | 4.02 x10⁻⁹ |
| | cAb1-z5-p7 | 2.05x10⁴ | 7.21x10⁻⁵ | 3.51 x10⁻⁹ |
| | 28-B1-E8-C11 | 1.432x10⁵ | 8.464x10⁻⁴ | 5.910x10⁻⁹ |
| | cAb2-z1-p9 | 1.652x10⁵ | 8.018x10⁻⁴ | 4.854x10⁻⁹ |
| | cAb2-z5-p6 | 1.843x10⁵ | 1.217x10⁻³ | 6.603x10⁻⁹ |

### 4.3 Internalization Ability Detection of Humanized Antibodies - FACS

The test antibodies were labeled with the pHAb Amine and Thiol Reactive Dyes kit (Promega, G9845), to detect the internalization ability of the antibodies in Calu-6 cells and NCI-H446 cells.

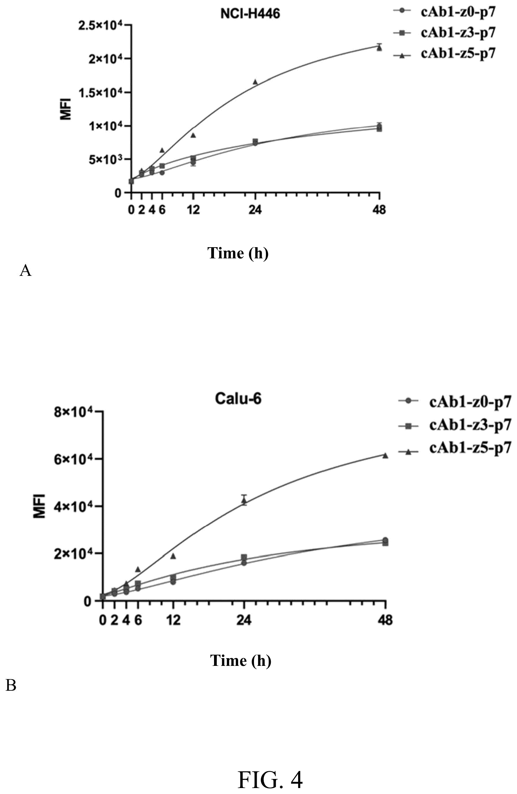

Calu-6 cells and NCI-H446 cells were resuscitated and passaged at least twice. When the cells were cultured to the logarithmic growth phase, the cells were collected. The cells were plated at 1*10E5/well in a 24-well cell culture plate and cultured overnight at 37°C, 5% CO₂. The next day, the original culture medium was replaced with an antibody solution labeled with pHAb Amine and Thiol Reactive Dyes kit (Promega, G9845) formulated with cell culture medium at 10 µg/mL. The culture dishes were incubated in an incubator at 37°C for 0 h, 2 h, 4 h, 6 h, 12 h, 24 h, and 48 h, respectively. After incubation, the cells were collected, and the mean fluorescence intensity (MFI) of the PE channel (pHAb dye) was determined on-machine. A time-MFI curve was plotted using the four-parameter equation in GraphPad Prism 8.0, with time on the x-axis and the mean of MFI on the y-axis, for internalization kinetic analysis. The results are shown in FIG. 4.

According to the results of the endocytosis assay, the candidate antibodies cAb1-z0-p7, cAb1-z3-p7, and cAb1-z5-p7 all exhibited endocytotic effects on Calu-6 and NCI-H446 cells. cAb1-z5-p7 antibody showed the strongest internalization ability, and the internalization abilities of cAb1-z0-p7 and cAb1-z3-p7 antibodies were essentially consistent.

### 4.4 Species Specificity of Humanized Antibody - FACS

The affinity of humanized antibodies for binding to B7-H3 Cyno-His antigen was detected using Biacore 8K with the SPR technique. Humanized antibodies were captured by an anti-human IgG Fc antibody immobilized on a CM5 chip. The analyte B7-H3 Cyno-His at 7 serial gradient dilutions (18.75 nM, 37.5 nM, 75 nM, 150 nM, 300 nM, 600 nM, and 1200 nM) and buffer were flowed through the chip channel at an injection rate of 30 µL/min, with an association time of 180 s, and a dissociation time of 3600 s. After each association and dissociation, the chip surface was regenerated by treatment with 10 mM Glycine-HCl, pH 1.5 buffer. The final association-dissociation curve was the result of subtracting the reference channel (Fc1) and the buffer channel. The experimental data were fitted with a 1: 1 binding model to obtain kinetic data. The results are shown in Table 12.

**Table 12. Affinity for Binding of Humanized Antibodies and Their Parental Chimeric Antibodies to B7-H3 Cyno-His Antigen**

| Antigen | **Antibody** | ***kₐ* (1/Ms)** | ***k_{off}* (1/s)** | **K_{D} (M)** |
|---|---|---|---|---|
| B7-H3 Cyno-his | cAb1-uIgG1K | 1.63 x10⁴ | 5.47 x10⁻⁵ | 3.35 x10⁻⁹ |
| | cAb 1-z0-p7 | 9.77 x10³ | 7.68 x10⁻⁵ | 7.86 x10⁻⁹ |
| | cAb1-z3-p7 | 1.51 x10⁴ | 5.82 x10⁻⁵ | 3.87 x10⁻⁹ |
| | cAb1-z5-p7 | 1.58 x10⁴ | 6.17 x10⁻⁵ | 3.90 x10⁻⁹ |

From Tables 11 and 12, it can be seen that the humanized antibodies can bind to both human B7-H3 and cynomolgus monkey B7-H3.

### 4.5 Target Binding Specificity of Humanized Antibodies - ELISA

B7-H3 family His-tagged antigen proteins, hB7-H1 (PD-L1), B7-H2, B7-H4, B7-H5, B7-H6, B7-1, B7-2, PD-L2, B7-H7, and Human B7-H3-His (ACRO, CAT# B7B-H52E7) purchased from Acro were selected. The antigen proteins were diluted with sterile CBS to a final concentration of 1 µg/mL, and 100 µL per well was added to a 96-well plate. After washing 3 times with PBST (containing 0.5% Tween), 200 µL/well of 3% BSA was added for blocking at 37°C for 2 hours. After washing, 100 µL gradient dilutions of test humanized antibodies, cAb1-z0-p7, cAb1-z3-p7, and cAb1-z5-p7 (starting concentration 10 µg/ml, with 7 points in 5-fold dilution) were added to each well, incubated at room temperature for 1 hour, and washed 3 times with PBST. 100 µL of HRP-Protein A (1:10000 dilution) was added and incubated at room temperature for 1 h. After washing with PBST, 100 µL/well of TMB substrate was added. The OD450 values in the wells were read using a microplate reader.

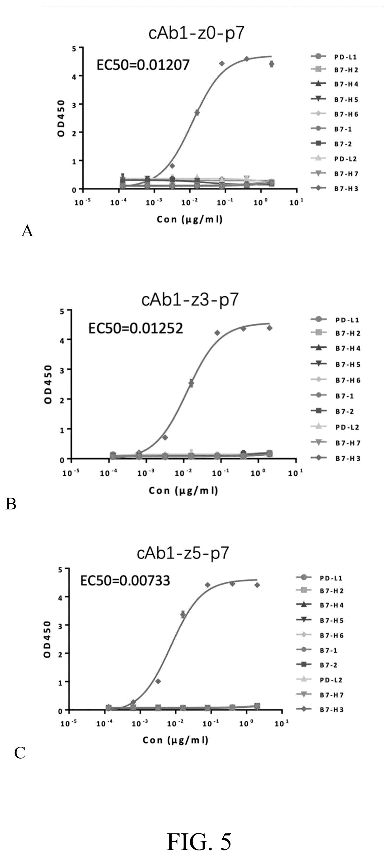

The results are shown in FIG. 5. The results show that the three humanized candidate antibodies, cAb1-z0-p7, cAb1-z3-p7, and cAb1-z5-p7, all exhibit strong binding to B7-H3, while not to other proteins, demonstrating binding specificity for B7-H3.

## Claims

1. An antibody that specifically binds to B7-H3 or an antigen-binding fragment thereof, wherein the antibody comprises a heavy chain variable region (VH) and a light chain variable region (VL), and wherein
(1) the VH comprises HCDR1, HCDR2, and HCDR3 in a heavy chain variable region having the amino acid sequence as set forth in SEQ ID NO: 3, and the VL comprises LCDR1, LCDR2, and LCDR3 in a light chain variable region having the amino acid sequence as set forth in SEQ ID NO: 13;
(2) the VH comprises HCDR1, HCDR2, and HCDR3 in a heavy chain variable region having the amino acid sequence as set forth in SEQ ID NO: 26, and the VL comprises LCDR1, LCDR2, and LCDR3 in a light chain variable region having the amino acid sequence as set forth in SEQ ID NO: 27;
(3) the VH comprises HCDR1, HCDR2, and HCDR3 in a heavy chain variable region having the amino acid sequence as set forth in SEQ ID NO: 28, and the VL comprises LCDR1, LCDR2, and LCDR3 in a light chain variable region having the amino acid sequence as set forth in SEQ ID NO: 29;
(4) the VH comprises HCDR1, HCDR2, and HCDR3 in a heavy chain variable region having the amino acid sequence as set forth in SEQ ID NO: 30, and the VL comprises LCDR1, LCDR2, and LCDR3 in a light chain variable region having the amino acid sequence as set forth in SEQ ID NO: 29;
(5) the VH comprises HCDR1, HCDR2, and HCDR3 in a heavy chain variable region having the amino acid sequence as set forth in SEQ ID NO: 22, and the VL comprises LCDR1, LCDR2, and LCDR3 in a light chain variable region having the amino acid sequence as set forth in SEQ ID NO: 23;
(6) the VH comprises HCDR1, HCDR2, and HCDR3 in a heavy chain variable region having the amino acid sequence as set forth in SEQ ID NO: 33, and the VL comprises LCDR1, LCDR2, and LCDR3 in a light chain variable region having the amino acid sequence as set forth in SEQ ID NO: 42;
(7) the VH comprises HCDR1, HCDR2, and HCDR3 in a heavy chain variable region having the amino acid sequence as set forth in SEQ ID NO: 34, and the VL comprises LCDR1, LCDR2, and LCDR3 in a light chain variable region having the amino acid sequence as set forth in SEQ ID NO: 42;
(8) the VH comprises HCDR1, HCDR2, and HCDR3 in a heavy chain variable region having the amino acid sequence as set forth in SEQ ID NO: 33, and the VL comprises LCDR1, LCDR2, and LCDR3 in a light chain variable region having the amino acid sequence as set forth in SEQ ID NO: 41;
(9) the VH comprises HCDR1, HCDR2, and HCDR3 in a heavy chain variable region having the amino acid sequence as set forth in SEQ ID NO: 34, and the VL comprises LCDR1, LCDR2, and LCDR3 in a light chain variable region having the amino acid sequence as set forth in SEQ ID NO: 39;
(10) the VH comprises HCDR1, HCDR2, and HCDR3 in a heavy chain variable region having the amino acid sequence as set forth in SEQ ID NO: 34, and the VL comprises LCDR1, LCDR2, and LCDR3 in a light chain variable region having the amino acid sequence as set forth in SEQ ID NO: 40;
(11) the VH comprises HCDR1, HCDR2, and HCDR3 in a heavy chain variable region having the amino acid sequence as set forth in SEQ ID NO: 34, and the VL comprises LCDR1, LCDR2, and LCDR3 in a light chain variable region having the amino acid sequence as set forth in SEQ ID NO: 41;
(12) the VH comprises HCDR1, HCDR2, and HCDR3 in a heavy chain variable region having the amino acid sequence as set forth in SEQ ID NO: 1, and the VL comprises LCDR1, LCDR2, and LCDR3 in a light chain variable region having the amino acid sequence as set forth in SEQ ID NO: 2;
(13) the VH comprises HCDR1, HCDR2, and HCDR3 in a heavy chain variable region having the amino acid sequence as set forth in SEQ ID NO: 3, and the VL comprises LCDR1, LCDR2, and LCDR3 in a light chain variable region having the amino acid sequence as set forth in SEQ ID NO: 4;
(14) the VH comprises HCDR1, HCDR2, and HCDR3 in a heavy chain variable region having the amino acid sequence as set forth in SEQ ID NO: 5, and the VL comprises LCDR1, LCDR2, and LCDR3 in a light chain variable region having the amino acid sequence as set forth in SEQ ID NO: 6;
(15) the VH comprises HCDR1, HCDR2, and HCDR3 in a heavy chain variable region having the amino acid sequence as set forth in SEQ ID NO: 7, and the VL comprises LCDR1, LCDR2, and LCDR3 in a light chain variable region having the amino acid sequence as set forth in SEQ ID NO: 8;
(16) the VH comprises HCDR1, HCDR2, and HCDR3 in a heavy chain variable region having the amino acid sequence as set forth in SEQ ID NO: 9, and the VL comprises LCDR1, LCDR2, and LCDR3 in a light chain variable region having the amino acid sequence as set forth in SEQ ID NO: 10;
(17) the VH comprises HCDR1, HCDR2, and HCDR3 in a heavy chain variable region having the amino acid sequence as set forth in SEQ ID NO: 11, and the VL comprises LCDR1, LCDR2, and LCDR3 in a light chain variable region having the amino acid sequence as set forth in SEQ ID NO: 12;
(18) the VH comprises HCDR1, HCDR2, and HCDR3 in a heavy chain variable region having the amino acid sequence as set forth in SEQ ID NO: 14, and the VL comprises LCDR1, LCDR2, and LCDR3 in a light chain variable region having the amino acid sequence as set forth in SEQ ID NO: 15;
(19) the VH comprises HCDR1, HCDR2, and HCDR3 in a heavy chain variable region having the amino acid sequence as set forth in SEQ ID NO: 16, and the VL comprises LCDR1, LCDR2, and LCDR3 in a light chain variable region having the amino acid sequence as set forth in SEQ ID NO: 17;
(20) the VH comprises HCDR1, HCDR2, and HCDR3 in a heavy chain variable region having the amino acid sequence as set forth in SEQ ID NO: 18, and the VL comprises LCDR1, LCDR2, and LCDR3 in a light chain variable region having the amino acid sequence as set forth in SEQ ID NO: 19;
(21) the VH comprises HCDR1, HCDR2, and HCDR3 in a heavy chain variable region having the amino acid sequence as set forth in SEQ ID NO: 20, and the VL comprises LCDR1, LCDR2, and LCDR3 in a light chain variable region having the amino acid sequence as set forth in SEQ ID NO: 21; or
(22) the VH comprises HCDR1, HCDR2, and HCDR3 in a heavy chain variable region having the amino acid sequence as set forth in SEQ ID NO: 24, and the VL comprises LCDR1, LCDR2, and LCDR3 in a light chain variable region having the amino acid sequence as set forth in SEQ ID NO: 25.

2. The antibody or the antigen-binding fragment thereof according to claim 1, wherein
(1) the VH comprises HCDR1 as set forth in SEQ ID NO: 84, HCDR2 as set forth in SEQ ID NO: 86, and HCDR3 as set forth in SEQ ID NO: 88, and the VL comprises LCDR1 as set forth in SEQ ID NO: 173, LCDR2 as set forth in SEQ ID NO: 175, and LCDR3 as set forth in SEQ ID NO: 177;
(2) the VH comprises HCDR1 as set forth in SEQ ID NO: 84, HCDR2 as set forth in SEQ ID NO: 86, and HCDR3 as set forth in SEQ ID NO: 126, and the VL comprises LCDR1 as set forth in SEQ ID NO: 213, LCDR2 as set forth in SEQ ID NO: 175, and LCDR3 as set forth in SEQ ID NO: 177;
(3) the VH comprises HCDR1 as set forth in SEQ ID NO: 111, HCDR2 as set forth in SEQ ID NO: 113, and HCDR3 as set forth in SEQ ID NO: 115, and the VL comprises LCDR1 as set forth in SEQ ID NO: 201, LCDR2 as set forth in the amino acid sequence NAK, and LCDR3 as set forth in SEQ ID NO: 205;
(4) the VH comprises HCDR1 as set forth in SEQ ID NO: 111, HCDR2 as set forth in SEQ ID NO: 113, and HCDR3 as set forth in SEQ ID NO: 115, and the VL comprises LCDR1 as set forth in SEQ ID NO: 201, LCDR2 as set forth in the amino acid sequence NAK, and LCDR3 as set forth in SEQ ID NO: 203;
(5) the VH comprises HCDR1 as set forth in SEQ ID NO: 44, HCDR2 as set forth in SEQ ID NO: 46, and HCDR3 as set forth in SEQ ID NO: 48, and the VL comprises LCDR1 as set forth in SEQ ID NO: 135, LCDR2 as set forth in the amino acid sequence YAS, and LCDR3 as set forth in SEQ ID NO: 139;
(6) the VH comprises HCDR1 as set forth in SEQ ID NO: 51, HCDR2 as set forth in SEQ ID NO: 53, and HCDR3 as set forth in SEQ ID NO: 55, and the VL comprises LCDR1 as set forth in SEQ ID NO: 142, LCDR2 as set forth in the amino acid sequence YAS, and LCDR3 as set forth in SEQ ID NO: 145;
(7) the VH comprises HCDR1 as set forth in SEQ ID NO: 58, HCDR2 as set forth in SEQ ID NO: 60, and HCDR3 as set forth in SEQ ID NO: 62, and the VL comprises LCDR1 as set forth in SEQ ID NO: 148, LCDR2 as set forth in the amino acid sequence KVS, and LCDR3 as set forth in SEQ ID NO: 152;
(8) the VH comprises HCDR1 as set forth in SEQ ID NO: 65, HCDR2 as set forth in SEQ ID NO: 67, and HCDR3 as set forth in SEQ ID NO: 69, and the VL comprises LCDR1 as set forth in SEQ ID NO: 154, LCDR2 as set forth in the amino acid sequence RMS, and LCDR3 as set forth in SEQ ID NO: 158;
(9) the VH comprises HCDR1 as set forth in SEQ ID NO: 72, HCDR2 as set forth in SEQ ID NO: 74, and HCDR3 as set forth in SEQ ID NO: 76, and the VL comprises LCDR1 as set forth in SEQ ID NO: 160, LCDR2 as set forth in the amino acid sequence GAS, and LCDR3 as set forth in SEQ ID NO: 164;
(10) the VH comprises HCDR1 as set forth in SEQ ID NO: 79, HCDR2 as set forth in SEQ ID NO: 81, and HCDR3 as set forth in SEQ ID NO: 83, and the VL comprises LCDR1 as set forth in SEQ ID NO: 166, LCDR2 as set forth in the amino acid sequence SGS, and LCDR3 as set forth in SEQ ID NO: 170;
(11) the VH comprises HCDR1 as set forth in SEQ ID NO: 90, HCDR2 as set forth in SEQ ID NO: 92, and HCDR3 as set forth in SEQ ID NO: 94, and the VL comprises LCDR1 as set forth in SEQ ID NO: 179, LCDR2 as set forth in the amino acid sequence FAS, and LCDR3 as set forth in SEQ ID NO: 183;
(12) the VH comprises HCDR1 as set forth in SEQ ID NO: 96, HCDR2 as set forth in SEQ ID NO: 98, and HCDR3 as set forth in SEQ ID NO: 100, and the VL comprises LCDR1 as set forth in SEQ ID NO: 185, LCDR2 as set forth in the amino acid sequence KVS, and LCDR3 as set forth in SEQ ID NO: 186;
(13) the VH comprises HCDR1 as set forth in SEQ ID NO: 101, HCDR2 as set forth in SEQ ID NO: 102, and HCDR3 as set forth in SEQ ID NO: 104, and the VL comprises LCDR1 as set forth in SEQ ID NO: 188, LCDR2 as set forth in the amino acid sequence DTS, and LCDR3 as set forth in SEQ ID NO: 192;
(14) the VH comprises HCDR1 as set forth in SEQ ID NO: 79, HCDR2 as set forth in SEQ ID NO: 107, and HCDR3 as set forth in SEQ ID NO: 109, and the VL comprises LCDR1 as set forth in SEQ ID NO: 195, LCDR2 as set forth in the amino acid sequence WAS, and LCDR3 as set forth in SEQ ID NO: 199; or
(15) the VH comprises HCDR1 as set forth in SEQ ID NO: 117, HCDR2 as set forth in SEQ ID NO: 119, and HCDR3 as set forth in SEQ ID NO: 121, and the VL comprises LCDR1 as set forth in SEQ ID NO: 208, LCDR2 as set forth in the amino acid sequence DTS, and LCDR3 as set forth in SEQ ID NO: 211.

3. The antibody or the antigen-binding fragment thereof according to claim 1 or 2, wherein
(1) the VH comprises an amino acid sequence having at least 80%, at least 85%, at least 90%, at least 95%, at least 98%, at least 99%, or 100% sequence identity to SEQ ID NO: 3, and the VL comprises an amino acid sequence having at least 80%, at least 85%, at least 90%, at least 95%, at least 98%, at least 99% or 100% sequence identity to SEQ ID NO: 13;
(2) the VH comprises an amino acid sequence having at least 80%, at least 85%, at least 90%, at least 95%, at least 98%, at least 99%, or 100% sequence identity to SEQ ID NO: 26, and the VL comprises an amino acid sequence having at least 80%, at least 85%, at least 90%, at least 95%, at least 98%, at least 99% or 100% sequence identity to SEQ ID NO: 27;
(3) the VH comprises an amino acid sequence having at least 80%, at least 85%, at least 90%, at least 95%, at least 98%, at least 99%, or 100% sequence identity to SEQ ID NO: 28, and the VL comprises an amino acid sequence having at least 80%, at least 85%, at least 90%, at least 95%, at least 98%, at least 99% or 100% sequence identity to SEQ ID NO: 29;
(4) the VH comprises an amino acid sequence having at least 80%, at least 85%, at least 90%, at least 95%, at least 98%, at least 99%, or 100% sequence identity to SEQ ID NO: 30, and the VL comprises an amino acid sequence having at least 80%, at least 85%, at least 90%, at least 95%, at least 98%, at least 99% or 100% sequence identity to SEQ ID NO: 29;
(5) the VH comprises an amino acid sequence having at least 80%, at least 85%, at least 90%, at least 95%, at least 98%, at least 99%, or 100% sequence identity to SEQ ID NO: 22, and the VL comprises an amino acid sequence having at least 80%, at least 85%, at least 90%, at least 95%, at least 98%, at least 99% or 100% sequence identity to SEQ ID NO: 23;
(6) the VH comprises an amino acid sequence having at least 80%, at least 85%, at least 90%, at least 95%, at least 98%, at least 99%, or 100% sequence identity to SEQ ID NO: 33, and the VL comprises an amino acid sequence having at least 80%, at least 85%, at least 90%, at least 95%, at least 98%, at least 99% or 100% sequence identity to SEQ ID NO: 42;
(7) the VH comprises an amino acid sequence having at least 80%, at least 85%, at least 90%, at least 95%, at least 98%, at least 99%, or 100% sequence identity to SEQ ID NO: 34, and the VL comprises an amino acid sequence having at least 80%, at least 85%, at least 90%, at least 95%, at least 98%, at least 99% or 100% sequence identity to SEQ ID NO: 42;
(8) the VH comprises an amino acid sequence having at least 80%, at least 85%, at least 90%, at least 95%, at least 98%, at least 99%, or 100% sequence identity to SEQ ID NO: 33, and the VL comprises an amino acid sequence having at least 80%, at least 85%, at least 90%, at least 95%, at least 98%, at least 99% or 100% sequence identity to SEQ ID NO: 41;
(9) the VH comprises an amino acid sequence having at least 80%, at least 85%, at least 90%, at least 95%, at least 98%, at least 99%, or 100% sequence identity to SEQ ID NO: 34, and the VL comprises an amino acid sequence having at least 80%, at least 85%, at least 90%, at least 95%, at least 98%, at least 99% or 100% sequence identity to SEQ ID NO: 39;
(10) the VH comprises an amino acid sequence having at least 80%, at least 85%, at least 90%, at least 95%, at least 98%, at least 99%, or 100% sequence identity to SEQ ID NO: 34, and the VL comprises an amino acid sequence having at least 80%, at least 85%, at least 90%, at least 95%, at least 98%, at least 99% or 100% sequence identity to SEQ ID NO: 40;
(11) the VH comprises an amino acid sequence having at least 80%, at least 85%, at least 90%, at least 95%, at least 98%, at least 99%, or 100% sequence identity to SEQ ID NO: 34, and the VL comprises an amino acid sequence having at least 80%, at least 85%, at least 90%, at least 95%, at least 98%, at least 99% or 100% sequence identity to SEQ ID NO: 41;
(12) the VH comprises an amino acid sequence having at least 80%, at least 85%, at least 90%, at least 95%, at least 98%, at least 99%, or 100% sequence identity to SEQ ID NO: 1, and the VL comprises an amino acid sequence having at least 80%, at least 85%, at least 90%, at least 95%, at least 98%, at least 99% or 100% sequence identity to SEQ ID NO: 2;
(13) the VH comprises an amino acid sequence having at least 80%, at least 85%, at least 90%, at least 95%, at least 98%, at least 99%, or 100% sequence identity to SEQ ID NO: 3, and the VL comprises an amino acid sequence having at least 80%, at least 85%, at least 90%, at least 95%, at least 98%, at least 99% or 100% sequence identity to SEQ ID NO: 4;
(14) the VH comprises an amino acid sequence having at least 80%, at least 85%, at least 90%, at least 95%, at least 98%, at least 99%, or 100% sequence identity to SEQ ID NO: 5, and the VL comprises an amino acid sequence having at least 80%, at least 85%, at least 90%, at least 95%, at least 98%, at least 99% or 100% sequence identity to SEQ ID NO: 6;
(15) the VH comprises an amino acid sequence having at least 80%, at least 85%, at least 90%, at least 95%, at least 98%, at least 99%, or 100% sequence identity to SEQ ID NO: 7, and the VL comprises an amino acid sequence having at least 80%, at least 85%, at least 90%, at least 95%, at least 98%, at least 99% or 100% sequence identity to SEQ ID NO: 8;
(16) the VH comprises an amino acid sequence having at least 80%, at least 85%, at least 90%, at least 95%, at least 98%, at least 99%, or 100% sequence identity to SEQ ID NO: 9, and the VL comprises an amino acid sequence having at least 80%, at least 85%, at least 90%, at least 95%, at least 98%, at least 99% or 100% sequence identity to SEQ ID NO: 10;
(17) the VH comprises an amino acid sequence having at least 80%, at least 85%, at least 90%, at least 95%, at least 98%, at least 99%, or 100% sequence identity to SEQ ID NO: 11, and the VL comprises an amino acid sequence having at least 80%, at least 85%, at least 90%, at least 95%, at least 98%, at least 99% or 100% sequence identity to SEQ ID NO: 12;
(18) the VH comprises an amino acid sequence having at least 80%, at least 85%, at least 90%, at least 95%, at least 98%, at least 99%, or 100% sequence identity to SEQ ID NO: 14, and the VL comprises an amino acid sequence having at least 80%, at least 85%, at least 90%, at least 95%, at least 98%, at least 99% or 100% sequence identity to SEQ ID NO: 15;
(19) the VH comprises an amino acid sequence having at least 80%, at least 85%, at least 90%, at least 95%, at least 98%, at least 99%, or 100% sequence identity to SEQ ID NO: 16, and the VL comprises an amino acid sequence having at least 80%, at least 85%, at least 90%, at least 95%, at least 98%, at least 99% or 100% sequence identity to SEQ ID NO: 17;
(20) the VH comprises an amino acid sequence having at least 80%, at least 85%, at least 90%, at least 95%, at least 98%, at least 99%, or 100% sequence identity to SEQ ID NO: 18, and the VL comprises an amino acid sequence having at least 80%, at least 85%, at least 90%, at least 95%, at least 98%, at least 99% or 100% sequence identity to SEQ ID NO: 19;
(21) the VH comprises an amino acid sequence having at least 80%, at least 85%, at least 90%, at least 95%, at least 98%, at least 99%, or 100% sequence identity to SEQ ID NO: 20, and the VL comprises an amino acid sequence having at least 80%, at least 85%, at least 90%, at least 95%, at least 98%, at least 99% or 100% sequence identity to SEQ ID NO: 21; or
(22) the VH comprises an amino acid sequence having at least 80%, at least 85%, at least 90%, at least 95%, at least 98%, at least 99%, or 100% sequence identity to SEQ ID NO: 24, and the VL comprises an amino acid sequence having at least 80%, at least 85%, at least 90%, at least 95%, at least 98%, at least 99% or 100% sequence identity to SEQ ID NO: 25.

4. The antibody or the antigen-binding fragment thereof according to claim 3, wherein
(1) the VH comprises the amino acid sequence as set forth in SEQ ID NO: 3, and the VL comprises the amino acid sequence as set forth in SEQ ID NO: 13;
(2) the VH comprises the amino acid sequence as set forth in SEQ ID NO: 26, and the VL comprises the amino acid sequence as set forth in SEQ ID NO: 27;
(3) the VH comprises the amino acid sequence as set forth in SEQ ID NO: 28, and the VL comprises the amino acid sequence as set forth in SEQ ID NO: 29;
(4) the VH comprises the amino acid sequence as set forth in SEQ ID NO: 30, and the VL comprises the amino acid sequence as set forth in SEQ ID NO: 29;
(5) the VH comprises the amino acid sequence as set forth in SEQ ID NO: 22, and the VL comprises the amino acid sequence as set forth in SEQ ID NO: 23;
(6) the VH comprises the amino acid sequence as set forth in SEQ ID NO: 33, and the VL comprises the amino acid sequence as set forth in SEQ ID NO: 42;
(7) the VH comprises the amino acid sequence as set forth in SEQ ID NO: 34, and the VL comprises the amino acid sequence as set forth in SEQ ID NO: 42;
(8) the VH comprises the amino acid sequence as set forth in SEQ ID NO: 33, and the VL comprises the amino acid sequence as set forth in SEQ ID NO: 41;
(9) the VH comprises the amino acid sequence as set forth in SEQ ID NO: 34, and the VL comprises the amino acid sequence as set forth in SEQ ID NO: 39;
(10) the VH comprises the amino acid sequence as set forth in SEQ ID NO: 34, and the VL comprises the amino acid sequence as set forth in SEQ ID NO: 40;
(11) the VH comprises the amino acid sequence as set forth in SEQ ID NO: 34, and the VL comprises the amino acid sequence as set forth in SEQ ID NO: 41;
(12) the VH comprises the amino acid sequence as set forth in SEQ ID NO: 1, and the VL comprises the amino acid sequence as set forth in SEQ ID NO: 2;
(13) the VH comprises the amino acid sequence as set forth in SEQ ID NO: 3, and the VL comprises the amino acid sequence as set forth in SEQ ID NO: 4;
(14) the VH comprises the amino acid sequence as set forth in SEQ ID NO: 5, and the VL comprises the amino acid sequence as set forth in SEQ ID NO: 6;
(15) the VH comprises the amino acid sequence as set forth in SEQ ID NO: 7, and the VL comprises the amino acid sequence as set forth in SEQ ID NO: 8;
(16) the VH comprises the amino acid sequence as set forth in SEQ ID NO: 9, and the VL comprises the amino acid sequence as set forth in SEQ ID NO: 10;
(17) the VH comprises the amino acid sequence as set forth in SEQ ID NO: 11, and the VL comprises the amino acid sequence as set forth in SEQ ID NO: 12;
(18) the VH comprises the amino acid sequence as set forth in SEQ ID NO: 14, and the VL comprises the amino acid sequence as set forth in SEQ ID NO: 15;
(19) the VH comprises the amino acid sequence as set forth in SEQ ID NO: 16, and the VL comprises the amino acid sequence as set forth in SEQ ID NO: 17;
(20) the VH comprises the amino acid sequence as set forth in SEQ ID NO: 18, and the VL comprises the amino acid sequence as set forth in SEQ ID NO: 19;
(21) the VH comprises the amino acid sequence as set forth in SEQ ID NO: 20, and the VL comprises the amino acid sequence as set forth in SEQ ID NO: 21; or
(22) the VH comprises the amino acid sequence as set forth in SEQ ID NO: 24, and the VL comprises the amino acid sequence as set forth in SEQ ID NO: 25.

5. The antibody or the antigen-binding fragment thereof according to any one of claims 1-4, wherein the antibody is a murine antibody, a chimeric antibody, a humanized antibody, or a human antibody.

6. The antibody or the antigen-binding fragment thereof according to any one of claims 1-5, wherein the antibody is of an isotype selected from the group consisting of IgG, IgA, IgM, IgE, and IgD.

7. The antibody or the antigen-binding fragment thereof according to any one of claims 1-6, wherein the antibody is of a subtype selected from the group consisting of IgG1, IgG2, IgG3, and IgG4.

8. The antibody or the antigen-binding fragment thereof according to any one of claims 1-7, wherein the antigen-binding fragment is selected from the group consisting of Fab, Fab', F(ab')2, Fv, scFv, and ds-scFv.

9. The antibody or the antigen-binding fragment thereof according to any one of claims 1-8, wherein the antibody is a monoclonal antibody.

10. The antibody or the antigen-binding fragment thereof of claim 9, wherein the antibody comprises a light chain and a heavy chain, wherein
(1) the heavy chain comprises an amino acid sequence having at least 80%, at least 85%, at least 90%, at least 95%, at least 98%, at least 99%, or 100% sequence identity to SEQ ID NO: 230, and the light chain comprises an amino acid sequence having at least 80%, at least 85%, at least 90%, at least 95%, at least 98%, at least 99% or 100% sequence identity to SEQ ID NO: 231;
(2) the heavy chain comprises an amino acid sequence having at least 80%, at least 85%, at least 90%, at least 95%, at least 98%, at least 99%, or 100% sequence identity to SEQ ID NO: 248, and the light chain comprises an amino acid sequence having at least 80%, at least 85%, at least 90%, at least 95%, at least 98%, at least 99% or 100% sequence identity to SEQ ID NO: 249;
(3) the heavy chain comprises an amino acid sequence having at least 80%, at least 85%, at least 90%, at least 95%, at least 98%, at least 99%, or 100% sequence identity to SEQ ID NO: 250, and the light chain comprises an amino acid sequence having at least 80%, at least 85%, at least 90%, at least 95%, at least 98%, at least 99% or 100% sequence identity to SEQ ID NO: 251;
(4) the heavy chain comprises an amino acid sequence having at least 80%, at least 85%, at least 90%, at least 95%, at least 98%, at least 99%, or 100% sequence identity to SEQ ID NO: 252, and the light chain comprises an amino acid sequence having at least 80%, at least 85%, at least 90%, at least 95%, at least 98%, at least 99% or 100% sequence identity to SEQ ID NO: 251;
(5) the heavy chain comprises an amino acid sequence having at least 80%, at least 85%, at least 90%, at least 95%, at least 98%, at least 99%, or 100% sequence identity to SEQ ID NO: 240, and the light chain comprises an amino acid sequence having at least 80%, at least 85%, at least 90%, at least 95%, at least 98%, at least 99% or 100% sequence identity to SEQ ID NO: 241;
(6) the heavy chain comprises an amino acid sequence having at least 80%, at least 85%, at least 90%, at least 95%, at least 98%, at least 99%, or 100% sequence identity to SEQ ID NO: 255, and the light chain comprises an amino acid sequence having at least 80%, at least 85%, at least 90%, at least 95%, at least 98%, at least 99% or 100% sequence identity to SEQ ID NO: 264;
(7) the heavy chain comprises an amino acid sequence having at least 80%, at least 85%, at least 90%, at least 95%, at least 98%, at least 99%, or 100% sequence identity to SEQ ID NO: 256, and the light chain comprises an amino acid sequence having at least 80%, at least 85%, at least 90%, at least 95%, at least 98%, at least 99% or 100% sequence identity to SEQ ID NO: 264;
(8) the heavy chain comprises an amino acid sequence having at least 80%, at least 85%, at least 90%, at least 95%, at least 98%, at least 99%, or 100% sequence identity to SEQ ID NO: 255, and the light chain comprises an amino acid sequence having at least 80%, at least 85%, at least 90%, at least 95%, at least 98%, at least 99% or 100% sequence identity to SEQ ID NO: 263;
(9) the heavy chain comprises an amino acid sequence having at least 80%, at least 85%, at least 90%, at least 95%, at least 98%, at least 99%, or 100% sequence identity to SEQ ID NO: 256, and the light chain comprises an amino acid sequence having at least 80%, at least 85%, at least 90%, at least 95%, at least 98%, at least 99% or 100% sequence identity to SEQ ID NO: 261;
(10) the heavy chain comprises an amino acid sequence having at least 80%, at least 85%, at least 90%, at least 95%, at least 98%, at least 99%, or 100% sequence identity to SEQ ID NO: 256, and the light chain comprises an amino acid sequence having at least 80%, at least 85%, at least 90%, at least 95%, at least 98%, at least 99% or 100% sequence identity to SEQ ID NO: 262;
(11) the heavy chain comprises an amino acid sequence having at least 80%, at least 85%, at least 90%, at least 95%, at least 98%, at least 99%, or 100% sequence identity to SEQ ID NO: 256, and the light chain comprises an amino acid sequence having at least 80%, at least 85%, at least 90%, at least 95%, at least 98%, at least 99% or 100% sequence identity to SEQ ID NO: 263;
(12) the heavy chain comprises an amino acid sequence having at least 80%, at least 85%, at least 90%, at least 95%, at least 98%, at least 99%, or 100% sequence identity to SEQ ID NO: 218, and the light chain comprises an amino acid sequence having at least 80%, at least 85%, at least 90%, at least 95%, at least 98%, at least 99% or 100% sequence identity to SEQ ID NO: 219;
(13) the heavy chain comprises an amino acid sequence having at least 80%, at least 85%, at least 90%, at least 95%, at least 98%, at least 99%, or 100% sequence identity to SEQ ID NO: 220, and the light chain comprises an amino acid sequence having at least 80%, at least 85%, at least 90%, at least 95%, at least 98%, at least 99% or 100% sequence identity to SEQ ID NO: 221;
(14) the heavy chain comprises an amino acid sequence having at least 80%, at least 85%, at least 90%, at least 95%, at least 98%, at least 99%, or 100% sequence identity to SEQ ID NO: 222, and the light chain comprises an amino acid sequence having at least 80%, at least 85%, at least 90%, at least 95%, at least 98%, at least 99% or 100% sequence identity to SEQ ID NO: 223;
(15) the heavy chain comprises an amino acid sequence having at least 80%, at least 85%, at least 90%, at least 95%, at least 98%, at least 99%, or 100% sequence identity to SEQ ID NO: 224, and the light chain comprises an amino acid sequence having at least 80%, at least 85%, at least 90%, at least 95%, at least 98%, at least 99% or 100% sequence identity to SEQ ID NO: 225;
(16) the heavy chain comprises an amino acid sequence having at least 80%, at least 85%, at least 90%, at least 95%, at least 98%, at least 99%, or 100% sequence identity to SEQ ID NO: 226, and the light chain comprises an amino acid sequence having at least 80%, at least 85%, at least 90%, at least 95%, at least 98%, at least 99% or 100% sequence identity to SEQ ID NO: 227;
(17) the heavy chain comprises an amino acid sequence having at least 80%, at least 85%, at least 90%, at least 95%, at least 98%, at least 99%, or 100% sequence identity to SEQ ID NO: 228, and the light chain comprises an amino acid sequence having at least 80%, at least 85%, at least 90%, at least 95%, at least 98%, at least 99% or 100% sequence identity to SEQ ID NO: 229;
(18) the heavy chain comprises an amino acid sequence having at least 80%, at least 85%, at least 90%, at least 95%, at least 98%, at least 99%, or 100% sequence identity to SEQ ID NO: 232, and the light chain comprises an amino acid sequence having at least 80%, at least 85%, at least 90%, at least 95%, at least 98%, at least 99% or 100% sequence identity to SEQ ID NO: 233;
(19) the heavy chain comprises an amino acid sequence having at least 80%, at least 85%, at least 90%, at least 95%, at least 98%, at least 99%, or 100% sequence identity to SEQ ID NO: 234, and the light chain comprises an amino acid sequence having at least 80%, at least 85%, at least 90%, at least 95%, at least 98%, at least 99% or 100% sequence identity to SEQ ID NO: 235;
(20) the heavy chain comprises an amino acid sequence having at least 80%, at least 85%, at least 90%, at least 95%, at least 98%, at least 99%, or 100% sequence identity to SEQ ID NO: 236, and the light chain comprises an amino acid sequence having at least 80%, at least 85%, at least 90%, at least 95%, at least 98%, at least 99% or 100% sequence identity to SEQ ID NO: 237;
(21) the heavy chain comprises an amino acid sequence having at least 80%, at least 85%, at least 90%, at least 95%, at least 98%, at least 99%, or 100% sequence identity to SEQ ID NO: 238, and the light chain comprises an amino acid sequence having at least 80%, at least 85%, at least 90%, at least 95%, at least 98%, at least 99% or 100% sequence identity to SEQ ID NO: 239; or
(22) the heavy chain comprises an amino acid sequence having at least 80%, at least 85%, at least 90%, at least 95%, at least 98%, at least 99%, or 100% sequence identity to SEQ ID NO: 242, and the light chain comprises an amino acid sequence having at least 80%, at least 85%, at least 90%, at least 95%, at least 98%, at least 99% or 100% sequence identity to SEQ ID NO: 243.

11. The antibody or the antigen-binding fragment thereof according to any one of claims 1-10, wherein the antibody is a bispecific antibody or a multispecific antibody.

12. A nucleic acid encoding the antibody or the antigen-binding fragment thereof according to any one of claims 1-11.

13. A vector comprising the nucleic acid according to claim 12.

14. A host cell comprising the nucleic acid according to claim 12 or the vector according to claim 13.

15. A pharmaceutical composition comprising (i) the antibody or the antigen-binding fragment thereof according to any one of claims 1-11, the nucleic acid according to claim 12, the vector according to claim 13, or the host cell according to claim 14; and (ii) a pharmaceutically acceptable carrier or excipient.

16. The pharmaceutical composition according to claim 15, further comprising a second therapeutic agent.

17. The pharmaceutical composition according to claim 16, wherein the second therapeutic agent is selected from the group consisting of a cytokine, an antibody, a chemotherapeutic agent, and a small molecule drug.

18. A conjugate comprising the antibody or the antigen-binding fragment thereof according to any one of claims 1-11 and a chemical moiety conjugated thereto.

19. The conjugate according to claim 18, wherein the chemical moiety is selected from the group consisting of a therapeutic agent, a detectable moiety, and an immunostimulatory molecule.

20. Use of the antibody or the antigen-binding fragment thereof according to any one of claims 1-11, the nucleic acid according to claim 12, the vector according to claim 13, or the host cell according to claim 14, the pharmaceutical composition according to any one of claims 15-17, or the conjugate according to claim 18 or 19 in the manufacture of a medicament for treating a cancer in a subject, wherein the cancer is a B7-H3 positive cancer.

21. The use according to claim 20, wherein the cancer is selected from the group consisting of: adrenal tumor, AIDS-related cancer, alveolar soft part sarcoma, astrocytoma, adrenal cancer, bladder cancer, bone cancer, brain and spinal cord cancer, metastatic brain tumor, B-cell cancer, breast cancer, carotid body tumor, cervical cancer, chondrosarcoma, chordoma, chromophobe renal cell carcinoma, clear cell carcinoma, colon cancer, colorectal cancer, cutaneous benign fibrous histiocytoma, desmoplastic small round cell tumor, ependymoma, Ewing's tumor, extraskeletal myxoid chondrosarcoma, incomplete bone fibrogenesis, fibrous dysplasia of bone, gallbladder or bile duct cancer, gastric cancer, gestational trophoblast disease, germ cell tumor, head and neck cancer, hepatocellular carcinoma, islet cell tumor, Kaposi's sarcoma, kidney cancer, leukemia, liposarcoma/malignant lipomatous tumor, liver cancer, lymphoma, lung cancer, medulloblastoma, melanoma, meningioma, multiple endocrine neoplasia, multiple myeloma, myelodysplastic syndrome, neuroblastoma, neuroendocrine tumor, ovarian cancer, pancreatic cancer, papillary thyroid carcinoma, parathyroid tumor, pediatric cancer, peripheral nerve sheath tumor, pheochromocytoma, pituitary tumor, prostate cancer, posterious uveal melanoma, renal metastatic cancer, rhabdoid tumor, rhabdomyosarcoma, sarcoma, skin cancer, soft tissue sarcoma, squamous cell carcinoma, gastric carcinoma, synovial sarcoma, testicular carcinoma, thymic carcinoma, thymoma, metastatic thyroid cancer, and uterine cancer.

22. The use according to claim 20 or 21, wherein the medicament is in combination with a second therapeutic agent.

23. The use of claim 22, wherein the second therapeutic agent is selected from the group consisting of a cytokine, an antibody, a chemotherapeutic agent, and a small molecule drug.
